(19) Europäisches Patentamt
European Patent Office
Office européen des brevets

(11) EP 0 687 300 B1

(12) EUROPEAN PATENT SPECIFICATION

(45) Date of publication and mention of the grant of the patent:
**12.09.2001 Bulletin 2001/37**

(21) Application number: **94909270.4**

(22) Date of filing: **04.03.1994**

(51) Int Cl.$^7$: **C12N 15/13**, C12P 21/08, C07K 16/28 // A61K39/395

(86) International application number:
**PCT/IB94/00043**

(87) International publication number:
**WO 94/20619 (15.09.1994 Gazette 1994/21)**

# (54) LO-CD2a ANTIBODY AND USES THEREOF FOR INHIBITING T-CELL ACTIVATION AND PROLIFERATION

LO-CD2A ANTIKÖRPER UND DESSEN VERWENDUNG ZUR INHIBITION VON T-ZELL-AKTIVIERUNG UND -WACHSTUM

ANTICORPS LO-CD2a ET SES UTILISATIONS DANS L'INHIBITION DE L'ACTIVATION ET DE LA PROLIFERATION DES LYMPHOCYTES T

(84) Designated Contracting States:
**AT BE CH DE DK ES FR GB GR IE IT LI LU MC NL PT SE**

(30) Priority: **05.03.1993 US 27008**
**09.09.1993 US 119032**

(43) Date of publication of application:
**20.12.1995 Bulletin 1995/51**

(73) Proprietor: **UNIVERSITE CATHOLIQUE DE LOUVAIN**
**1348 Louvain la Neuve (BE)**

(72) Inventors:
- **BAZIN, Hervé**
  **B-1200 Brussels (BE)**
- **LATINNE, Dominique**
  **B-1150 Brussels (BE)**

(74) Representative:
**LOUIS, PÖHLAU, LOHRENTZ & SEGETH**
**Postfach 3055**
**90014 Nürnberg (DE)**

(56) References cited:
**US-A- 4 381 292**

- **Belgian Journal of Zoology 121 (suppl.1), p13**
- **TIBTECH vol. 10 , April 1992 , CAMBRIDGE,GB C.CUNNINGHAM AND W.J.HARRIS 'Antibody engineering-how to be human'**
- **NATURE. vol. 332, no. 6162 , 24 March 1988 , LONDON GB pages 323 - 327 L. RIECHMANN ET AL. 'Reshaping human antibodies for therapy'**
- **H. Xia et al., Rat monoclonal antibodies specific for human T lymphocytes in: "Rat hybridomas and rat monoclonal antibodies, (H. Bazin Editor) CRC Press Boca Raton USA 1990, pages 309-322**

Remarks:
The file contains technical information submitted after the application was filed and not included in this specification

EP 0 687 300 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

This invention relates to an antibody (or fragment or derivative thereof) and preferably, to an antibody (or fragment or derivative thereof) which binds to human lymphocytes. More particularly, this invention relates to preventing and/or inhibiting on-going immune responses in a patient through the administration of such antibody (or fragment or derivative thereof) to a patient. Preferably, this invention relates to preventing or inhibiting T cell activation and proliferation through the administration of such antibody or fragment or derivative thereof to a patient.

The prior art has disclosed the possibility of using antibodies to CD2 antigen for inhibiting graft rejection. In general, the prior art discloses the use of antibodies which bind to CD2 antigens as being possibly useful for inhibiting graft rejection, see, Ortho Pharmaceutical Corp., U.S. Pat. Nos. 4,364,973; 4,614,720; 4,515,893; 4,743,681) and 4,798,806.

Such antibodies have not been known to be useful in inhibiting graft rejection in human patients or in animals. As exemplified in the following references, J. V. Giorgi, et al., Immunosuppressive Effect and Immunogenicity of OKTllA Monoclonal Antibody in Monkey Allograft Recipients, Transplantation Proceedings Vol. XV No. 1, March 1983, and P. J. Thurlow, et al., A Monoclonal Anti-Pan-T-Cell Antibody, Transplantation, Vol. 36, No. 3, Pg. 293-298.

### Detailed Description of the Figures

FIGURE 1

Two color staining of peripheral blood mononuclear cells (PBMC) with biotinylated LO-CD2a and Leu-5bPE.

For this staining, the following parameters were followed:

PARAMETER:FLI-H\(LOG) FL2-h(LOG) QUAD LOCATION: 17.15,9

| TOTAL = QUAD | 5000 EVENTS | GATED = % GATED | 1290 % TOTAL | X MEAN | Y MEAN |
|---|---|---|---|---|---|
| 1UL | 299 | 23.18 | 3.98 | 11.41 | 284.69 |
| 1UR | 851 | 65.97 | 17.02 | 32.70 | 630.65 |
| 3LL | 135 | 10.47 | 2.70 | 4.08 | 3.31 |
| 4LR | 5 | 0.39 | 0.10 | 25.11 | 6.54 |

FIGURE 2

Human PBMC were stained with LO-CD2a-FITC and then a) stained with T11-PE(Coulter antibody to CD2) conjugated with phycoerythrin (PK) or b) Leu-5B-PE (Becton Dickinson antibody to CD2) conjugated to phycoerythrin (PE). In neither case was staining by the second antibody altered by pretreatment with LO-CD2ε.

FIGURES 3a and 3b

Effects of LO-CD2a on membrane markers. PBMC at 2x$10^6$ cells/ml were cultured in the absence (solid lines) or in the presence (broken lines) of LO-CD2a (200 ng/ml). At the times indicated in the figures, cells were harvested and treated for cytofluorometric analysis, a) and b); PBMC were labeled with anti-CD3 (Leu-4a-FITC), anti-CD4 (T4-RD) mAbs, anti-class II antigene (LO-DRa-FITC) or anti-CD8 (T8-RD) monocolnal antibodies (mAbs). Negative controls for commercial mouse mAbs were aliquots of the same cells stained with FITC or Rhodamine-labeled mouse IgGs. Negative controls for rat mAbs were cells incubated with normal rat serum followed by a FITC-labeled mouse anti-rat mAb (MARK-FITC). Results are expressed as percentage positive cells.

FIGURES 4a and 4b

Effects of LO-CD2a on membrane markers and human blood lymphocyte culture with and without addition of LO-CD2a. Lymphocyte Cultures at 1x$10^8$ cells ml were labeled with (a) anti-CD2 (Leu-5b-FITC), anti-CD4 (T4-RD1) mAb, or anti-CD8 (T8-RD) mAb at times indicated; (b) mixed lymphocyte cultures at 1x$10^6$ cells par ml of each donor were stained with anti-CD2 mAb Leu-5b (FITC-labeled). Negative controle for commercial mouse mAbs were aliquots of the same cells stained with FITC or Rhodamine-labeled mouse IgGs. Negative controls for rat mAbs were cells incubated with normal rat serum followed by a FITC-labeled mouse anti-rat mAb (MARK-FITC). Results are expressed as percentage positive cells.

FIGURES 5a and 5b

Effects of LO-CD2a and Leu-5b on CD2 expression. Human PbMC were incubated with a) LO-CD2a (200 ng/ml) or b) Leu-5b (dialyzed against PBc, diluted 1:2) for the times indicated and a) stained for expression of CD2 (Leu-5b-FITC and Tll-RD1) and for binding of LO-CD2a (Mark-3-FITC) or b) CD2 (LO-CD2a-FITC, T11-RD1) and for binding of Leu-5b Goat anti-mouse (GAM-FITC).

FIGURE 6

Effects of LO-CD2a on MLR. a) inhibition of MLR in mixed lymphocyte cultures incubated for 6 days in the presence of increasing concentrations of LO-CD2a added at time 0. Cultures were harvested at day 6; b) inhibition of MLR in mixed lymphocyte cultures incubated with different concentrations of LO-CD2a added at time 0. Cultures were harvested at 24 h intervals; c) $^3$H-Thymidine ($^3$H-T) incorporation (cpm) by mixed lymphocyte cultures in the absence (solid line) or in the presence (broken line) of LO-CD2a (200 ng/ml); d) inhibition of MLR by LO-CD2a (200 ng/ml) added at different times after the start of incubation. Cultures were harvested at day 6. All cultures were made in triplicate (1x$10^4$ cells of each donor/ml) in a final volume of 200 μl/well. $^3$H-Thymidine was added 8 h before harvesting cultures, Results in c) are shown as cpm x $10^{-3}$ incorporated per well harvested at the time indicated. Results in a), b) and d) are expressed as percentage inhibition of MLR of triplicate cultures (mean ± S.D.), as compared to control cultures (without LO-CD2a).

FIGURE 7

Peripheral blood mononuclear cells were cultured in mixed lymphocyte cultures with or without the addition of 200 ng/ml of LO-CD2a. At the indicated times cells were removed and analyzed by flow cytometry after staining with antibody to CD2 (Leu5b-FITC). Blast cells were gated by forward and side scatter and the expression of the indicated markers quantified on the blast cells.

FIGURE 6

Effects of LO-CD2a on blast cells. Human lymphocytes were cultured with and without the addition of LO-CD2a (200 ng/ml). At the indicated times cells were removed and stained for CD3 (Leu 4a-FITC), CD4 (T4-RD1) CD8 (T8-RD1) or CD25 (LOTACT-1-FITC). The resting lymphocytes were identified by differential gating for size and granularity and the results are expressed as the per cent of total resting lymphocytes staining with the indicated antibody.

FIGURE 9

Effects of LO-CD2a on mitogen-stimulated lymphocytes. PBMC were cultured for 96 h in the absence (hatched bars) or in the presence (solid bars) of OKT3 (100 ng/ml), Con-A (10 μg/ml) and FHA (1 μg/ml). In parallel cultures, LO-CD2a (200 ng/ml) was added 1 h after mitogens (gray bars) or 1 h before mitogens (blank bars). Squares bars represent cultures performed in the presence of LO-CD2a alone. Cultures (in triplicate) were pulselabeled with $^3$H-Thymidine during the last 8 h of incubation.

FIGURES 10 a and 10b

Inhibition of NK activity by incubation of the effector and target cells ($^{51}$CR labeled K562 cells) in presence of LO-CD2a. Three concentrations of LO-CD2a have been tested: 5 μg/ml, 1 μg/ml, 0.5 μg/ml, Effector cells were peripheral blood lymphocytes of NK activity is expressed as percent lysis of labeled target cells. Two normal subjects were tested at 3 E/T ratios: 200/1, 100/1, 50/1.

FIGURE 11

Effects of LO-CD2a on mitogen-driven activation of PMBC. PMBCs from two donors were cultured for 95h in the presence of OKT3 (100 ng/ml), CON-A (10 μg/ml) and PHA (1 μg/ml). In parallel cultures, LO-CD2a (200 ng/ml) was added at Day 0 (0h) after the initiation of the culture, Day 1 (24h), or Day 2 (48h). The graph depicts the percentage inhibition by LO-CD2a of the mitogen-induced proliferation in each donor.

FIGURE 12

Total lymphocytes per µl of peripheral blood of a cynomolgus monkey receiving 20 mg/day of LO-CD2a for 10 days (days 0-9).

FIGURE 13

PBMC cells from the cynomolgue monkey receiving LO-CD2a at 20 mg/day for 10 day (day 0 to 9) were stained with monoclonal antibodies to CD2 (Leu-5b), CD4 (Leu3a), CD8 (Leu 2a), Natural Killer cells (CD8 and CD11b), and B cells (anti-IgM) on the days indicated and analyzed by flow cytometry. Results are presented as the percentage of the total number of staining cells per microliter of blood.

FIGURE 14

NK activity of a cynomolgus monkey receiving 20 mg/day of LO-CD2a for 10 days (day 0-9). NK activity was assayed on days 11 and 22 and presented as % lysis at E/T of 25/1, 50/1 and 100/1.

FIGURE 15

Serum concentration of LO-CD-2a of a cynomolgus monkey receiving the antibody at 20 mg/day for 10 days (day 0-9). The monoclonal antibody was measured by ELISA as described in the text and expressed in µg/ml.

FIGURE 16

Development of IgG antibody to LO-CD2a in a cynomolgus monkey receiving 20 mg/day of LO-CD2a for 10 days (days 0-9). The antibody to the monoclonal antibody was measured in serial dilutions of serum drawn on the indicated days by the sandwich ELISA described in the text and is expressed as the optical density at 492 nm.

FIGURES 17a and 17b

Effect of LO-CD2a on baboon lymphocytes.

a) On the days indicated blood was obtained from the baboon and cells were stained with the anti-CD2 antibodies Tll-RDl and Leu-5b-FITC, LO-CD2a and MARK3-FITC a mouse anti-rat kalpa 1b antibody coupled to fluorescein to detect bound LO-CD2a.
b) Serum samples taken on the indicated days were evaluated for levels of LO-CD2a by ELISA.

FIGURES 18a and 18b

Effect of LO-CD2a on baboon lymphocytes.
On the indicated days blood was taken and the cells stained to detect bound LO-CD2a with MARK3-FITC and MARK2b-a-biotin (a mouse monoclonal anti-rat IgG2b antibody coupled to biotin) detected with PE-coupled streptavidin).

a) No pretreatment of cells;
b) incubation with 2.5 µg/ml LO-CD2a prior to staining to detect any sites unoccupied by circulating antibody.

FIGURE 19

Effect of LO-CD2a on baboon lymphocytes.
On the indicated days blood samples were taken and stained with T4-RD1 (CD4); T8-RD1 (CD8) or MARK3-FITC (bound LO-CD2a).

FIGURE 20

Leukocytes, lymphocytes and creatinine in patient #1 treated with ATG then LO-CD2a for allograft rejection.

FIGURE 21

Serum levels of LO-CD2a during and following treatment in patient #l.

FIGURE 22

Creatinine in patient #2 prior to, during and following treatment with LO-CD2a.

FIGURE 23

Leukocyte and lymphocyte counts in patient #2 prior to, during and following treatment with LO-CD2a.

FIGURE 24

Serum levels of LO-CD2a in patient #2, drawn just prior to and 2.5 hours after each injection.

FIGURE 25

Leukocyte count, lymphocyte count and serum creatinine level in patient #3 receiving LO-CD2a for rejection of renal allograft.

FIGURE 26

Dual color staining with LO-CD2a and (2) Leu5b, (3) Leu 4(CD3), (4) Leu3a(CD4), (5) Leu2b(CD8) and (6) Leull (anti-CD16) a marker for NK cells. LO-CD2a binding was detected with goat anti-rat IG-FITC. The upper set (1-6) of two color histograms shows the double staining. The low set (7-12) shows single staining with each antibody.

FIGURE 27

Two color staining of human PBL with a rat isotype control for LO-CD2a (Pharmingen, purified rat IgG2b, kappa) or LO-CD2a and phycoerthyrin conjugated antibodies to CD4 (c,d), Cd8 (e,f), CD16(g,h), CD19(i,j) and CD2 (k,l). LO-CD2a and the isotype control were detected with FITC conjugated affinity purified F(ab')2 anti-rat immunoglobulin (Southern Biotechnology). The antibodies to the CD antigens were all phycoerythrin conjugated antibodies obtained from Becton-Dickinson [CD4(Leu3a), CD8(Leu2a), CD16(Leu-11b), CD19(Leu 12) and CD2 (Leu5b)]. In each case staining with the isotype control is shown in the first histogram and the LO-CD2a in the second histogram. Histogram a shows the pattern with the isotype control and b with LO-CD2a.

FIGURE 28

Cytofluorograph analysis of the staining of COS cells transfected with wild-type CD2. The left panels show the histograms of staining of a COS cell transfected with the control vector, not containing CD2; the right set of panels staining of a COS cell translently transfected with a vector containing the entire CD2 molecule. In each set the top histogram shows the staining with murine W632 (antibody to Class I, known to be expressed by COS cells) and 76-2-11 (an isotype control for the murine W632); the middle panel shows staining with Leu5b (anti-CD2 from Becton Dickinson) and 76-2-11. an isotype matched control for Leu5b staining, the bottom panel staining with LO-CD2a and a rat isotype matched control for LO-CD2a.

FIGURES 29a and 29b

Nucleotide and amino acid sequences of the LO-CD2a $V_L$ chain. a) with leader sequence from MRC vector; b) with leader sequence from LO-CD2a gene.

FIGURES 30a and 30b

Nucleotide and amino acid sequences of the LO-CD2a $V_H$ chain. a) with leader sequence from MRC vector; b) with leader sequence from LO-CD2a gene.

Detailed Description

In accordance with an aspect of the present invention, there is provided a molecule (preferably a monoclonal antibody or fragment thereof) which specifically binds to the same epitope (or a portion thereof) on human lymphocytes as the monoclonal antibody produced by the cell line deposited as ATCC Deposit No. HB 11423. The antibody which is produced by the deposited cell line is hereinafter sometimes referred to as LO-CD2a. The term "molecule" or "antibody that binds to the same epitope as LO-CD2a" includes LO-CD2a. The term "LO-CD2a" includes the antibody produced by the deposited cell line ATCC HB 11423 and those identical thereto which may be produced, for example, by recombinant technology. The present invention also relates to the use of such an antibody as well as to a composition comprising the same and a pharmaceutically acceptable diluent or a pharmaceutically acceptable carrier.

The molecules or antibodies of the present invention inhibit human T-cell activation and proliferation and Applicant has found that such inhibition can be effected when adding the molecule or antibody either before or after an agent which stimulates T-cell activation.

The molecules or antibodies of the present invention have the characteristics of binding to an epitope of a CD2 antigen (CD2 positive human T-cells) but it is to be understood, however, that the ability of such molecules or antibodies to inhibit T-cell activation or proliferation may or may not be effected through binding to CD2 positive cells, although Applicant presently believes that the mechanism of action involves binding of the molecule or antibody to CD2 positive cells.

In accordance with another aspect of the present invention there is provided a method of preventing and/or inhibiting on-going immune response in human patients through the administration to the patient of an antibody, hereinafter referred to as LO-CD2a (or fragment or derivative thereof) or any molecule that mimics such antibody or derivative or fragment thereof.

A cell line which produces LO-CD2a, was deposited on July 28, 1993, at the American Type Culture Collection, 12301 Parklawn Drive, Rockville, MD 20852, and was given the ATCC accession number ATCC HB 11423. Such antibody is a rat monoclonal antibody.

Although Applicants do not want to limit the invention to any theoretical reasoning, it is believed that the mechanism which anables the monoclonal antibody of this invention to prevent or reduce the severity of an immune response, and to inhibit the activation and proliferation of T-cells, is the fact that the LO-CD2a antibody decreases the density of CD2 expressed on T cell surfaces and thus decreases the number of $CD2^+$ T lymphocytes. It is believed that these mechanisms of action are responsible for not only the prevention of immune response, but also the reduction in severity of on-going immune responses. In addition, the LO-CD2a antibody inhibits natural killer (NK) cell activity in vitro as exemplified herein. This is pertinent to the present invention since it is believed that a non-MHC restricted cytotoxic mechanism such as NK cell activity has been implicated in graft versus host disease.

In accordance with an aspect of the present invention there is provided a process for inhibiting initial or further activation and proliferation of T cells in a human patient by administering to the patient an effective amount of a molecule (preferably an antibody) which binds to the same epitope (or any part thereof) on human lymphocytes as the LO-CD2a antibody. The preferred molecule is LO-CD2a or a chimeric and/or humanized form thereof. Such a molecule would, for example, contain the same complementarity determining region (CDR) as the LO-CD2a antibody.

The term "inhibit" as used herein throughout this Applicant is intended to mean prevention, or inhibition, or reduction in severity, or induction of tolerance to, or reversal of graft rejection. The term "graft" as used herein for purposes of this application shall mean any and all transplantation, including but not limited to, allograft and renograft transplantation. Such transplantation may by way of example include, but not be limited to, transplantation of cells, bone marrow, tissue, solid-organ, bone, etc.

The term "immune response(s)" as used herein is intended to mean immune responses dependent upon T cell activation and proliferation which includes both cellular effects and T cell dependent antibodies which may be elicited in response to, by way of example and not limitation: (i) grafts, (ii) graft versus host disease, and (iii) autoantigens resulting in autoimmune diseases, which by way of example include but are not limited to rheumatoid arthritis, systemic lupus, multiple sclerosis, diabetes, mellitus, etc.

The molecule employed in the present invention is one which binds to the same epitope (or a part of that epitope) as the LO-CD2a monoclonal antibody. The term "binds to the same epitope as LO-CD2a monoclonal antibody" is intended to describe not only the LO-CD2a monoclonal antibody but also describes other antibodies, fragments or derivatives thereof or molecules which bind to the same such epitope as the LO-CD2a monoclonal antibody.

Such other antibodies include by way of example and not limitation rat, murine, porcine, bovine, human, chimeric, humanized antibodies, or fragments or derivatives thereof.

The term "derivative" as used herein means a chimeric or humanised antibody, single chain antibody, bispecific antibody or other such antibody which binds to the same epitope (or a portion thereof) as recognized by the LO-CD2a monoclonal antibody.

The term "fragment" as used herein means a portion of an antibody, by way of example such portions of

antibodies shall include but not be limited to CDR, Fab, or such other portions, which bind to the same epitope or any portion thereof as recognised by LO-CD2a.

The term "antibody" as used herein includes polyclonal, monoclonal antibodies as well as antibody fragments, derivatives as well as antibodies prepared by recombinant techniques, such as chimeric or humanized antibodies, single chain or bispecific antibodies which bind to the same epitope or a portion thereof as recognized by the monoclonal antibody LO-CD2a. The term "molecules" includes by way of example and not limitation, peptides, oligonuclotides or other such compounds derived from any source which mimic the antibody or binds to the same epitope or a portion thereof as the antibody fragment or derivative thereof.

Another embodiment of the present invention provides for a method of treating a patient who is to receive or has received a graft transplant with an effective amount of at least one member selected from the group consisting of LO-CD2a antibody, or an antibody, or derivative or fragment thereof or molecules which bind to the same epitope (or a portion thereof) as the LO-CD2a antibody. The treatment is preferably affected with the whole or intact LO-CD2a antibody.

A monoclonal antibody of this invention as hereinabove described may be produced by techniques known in the art such as described by Kohler and Milstein (Nature 256, Pg. 495-497, 1975) as well as the techniques disclosed herein. The preparation of a monoclonal LO-CD2a antibody is described in more detail in Example 1 of this Application. As hereinabove indicated LO-CD2a antibodies may also be produced by recombinant techniques using procedures known in the art. The recombinant antibody may also be in the form of a chimeric antibody wherein the variable or CDR regions of a LO-CD2a rat antibody is combined with the constant region of an antibody of another species. Thus, for example, the monoclonal antibody may be humanized by combining the variable or CDR region of a rat LoCD2 monoclonal antibody with the constant region of a human antibody to provide a chimeric human-rat monoclonal antibody.

The antibody or molecule of this invention preferably: (i) binds to all T lymphocytes and also to null cells but not B lymphocytes as shown by two color staining of lymphocytes analyzed by flow cytometry (Figures 26 and 27); (ii) binds to all T cells (as determined by staining with the anti-CD3 antibody Leu4), all CD4 and CD8 positive cells as defined by Leu3a and Leu2b antibodies respectively and some lymphocytes which are CD3 negative (null cells); (iv) binds to null cells as corroborated by the staining of CD16 positive cells as detected with Leull, a marker for NK cells. (Figure 26); Staining of B cells, as defined by anti-CD19 binding, was not seen with LO-CD2a, (Figure 27). LO-CD2a antibody also preferably has the characteristic that the antibody binds to human null cells, and by double staining has a higher intensity of staining to human cells that are both $CD2^+$ and $CD4^+$ than to human cells that are both $CD2^+$ and $CD16^+$, and has a higher intensity of staining of human cells that are both $CD2^+$ and $CD8^+$ than to human cells that are both $CD2^+$ and $CD16^+$.

That Lo-CD2a binds to CD2 was confirmed by transiently expressing CD2 in COS cells.

COS cells were transiently transfected with the CDM plasmid containing the gene encoding for the entire CD2 molecule, as described in Peterson A. and Seed B., Nature Volume 329, 10/29/87, pp 842-846.

Transfection was accomplished by the DEAE-dextran method. Cells were harvested and stained with the anti-CD2 monoclonal antibody Leu5b (Becton-Dickinson) and LO-CD2a, with murine W632 an antibody to MHC class I as a positive control for staining and with the corresponding isotype-matched controls. Specificity of the reactivity was confirmed by assessing binding of the same panel of monoclonal antibodies on COS cells transfected with an irrelevant plasmid.

The staining pattern of these monoclonal antibodies on transiently expressed native CD2 (Figure 28) indicates that transfection with CD2 led to binding of both antibodies, supporting the ability of LO-CD2a to bind to CD2.

The preparation of LO-CD2a monoclonal antibody suitable for the purposes of the present invention should be apparent to those skilled in the art from the teachings herein.

An antibody or fragment or derivative thereof or molecule of the type hereinabove described may be administered in vivo in accordance with the present invention to inhibit the activation and proliferation of T-cells, and decrease the density of CD2 expression on the cell surface and thereby reduce the number of $CD2^+$ T lymphocytes.

Thus, for example, in an in vivo procedure, such LO-CD2a antibodies are administered to prevent and/or inhibit immune response and thereby inhibit T cell activation and proliferation.

An antibody or fragment or derivative thereof or molecule of the type herein above described may be administered ex vivo in accordance with the present invention to decrease the density of $CD2^+$ expression on the cell surface and thus reduce the number of $CD2^+$ cells of the donor cells. By way of example and not limitation, in an ex vivo procedure, such antibodies or fragments or derivatives thereof or molecules would be infused into donor bone marrow prior to transplantation to prevent the onset of graft versus host disease upon transplantation.

In such an in vivo or ex vivo technique, the antibody or fragment or derivative thereof or molecule will be administered in a pharmaceutically acceptable carrier. As representative examples of such carriers, there may be mentioned normal saline solution, buffers, etc. Such pharmaceutical carriers are well known in the art and the selection of a suitable carrier is deemed to be within the scope of those skilled in the art from the teachings contained herein.

The LO-CD2a antibody or other molecule of the present invention may be administered in vivo intravenously or by intramuscular administration, etc.

As herein above indicated, LO-CD2a antibody or other molecule of the present invention is administered in vivo in an amount effective to inhibit graft rejection. The term "an effective amount" for purposes of this Application shall mean that amount of monoclonal antibody capable of producing the desired effect, i.e., the inhibition of graft rejection or inhibition of the activation of T-cells. In general, such antibody is administered in an amount of at least 1 mg. It is to be understood that lower amounts could be used. In addition after the initial treatment, the herein above described amounts may be reduced for subsequent treatments, if any. Thus the scope of the invention is not limited by such amounts.

In accordance with the present embodiment, such antibodies are repeatedly administered in order to maintain the inhibition of T-cell activation and graft rejection. Thus, by way of example and not limitation, the antibody may be administered by intravenous infusion over a one to two hour period in amount of from about 1 mg/dose to about 50 mg/dose in a physiologically acceptable carrier suspension once or twice a day for a period of from about eight days or more, as needed. such treatment for graft rejection is preferably started at, or immediately prior to, or shortly after transplantation or when graft rejection occurs. The treatment could be given once or twice a day for as little as one or two days when started at the time of transplantation to induce a selective hyporesponsive state to the transplant. Such treatment for autoimmune diseases with respect to the administration of the antibody or fragment or derivative thereof or molecule in accordance with the present invention is begun when the attending physician has determined it is desirable to inhibit a pathological immune response.

The techniques of the present invention for inhibiting the activation of T-cells may be employed alone or in combination with other techniques, drugs or compounds for inhibiting the activation of T-cells or inhibiting graft rejection or graft versus host disease.

The invention will be further described with respect to the following examples, which are illustrative and which are not intended to limit the scope of the invention.

The cells, cultures, mAbs and mitogens used in the examples may be prepared and used by processes and procedures known and practiced in by those of ordinary skill in the art. The following is an example of a process or procedure that may be used for the preparation and use of the cells, cultures, mAbs and mitogene used in the examples which follow.

Cells and cultures

PBMC were obtained by Ficoll-Hypaque (Pharmacia, Sweden) sedimentation of heparinized blood obtained from the local Blood Donor Center. Isolated PSMC were resuspended in enriched medium: RPMI 1640 medium (Cibco, Belgium), supplemented with 100 U/ml penicillin, 100 μg/ml streptomycin, 20 mM L-Glutamine, and 20% pooled human AB serum or 15% heat-inactivated fetal calf serum. PBMC were cultured at $1x10^5$ cells/well in 96 U-well micro plates (Falcon) in a final volume of 200 μl of culture medium/well. Bidirectional HLC were performed with $1x10^5$ cells of each donor/well in the same volume of culture medium as noted above. All cultures were made in triplicate. Eight hours before the times indicated in the results, cultures were pulse-labelled with 2.0 μCi/well of $3_H$-T (Amersham, Belgium) 247.9 GBq/mmol; 6.7 Ci/mmol) and the radioisotope incorporated in cultures was quantified by liquid scintillation in a Betacounter (Beckman L5 6000 SE). The percentage of inhibition was calculated as follows: % Inhibition - [1- (mean cpm of tested culture/mean cpm control culture)] x100. All results are expressed as the mean of three independent cultures. Standard deviation was always less than 15% of the mean, except for those cases where these values are indicated on the graphics.

Cytofluorometric analyses were performed using a FACScan cytofluoro graf (Becton Dickinson) with Hewlett-Packard hardware equipped with the Consort 30 program. Independent analysis of staining of lymphocytes and blast-cells was possible using differential gating as defined by size and granularity. 25,000 events were analyzed for each sample. In these experiments, LO-CD2a final concentration was 200 ng/ml, except when indicated.

Mabs and Mitogen

LO-DRA and LO-Tact-1 (both FITC-labelled), are rat mAbs produced in our laboratory (op. cit. H. Dazin (Ed) 1990 p. 287). LO-Tact-1 is directed against the p55 chain of the IL-2 receptor (op. cit. H. Bazin Immunol. 1984 and Janszen, M., Buck, D. and Maino, V.C. in Leucocyte Typing IV White Cell Differentiation Antigens, W. Knapp (ED), Oxford University Press, 1989, p. 403). Mouse anti-human-CD2 and anti-CD3 mAbs (Leu-5b and Leu-4a-FITC-labelled) were obtained from Becton Dickinson (Belgium). Mouse anti-human-CD4 or anti-human-CD8 mAbs (phcoerythrine-labelled), and mouse IgG FITC- or phcoerythrine-labelled (negative controls) were obtained from Coulter. OKT3 (Ortho-Cilag, Belgium) was used at a final concentration of 100 ng/ml. Phytohemagglutinin A (PHA; Wellcome Labs, UK) and Concanavalin A (Con A; Calbiochem Co., USA) were used at a final concentration of 1 and 10 μg/ml, respec-

tively.

Biotinylation of LO-CD2a. The concentration of purified LO-CD2a was adjusted to 1 mg/ml in 0.1M sodium bicarbonate buffer, pH 8.4.NHS-biotin (Boahringer Mannheim 100S 960) was dissolved in DMSO at a concentration of 1.5 mg/ml. For each MAB, 0.1 ml of NHS-biotin solution was added. The mixture was rotated for 2 hrs. at ambient temperature. The reaction was completed by adding 0.1 ml of 2M tris-HCL, pH 8.0, for each ml of antibody (10 minutes at ambient temperature), followed by 1 ml of 1% BSA in phosphate buffered saline (PBS) for each ml of antibody. To remove free biotin, the solution was dialyzed overnight at 4°C in 1000 volumes PBS. Both the biotinylation reaction and the conjugated mAb were shielded from light by covering with aluminum foil.

Lysis of red blood cells (RBC). RBC were removed from whole blood by lysis with ammonium chloride. A 10X stock solution was prepared which consisted of 90g $NH_4Cl$, 10g $KHCO_3$, 370 mg EDTA, and $H_2O$ to a volume of 100 mls. Forty mls of 1X ammonium chloride was added to each 10 mls of blood and inoubated for 10 min. at room temperature. The mixture was then centrifuged at 1200 rpm for 10 min and the pellet resuspended in 10 ml PBS with 0.1% azide.

Staining of peripheral blood. Staining was carried out in round-bottom 96 well cluster plates (Costar #3790) at 4°C. For single color staining, ten µl of mAb was appropriately diluted in PBS containing 0,2 mg human immunoglobulin and added to each well. Red blood cell depleted blood was distributed into plates at a volume of 90 µl per well. Cells and mAb were mixed by gentle tapping and incubated 30 min. Fifty µl of cold PBS was added to each well and plates were centrifuged at 1900 rpms for 2 min. Supernatant was discarded by inversion and gentle flicking of the plate. Cells were dispersed by tapping the plate on the counter. The wash procedure was repeated twice by adding 200 µl of cold PBS, Ten µl of a 1/20 dilution of goat $F(ab')_2$ anti-rat lg-FITC was added to the dispersed cells in each well and incubated for 30 min. in the dark. Cells were washed by the addition of 180 µl of cold PBS to each well followed by centrifugation at 1900 rpms for 2 min. Supernatant was discarded, cells dispersed, and 200 µl of cold 0.5% paraformaldehyde was added to each well. Cells were transferred to tubes (Falcon #2054) and diluted to approximately 0.5 mls with 0.5% paraformaldehyde. Samples were evaluated on a Becton-Dickinson FAC8can machine using LYSIS II software.

Dual color staining was carried out by a similar protocol, After cells were incubated with the primary mAb and the FITC-conjugated anti-rat reagent, a 1/5 dilution of normal mouse serum was added to block any remsining sites on the anti-rat reagent. Following a 15 min. incubation (no wash), 20 µl of a PE-labeled mAb specific for a known CD determinant was added and incubated for 30 min. Cells were washed and fixed as described for single staining.

EXAMPLE 1

LO-CD2a is a rat (IgG2b-Kappa) anti-CD2 monoclonal antibody produced and characterized in our laboratory as indicated elsewhere (See the following references: Xia, H., Ravoet, A.M., Latinne, D., Ninanne, J., De Bruyere, M., Sokal, G. and Bazin, H., in H. Bazin (Ed), Rat Hybridomas and Rat monoclonal Antibodies, CRC Press, Inc., Boca Raton, Florida 1990, p.309 and Ravoet, A.M., Latinne, D., Seghers, J., Manouvriez, P., Ninanne, J., DeBruyere, M., Bazin, H. and Sokal, G.- in H. Bazin (Ed) Rat Hybridomas and Rat Monoclonal Antibodies, CRC Press Inc., Boca Raton, Florida, 1990, p. 287). LO-CD2a was purified from ascitic fluid by immunoaffinity chromatography taking advantage of the allotypic difference existing between the immunoglobulins of the rat receiving the producing hybridoma and the mAb secreted by the latter (Bazin, H., Cormont F. and DeClercq, L.. J. Immunol. Method. 1984, 71:9). It recognizes the total population stained by the mouse mAb Leu-5b (FITC-labelled) Figure 1 and roughly 90% of the population marked by the mouse T11 (Rhodamine-labeled) mAb ((data not shown) (see FIG. 1)). The epitope recognized by LO-CD2a on the CD2 molecule, is different from the epitopes recognized by the anti-CD2 mouse mAbs Leu-5b and T11 (Figure 2).

EXAMPLE 2

LO-CD2a exhibits modulatory but not mitogenic effects on PBMC

In order to determine the effects of the rat mAb LO-CD2a on resting lymphocytes, PBMC were incubated in the presence of increasing concentrations of this mAb. As can be seen in Table 1, PBMC incubated during 6 days in the presence of LO-CD2a show no significant variations in the rate of $^3$H-T incorporation as compared with control cultures. Cell viability at the end of this period was variable but averaged around 80% as assessed by trypan blue exclusion. When resting PBMC were incubated in the presence of LO-CD2a, there was no significant variation in the phenotypic expression of several membrane markers, as assessed by flow cytometry. Cellular markers of resting mature T-cells (such as CD3, CD4 and CD8) show the same pattern of variation during 6 days of culture in the presence or in the absence of LO-CD2a, and activation molecules such as CD25 (IL-2R/p55) are not expressed in these experimental conditions or are not modified by LO-CD2a as is the case of DR antigenic determinants. (Figure 3)

When PBMC were incubated for 6 days in the presence of LO-CD2a, a significant decrease was observed in the percentage of Leu-5b+ gated lymphocytes. (Figure 4) The percentage of CD4-and CD8- lymphocytes is not affected during a 6-day period of cultures by the presence of LO-CD2a, indicating that the observed decrease of CD2-bearing lymphocytes cannot be attributed to an elimination of these cells but rather to a disappearance of the CD2 molecule or to a conformational change in this glycoprotein produced by the binding of LO-CD2a.

In order to verify if the observed decrease in Leu-5b-lymphocytes was due to a conformational change of CD2 or to a disappearance (internalization or release) of this molecule after the binding of LO-CD2a, PBMC were cultured in the presence of 500 ng/ml of LO-CD2a and analyzed through 6 days in flow cytometry using Leu-5b (FITC-labelled), T11-RD1 (Rhodamine-labelled) and MARK-3 (FITC-labelled) . As shown in Figure 5a, Leu-5b or Tl1 mabs are not able to bind to PBMC after 2 to 4 days of culture in the presence of LO-CD2a. Under these conditions, the mouse anti-rat kappa chain mAb MARK-3 labelled 50% of cells at day 6 of culture indicating that only 35% of the original CD2-bearing cells show no LO-CD2a on their surfaces, yet Leu5b-FITC and T11-RD1 staining have decreased markedly at day 2. This suggests that a conformational alteration of CD2 rendering the epitope of Leu5b and T11 unavailable for binding occurs in response to LO-CD2a.

The analysis of the mean fluorescence of CD2+ cells indicated that the density of expression of this marker decreased with time in the presence of LO-CD2a. The same phenomenon was observed whether Leu-5b FITC-labelled or LO-CD2a (revealed by MARK-1 FITC-labelled) were used to detect the CD2+ lymphocytes. Aliquots of the same PBMC were cultured in parallel in the presence of Leu-5b (commercially available mAb, dialyzed against PBS, 1:2 final dilution in culture medium). As shown in Figure 6b, in those experimental conditions all the CD2-bearing cells are coated by the Leu-5b mAb (as revealed by goat anti-mouse-FITC). Staining by T11-RD1 was markedly reduced, whereas a smaller, slower decrease was observed in the percentage of cells presenting the epitope recognized the LO-CD2a-FITC mAb. Taken together these results indicate that CD2 molecules have partially changed their conformation in response to LO-CD2a, and that a slow modulation of CD2/LO-CD2a occurs. As determined by flow cytometry, the number of blast-cells present in unstimulated PBMC at day 0 of culture, remained constant (200 to 300 blast-cells over 25,000 events) or decreased during the period of study (both in the presence or in the absence of the rat mAb), indicating that no blastogenesis was induced by the presence of LO-CD2a.

<u>LO-CD2a inhibits MLR</u>

When MLC were performed (over a period of 6 days) in the presence of increasing concentrations of rat mAb, a significant inhibition of the MLR (as measured by $^3$H-Thymidine ($^3$H-T)-incorporation), was observed at concentrations of mAb as low as 125 ng/ml. In Figure 6a, we show a typical example of a dose-response curve of MLR inhibition by LO-CD2a. As can be seen in this Figure 6a, LO-CD2a induces 80% inhibition of MLR (6 days of culture) at 250 ng/ml and this percentage of inhibition remains almost constant or higher than 80% over a wide range of concentrations (0.25 to 5.0 μg/ml of mAb). Figure 6b shows a time-course of the inhibitory effects of different concentrations of LO-CD2a on MLR from day 0 to day 6 of culture. A typical example of $^3$H-T-incorporation on MLC (in the presence or in the absence of LO-CD2a) is shown in Figure 6c, where LO-CD2a was added at a final concentration of 200 ng/ml.

In Figure 6d we show the effects of LO-CD2a on MLR, when this mAb (at 200 ng/ml) is added at varying times after initiation of MLC. More than 90% inhibition of MLR (as measured by $^3$H-T incorporation) is obtained when this mAb is added at day 0, and this inhibitory effect is still present (45% inhibition in this example) when LO-CD2a is added 4 days after the beginning of MLC. Similar results (not shown) were obtained with higher concentrations (from 0.20 to 5.0 μg/ml) of LO-CD2a.

LO-CD2a blocks the pathway of IL-2R expression

When cytofluorograph analyses were performed on the lymphoblast subset of a MLC (Figure 7a and b), the following observations were made: a) the number of blast cells (around 300-500 blast cells of 25,000 events analyzed) already present at initiation of MLC rose sharply from day 4 to day 6 in control cultures (more than 1200 blast cells from 25,000 events analyzed); b) in MLC performed in the presence of LO-CD2a, there was no significant variation in the number of blast cells during the whole period of culture and at day 6 the number of blast cells is always lower or nearly the same as the initial number of blasts at day 0 (Figure 7a); c) the percentage of CD25 blasts rose sharply among cells incubated without LO-CD2a (Figure 7b); d) this percentage remains below 20% in the small number of blasts from the MLC incubated in the presence of mAb (Figure 7b), and the mean fluorescence (as a measure of CD25 expression) decreased by 75% as compared with blasts present in control cultures (results not shown); e) in the absence of mAb the percentage of CD3- blasts remains constant during the first 4 days of culture (Figure 7b) and on day 6 the percentage of CD3-cells increased to 90%, while in the presence of LO-CD2a the percentage of CD3- rises slowly to reach only about 45% at day 6. These results indicate that the presence of LO-CD2a inhibits the entrance of these cells in the pathway of activation characterized by the expression of IL-2 receptor (CD25). The number of CD2+ blasts

remains constant or decreases in the presence of LO-CD2a, and the density of expression of this membrane marker is strongly diminished under these conditions (data not shown).

When phenotypic analyses were performed through 6 days on the resting (non blast) lymphocyte subset of MLC, results similar to those described in Figure 3 were obtained: in the presence of LO-CD2a, no significant variation could be detected in the percentage of CD3+, CD4+ or CD8+ lymphocytes, as compared with control cultures (Figure 8); no CD25 expression (activation marker) could be detected whether in the presence or in the absence of LO-CD2a during 6 days of culture. These results suggest that LO-CD2a has a very weak, if any, effect on the resting subset of T-lymphocytes in MLC; that is to say, in T-cells not committed in the process of activation. At the same time, as shown in Figure 4b, LO-CD2a induces a significant decrease in the percentage of CD2+ lymphocytes during MLC. This phenomenon is accompanied by a drastic decrease of the mean fluorescence as detected by the Leu-5b mAb.

LO-CD2a can block the pathways of T-cell activation dependent on the TcR/CD3 complex or on mitogen receptors.

When LO-CD2a was added to mitogen-activated PBMC, a significant inhibition of $^3$H-T incorporation was observed. In one of three experiments of PBMC incubated with mitogens (OKT3, ConA and PHA) in the presence or in the absence of LO-CD2a added either at time 0 or 1 hour after the start of cultures. In the first case, mitogens were added 1 hour later. When LO-CD2a was added 1 hour after the initiation of culture, mitogens were added at time 0. This was done in order to know whether, preincubation of PBMC with mitogens or LO-CD2a could trigger events that could be affected by the addition of the second reagent. Cultures were harvested at 96 h, after a pulse-labelling (6 h) with $^3$H-T. More than 50% inhibition of $^3$H-T. incorporation was observed in the presence of LO-CD2a, whether it is added first or after mitogens. (Figure 9) The same effect was observed when cells were harvested 4 days after the onset of MLC and exposed to mitogens (results not shown). A drastic decrease in $^3$H-T incorporation was observed two days after the onset of MLC, in those cultures receiving both the mitogen and LO-CD2a, as compared with the same cultures receiving only mitogen (results not shown). Preincubation of MLC with LO-CD2a before addition of mitogen, lowered the $^3$H-T-uptake to values comparable with MLC without OKT3.

LO-CD2a was also able to inhibit mitogen induced proliferation if added one day after the initiation of mitogen induced proliferation. The results of experiments performed with two donors are shown in Figure 10. PMBC were incubated along with mitogens (OKT3, ConA and PHA). In these experiments, LO-CD2a was added either at time 0 (Day 0), 24h (Day 1) or 48h (Day 2) after the start of the cultures. The inhibition of proliferation in response to OKT3 and ConA by LO-CD2a was significant if added 24 hours after the addition of mitogen at time 0.

EXAMPLE 3

Inhibition of Natural Killer Cell (NK) Activity.

PBMC were isolated from heparinized blood by Ficoll Hypaque Sedimentation. After washing, the effector cells, suspended in enriched medium, were incubated overnight at a concentration of 1X10$^6$/ml in a Falcon plate to eliminate the monocytes (by adherence).

The target cells (K562 cell line) were labeled by overnight incubation with $^{51}$chromium $^{51}$Cr (0.9 ml of a cell suspension at 3x10$^6$/ml + 0.02 ml from a solution of 5mCi/ml$^{51}$Cr, Amersham).

After a 16-hour incubation, effector and target cells were washed four times, counted and incubated in a 96 V bottom microplate at different E/T ratios: 200/1 (100 ul of a suspension of 4x10$^6$/ml effector cells with 100 ul of 2x10$^4$/ml target cells) 100/1, 50/1 and 25/1.

After a four-hour incubation, the $^{51}$Cr release was measured by counting 100 µl supernatant from each well in a gamma counter.

Maximum (target cells + HCLIN) and spontaneous release (target cells + enriched medium) were used to calculate the specific lysis:

$$\%\text{Specific lysis} = \frac{\text{test- spontaneous release}}{\text{maximum - spontaneous release}} \text{ X } 100\%$$

Inclusion of LO-CD2a at 5, 1 and 0.5 ug/ml in the NK assay with two normal donors (Figures 10a and 10b) led to an inhibition of cytotoxicity of approximately 50% with all tested concentrations of antibody and over all tested E/T ratios. This is in comparison with essentially complete inhibition of proliferation in the MLR at doses at or above 0.25 ug/ml.

EXAMPLE 4.

IN VIVO STUDIES IN NON-HUMAN PRIMATES

MATERIAL AND METHODS

Monoclonal Antibodies

MARK3-FITC is a mouse mAb directed against the rat 1g kappa 1b allotype conjugated with FITC. MARG2b-biotin is a mouse anti-rat IgG2b immunoglobulin mAb conjugated with biotin. These two mAbs were produced and labeled in our laboratory. For immunofluorescent tests they were used at a final concentration of 2.5 μg/ml. Leu-5b-FITC (Becton-Dickinson) and T11 Rhodamine (COULTER) are two mouse anti-human CD2 mAbs. T4- and T8-Rhodamine-labeled (COULTER) are mouse anti-human CD4 and CD8 mAbs respectively.

Phenotype Analysis

Anti-human T-cell mAbs (anti-CD2, -CD4, -CD8, see above) were added to 100 μl samples of whole blood and incubated at 4°C for 45 min. Red blood cells were lysed with a Tris-buffered ammonium chloride-lysing buffer (144 mM $NH_4CL_1$/17 mM Tris, pH 7.2) and lymphocytes were washed with PBS/2% FCS/0.2% NaN3. For detection of non-labeled mAbs, a second mAb (FITC- or biotin-conjugate) was added to a final concentration of 2.5 μg/ml. After 45 min. incubation at 4°C, cells were washed with PBS/FCS/NaN3. For biotinylated mAbs, a further incubation (15 min) with Streptavidin-Phycoerythrin conjugate was done. Labeled human or monkey lymphocytes were resuspended in a 2% formalin solution and analyzed in a FACSan cytofluorometer (Becton-Dickinson) equipped with the lysis II program for gating lymphocytes as a function of size-vs-granularity. As a control for nonspecific staining, aliquots of cells were incubated with FITC- or Phycoerythrin-conjugated mouse lgs (Coulter).

Level of circulating Abs

LO-CD2a in serum was quantified by ELISA using a mouse anti-rat IgG2b mAb (MARG2b-8, produced in our laboratory ) as first layer (coating) and a mouse anti-rat kappa chain (MARK-3) mAb coupled to horseradish peroxidase for detection. Briefly, microtiter plates (Falcon) were incubated overnight with 100 μL/well of MARG2b-8 (5μg/ml) and unoccupied sites on plastic were saturated with PBS containing 5% powdered milk (bovine). After 1 h incubation at room temperature, plates were washed with PBS with 0.1% Tween-20, and incubated 1 h with 100μl/well of diluted monkey or human serum. After washing out unbound material, plates were incubated 1 h with 100 μl/well MARK3-peroxidase (2 μg/ml in PBS). After washing again, plates were incubated with OPD (o-phenylenediamine dihydrochloride, 0.4 mg/ml, Sigma Chemicals), in citrate-phosphate buffer containing 0.03% $H_2O_2$. The colored reaction product was detected at 492 nm. A standard curve was made in parallel with a known concentration of purified LO-CD2a serially diluted in a pool of control monkey or human serum.

The detection of monkey or human anti LO-CD2a antibodies was performed by ELISA using 96 well micro plates coated with LO-CD2a (5 μg/ml). Anti-LO-CD2a human or monkey antibodies bound on the plates, were revealed by horse-radish peroxidase labeled rat anti-human IgM (LO-HM-7) or IgG (LO-HG-22) mAbs.

A. CYNOMOLGUS MONKEYS

One Cynomolgus monkey received 10 mg/day of LO-CD2a for three consecutive days. The monoclonal antibody was well tolerated.

A lymphocyte depletion was observed after the first injection but a very low additional depletion was obtained after the 2d and 3d injections.

The second monkey received 20 mg/d for 10 days. The mAb was also well tolerated. No side effects were observed after dosing in that the animals were active, alert, eating well with no evidence of nausea or gastrointestinal disturbance.

The lymphocyte counts and cell populations in the second monkey are summarized in Figures 19 and 20. The NK activity was slightly reduced after the 10 injections (Figure 21). The circulating levels of MAb were very high (Figure 22) and immunization occurred at the end of the treatment (Figure 23).

B. BABOON

The experiment described here was undertaken to determine the tolerance of a baboon to LO-CD2a, to analyze the effects of this mAb on some of the membrane markers of baboon lymphocytes and to determine the half-life of LO-CD2a in serum.

Staining of baboon cells with LO-CD2a results in <20% positive cells at a mean fluorescence intensity significantly lower than that of stained human cells, consistent with modest cross reactivity with baboon cells.

The study was done on a male baboon (papio mormon) weighing 8.8Kg. Before each injection of LO-CD2a the monkey was anesthetized; the first time with Ketaler (2 ml) and Prazine (0.5 ml), the second time with Ketaler only and the subsequent times with ketaler and Prazine (0.3ml). LO-CD2a was injected intravenously (i.v. in 10 min.), diluted in 100 ml of physiological serum. For phenotypic analysis of lymphocytes and measurement of circulating antibodies (injected LO-CD2a and newly formed anti-LO-CD2a antibodies, and preexisting cross-reacting baboon anti-LO-CD2a antibodies), blood samples (10 ml) were taken in two tubes. The tube for lymphocyte typing contained EDTA. Samples were taken prior to the first treatment to determine baseline levels.

The first does (10 mg) of LO-CD2a was administered on day 0 of the study; the four following doses (10 mg/dose) were administered on days 7, 8, 9, and 10. Blood samples were taken a few minutes after each LO-CD2a dose. On days 7 and 9 a supplementary blood sample (in an EDTA-containing tube) was also taken before the LO-CD2a injections. Blood samples were taken on days 1, 2, 11, 12, 13, 16 and 24.

No abnormal reactions in activity or feeding habits were observed during LO-CD2a injections or throughout the period of study. The weight of the animal remained around the 8.8 Kg measured at day 0 (see table below).

| Weight of baboon (in Kg) from day 0 to day 24 | | | | | | | | | |
|---|---|---|---|---|---|---|---|---|---|
| Day | | 0 | 7 | 8 | 9 | 10 | 12 | 13 | 16 | 24 |
| Weight | 8.8 | 8.9 | 9.1 | 9.1 | 8.8 | 9.0 | 9.1 | 8.9 | 8.9 | |

ANALYSIS OF PHENOTYPE AND CIRCULATING mAb

The fluorescent staining of this baboon's peripheral blood lymphocytes revealed some interesting features:

a) Under the effect of LO-CD2a the CD2-positive subset of lymphocytes decreased significantly (as revealed by two different anti-CD2 mAbs) at the end of the 5th dose of LO-CD2a, that is at the time of a maximal accumulation of mAb in blood (see Figures 17a and 17b). Given that the CD4+ and the $CD8^+$ subsets of lymphocytes do not decrease during this period (Figure 19), and because the CD4+ and CD8+ cells comprise most of CD2 bearing lymphocytes, the decrease in $CD2^+$ cells indicates that it is the membrane marker expression that is decreasing, rather than the lymphocytes.

As can be seen in (Figure 17a), a slight decrease of CD2+ (Leu-5b$^+$ or T11$^+$) positive lymphocytes is observed after the first dose of LO-CD2a. Two days after this first dose, the level of CD2 positive cells (Leu 5b$^+$ of T11$^+$) rose to the starting values.

At the end of the four 24-hour spaced doses of LO-CD2a (days 7-10) the percentage of CD2 positive cells (Leu 5b$^+$ or T11$^+$) decreased sharply and began to rise slowly 3 days after the end of the LO-CD2a administration.

b) At the same time, the percentage of LO-CD2a positive cells, that is, the percentage of cells bound by the circulating mAb rose to 22% after the 2nd dose of LO-CD2a (day 7) and then decreased as did the $CD2^+$ cells revealed by the anti-CD2 mAbs Leu-5b and T11 (Figure 17). The decrease of LO-CD2a+ cells was revealed by the MARK-3FITC mAb (Figure 18).

The decrease of LO-CD2a+ cells was determined by detection of the LO-CD2a present on cells as detected by MARK3-FITC or by the MARG2b-8-biotin conjugated mAbs (Figure 18a). The same phenomenon was observed if cells were first incubated with LO-CD2a at 2.5 µg/ml to saturate all the sites unoccupied by circulating mAb. LO-CD2a was detected by MARK-3-FITC or MARG2b-8-biotin (Figure 18).

c) As can be seen in (Figure 19), the T4 positive subset of baboon lymphocytes showed a moderate rise during days 9 to 12 after when the percentage of $T4^+$ lymphocytes returned to its initial value. Concomitantly with the rise in $T4^+$ cells, the percentage of T8 positive lymphocytes rose from day 9 to day 11. After that day this percentage returned to initial values.

d) The levels of circulating mAb LO-CD2a decreased to background values 3 days after the first injection (see Figure 17b). When LO-CD2a is applied in four short-time spaced doses (days 7 to 10), the levels of serum LO-CD2a (around 3.7 mg/ml, maximal value in this period) decreased slowly after the last dose (days 10 to 16), indicating a relatively long half-life of the Ab in this animal model. No baboon anti-LO-CD2a antibodies were detected in the blood samples collected on days 11, 12, 13, 16 and 24.

CONCLUSION

LO-CD2a seems to be well tolerated by non-human primates, as demonstrated by the absence of apparent reactions in cynomolgus monkey baboon. LO-CD2a seems to have a relatively long half-life in the baboon. Twenty-four hours after the first dose of LO-CD2a (day 1 in Figure 24b), 50% of the maximal detectable level of MAb was still present in serum. Three days after the last dose of LO-CD2a (day 13 in Figure 24b), 50% of the maximal detectable level of mAb was still present in serum.

On the other hand, the decrease in the percentage of CD2 positive lymphocytes followed by a slow rise of this percentage of cells, show the same kinetics observed in human PBMC mononuclear cells cultured in the presence of LO-CD2a.

Example 5

Patients treated with LO-CD2a

Patients treated with LO-CD2a on a compassionate basis

PATIENT #1 (Mb.E.)

This was a female patient with chronic pyelonephritis, who was treated with a renal allograft for end-stage renal failure. A rejection crisis occurred and was treated with 10 days of OKT3. The creatinine level dropped from 2 to 1.4 mg/dl. Approximately four (4) months later a rejection crisis was diagnosed by a creatinine level of 2 mg/dl and a biopsy indicating moderate rejection. The patient was treated with 1.5 g Solumedrol and a course of ATG for the following eight (8) days at which time the creatinine level was 1.65 mg/dl. Seven days after treatment a biopsy was performed and indicated cellular rejection and moderate vascular rejection. Two days after the biopsy (day 0) the patient was anuric with a creatinine level of 2.4 mg/dl. That same day the patient received 10 mg of LO-CD2a, 1.5 g Solumedrol, plus 1 g Polarimin (an anti-histamine) and 1 g Dafalgan (acetaminophen). No side effects were noted. By the end of 23 hours, the patient produced 700 ml of urine and the creatine was 2.72 mg/dl. For the next 9 days she received 10 mg/day of LO-CD2a. The patient left the hospital without a follow-up biopsy at that time, Day 11.

Measurement of serum creatinine level during ATG treatment and during the following LO-CD2a treatment indicated the creatinine level rose despite ATG treatment and fell and stabilized with LO-CD2a (Figure 27).

The leukocyte count fell from a high of 10,000 to 2,000 during the treatment with LO-CD2a and continued to fall until the last measurement on day 21 (Figure 20). The lymphocyte count was low and variable during the period of observation.

The serum levels of LO-CD2a rose to peaks of 2.0-3.0 μg/ml immediately following each treatment and fell to lows of approximately 1.0 μg/ml between each treatment (Figure 21). With the last treatment on day 9, the level fell by 50% in 24 hours and to 0 by day 14. This patient returned to the clinic on day 40.

The patient's creatinine level was 2.27 on day 40, 2.48 on day 50 and rose to 3.11 by day 66 at which point a biopsy was obtained, with the initial report consistent with severe cellular rejection and interstitial hemorrhage (see below). The patient was treated with 150R of irradiation to the kidney, 3 x 125 mg Solumedrol, while continuing on maintenance therapy of cyclosporin plus 12.5 mg/day of steroids. The creatinine level continued to rise during the subsequent period. On day 70 the creatinine level was 3.3; day 80, 5.63; day 84, 8.35. By day 86 the creatinine level was 10.8 and a transplant nephrectomy was performed on day 88. This patient's compliance with maintenance immunosuppression during the period between her discharge on day 10 and her biopsy on day 66 is in question and the loss of the kidney despite the evidently successful rescue must factor in the uncertain compliance.

(ii) PATIENT #2

The patient was a 38 year old male who was Hepatitis $C^+$. He had received a renal allograft for the treatment of end stage renal failure due to chronic interstitial nephropathy. One year and three months later, he underwent a transplant nephrectomy due to acute cellular and vascular rejection resistant to a course of OKT3.

One year and ten months from the transplant nephrectomy, he received a second renal allograft. Three days later his creatinine level was 1.4 mg/dl. Three days later he received 500mg Solumedrol; the patient's creatinine later that day was 1.8 mg/dl. On the following day he received 500 mg of Solumedrol; creatinine was 3.25 mg/dl. The following day he received 500 mg Solumedrol; his creatinine was 2.95 mg/dl. Three days later his creatine level was 2.3 mg/dl and he underwent a biopsy which demonstrated 3 plus cellular rejection. Three days later he received 10 mg LO-CD2a, plus 200 mg Solumedrol. Polaramine and Dalfagan. Side effects observed were limited to sleepiness; no hyperthermia or hypertension were noted. For the next 9 days he received daily treatments of 10 mg of LO-CD2a. The day following

the end of such treatments a biopsy showed no signs of rejection.

The patient tolerated the course of LO-CD2a well with no evidence of clinical side effects, including no fever or hypertension with any dose. Routine hematological and clinical chemistry laboratory tests (including LFTs) obtained during the course of treatment demonstrated no alterations attributable to the administration of the antibody, except for a decrease in the lymphocyte count from 290/cubic mm to a low of 100/cubic mm and the reduction in creatinine level associated with resolution of the rejection crisis (from 2.7 mg/dl at the initiation of treatment with LO-CD2a to 1.10 at the end of the course).

Figure 22 shows the serum creatinine level of this patient, as falling from 2.5 to approximately 1.0 on the days following treatment with LO-CD2a. The patient was lymphopenic prior to and during treatment and the leukocyte count showed no dramatic alteration with treatment (Figure 23). In this patient the serum levels of LO-CD2a did not rise above 2.0 μg/ml after each treatment and fell to lows of 1.0 to less than 0.25 μg/ml (Figure 24). Eight months after the first treatment with LO-CD2a the patient was doing well with normal renal function and no evidence of recurrent rejection.

Patient 2 - Biopsy #1 - Diagnosis: Indeterminate.

The biopsy contained about 20 glomeruli which are unremarkable. There was a sparse mononuclear infiltrate with a minor degree of interstitial edema. Only minor degrees of tubular invasion were found and no vascular lesions. These findings are insufficient for the diagnosis of acute cellular rejection. There were rare mononuclear cells in small arteries, which were suspicious, but did not meet the criteria for the diagnosis of rejection.

Patient 2 - Biopsy #2 Approximately 2 weeks after the first biopsy - No diagnostic abnormality recognized. The biopsy looked similar to the previous biopsy and contained about 10 glomeruli. The infiltrate was very sparse and no vascular lesions were identified.

(iii) PATIENT #3

The patient was a 19 year old with von Willebrand disease who received a renal allograft for the treatment of end stage renal failure due to chronic pyelonephritis. The transplant was removed on 17 days later due to acute vascular rejection with secondary hypertension after failure of a 6 day course of OKT3.

Five and one-half months later he received a second renal allograft. Ten days later his creatinine level was 6 mg/dl. The next day the creatinine level was 7 mg/dl and a biopsy indicated 3 plus cellular rejection and vascular rejection (proliferative endarteritis without necrosis or thrombosis). That same day he received 10 mg of LO-CD2a, 40 mg Solumedrol and Polaramine and Dafalgan. No side effects were observed. For the next 9 days he received daily treatments of 10 mg of LO-CD2a, with no other drugs and no side effects. Two days after completion of the treatment, his creatinine level was 1.75 mg/dl and a biopsy indicated no sign of acute rejection, with interstitial necrosis and one focal spot of chronic rejection.

No clinical side effects (alteration in BP or temperature) were observed. Routine hematological and clinical chemistry laboratory tests (including LFTs) showed no changes attributable to administration of the antibody except the decrease in creatinine level associated with the resolution of the rejection crisis (from 7.10 mg/dl on the initiation of treatment with LO-CD2a to 1.75 mg/dl at the end of the 10 day course). The lymphocyte count was 340/cubic mm prior to treatment and fell to a low of 220/cubic mm during treatment, rose to 690/cubic mm 9 days after cessation of treatment with the LO-CD2a and had risen to 1000/cubic mm 23 days after the end of treatment.

The leukocyte count in this patient was not significantly altered by treatment (Figure 25). The serum creatinine level fell dramatically with treatment (Figure 25). Seven months after the first treatment with LO-CD2a the patient was doing well with normal renal function and no evidence of recurrent rejection.

Patient 3 - Biopsy #1 - Diagnosis: Severe cellular rejection affecting small arteries and to a lesser degree the interstitium and glomeruli.

An arcuate sized artery showed a marked mononuclear infiltration of the intima with disruption of the elastica. There was sparse infiltrate in the interstitium, which occasionally invaded tubules. The interstitium showed diffuse, mild interstitial edema. There were about 7 glomeruli present. These show hypercellularity with mononuclear cells and endothelial swelling. Overall, this pattern was diagnostic of severe, accute cellular rejection.

Patient 3 - Biopsy #2 Approximately 2 weeks after the first biopsy - Diagnosis: Consistent with treated rejection.

The biopsy showed a few small arteries, which show intimal fibrosis sometimes with a mucoid material but a very minimal cellular infiltrate. The interstitium showed a fine diffuse fibrosis and a minimal mononuclear infiltrate.

Tubules were locally atrophic but otherwise unremarkable. There was no evidence of active cellular rejection.

(iv) PATIENT #4

The patient who was suffering from severe graft versus host disease (severe skin, gut, renal and CNS toxicity resistant to high dose prednisone) after an allogeneic bone marrow transplant received 12 days of LO-CD2a at 10 mg/day. His symptoms improved; renal function returned to normal, diarrhea ceased, skin improved and confusion resolved. Four days after the antibody was stopped the symptoms recurred and the patient died despite the initiation of a second course of antibody.

LO-CD2a thus could be used to reverse ongoing immune responses to foreign tissues (allogeneic and xenogeneic, since it inhibits the xeno MLR as well as the allo MLR). The antibody would be given by i.v. infusion once or twice a day for 10 days to 14 days. It may also be used prophylactically to prevent activation of T cells as part of the induction protocol immediately following organ transplantation.

Although the present invention, in a preferred embodiment, is directed to inhibition of graft rejection, it is to be understood that the scope of the invention is not limited thereto and is generally useful for the inhibition of T-cell activation for any and all purposes.

Example 6

**Construction and Expression of Chimeric Antibody**

**A. Cloning and Sequencing of $V_H$ and $V_L$ of LO-CD2a**

Total RNA was isolated from the cell line LO-CD2a (ATCC HB 11423) according to the method of Chergwin (Biochemistry, 18:5294, 1979). mRNA was then prepared using The oligotex-dT mRNA kit (Qiagen, Chatsworth, CA). Approximately 200-300 ng mRNA was reverse transcribed using the RNA-PCR kit from Perkin-Elmer Cetus (Norwalk, CT). The reaction was carried out at 42°C for 1 hour. Oligonecleotide primers required for amplification of $V_H$ and $V_L$ genes were chosen using the following references: 1) Sequences of Proteins of Immunological Interest, Kabat et al., 5th ed., 1991, 2) Orlandi et al., Proc. Nat'l. Acad. Sci., (USA) 86:3833-3837 (1989).

$V_L$ sense

```
               Sma 1    #1  2   3   4   5   6   7   8
5'                                                    3'
           AA CCC GGG GAC ATT CAG CTG ACC CAG TCT CAA
```

$C_L$ antisense

```
               Sal 1  #115 114 113 112 111 110 109
5'                                                3'
           CA GTC GAC TAC AGT TGG TGC AGC ATC AGC
```

$V_H$ sense

```
               Sma 1    #1  2   3   4   5   6   7   8
5'                                                    3'
           AA CCC GGG GAG GTC CAG CTG CAG CAG TCT GG
```

$CH_1$ antisense

```
              Sal 1   #124 123 122 121 120 119
5'                                          3'
           AAG TCG ACC CAG TGG ATA GAC CGA TGG
```

The numbers refer to amino acid residues, as shown in Kabat, et al., 1991.

Polymerase chain reactions (PCR) were carried out in a Perkin-Elmer DNA Thermal Cycler 480 using the following conditions: 5 minutes at 94°C, 30 cycles consisting of 1 minute at 94°C, 2 minutes at 60°C, and 2 minutes at 72°C. This was followed by 5 minutes at 72°C. DNA fragments were gel purified from 1% agarose using the Qiaex gel extraction kit (Qiagen, Chatsworth, CA). The fragments were then blunt-ended according to the method of Kanungo and Pandey, BioTechniques, 14:912-913 (1993) and ligated into the Sma I site of Bluescript KSII$^+$ (Stratagene, La Jolla, CA). Multiple clones were sequenced by the dideoxy chain termination method using the Sequenase ™ T7 Polymerase Kit (U.S. Biochemical, Cleveland, OH).

Due to the potential error rate inherent in PCR,at least three separate reactions were performed. The most

commonly observed sequences for LO-CD2a $V_L$ and $V_H$ genes are shown in Figures 29a and b and 30a and b, wherein Figure 29a shows the nucleotide and amino acid sequences of the LO-CD2a $V_L$ chain including the leader sequence from MRC vector hcmv-vllys-kr-neo and Figure 29b shows the nucleotide and amino acid sequences of the LO-CD2a $V_L$ chain including the leader sequence from the Lo-CD2a gene; Figure 30a shows the nucleotide and amino acid sequences of the LO-CD2a $V_H$ chain including the leader sequences from MRC vector hcmv-Vh-Lys-gammal-neo; and Figure 30b shows the nucleotide and amino acid sequences of the LO-CD2a $V_H$ chain including the leader sequences from the LO-CD2a gene.

**B. Insertion Into Vectors for Transient Expression**

Two vectors were licensed from The Medical Research Council (MRC) in London for expression of chimeric light and heavy chains of LO-CD2a respectively. The 9.2kb light chain vector (hcmv-vllys-kr-neo) contains the genomic clone of the human kappa constant region and humanized $V_L$ domain of anti-lysozyme as a Hind III-Bam HI fragment. The 8.6kb heavy chain vector (hcmv-VhLys-gammal-neo) contains the genomic clone of human $\gamma$1 constant region and the reshaped $V_H$ domain of anti-lysozyme as a Hind III-Bam H1 fragment. These vectors are more fully described in Maeda, et al., Hum. Antibod. Hybridomas2:124-134, (1991).

Since DNA fragments containing the native signal peptides were unavailable, the V regions of LO-CD2a were cloned behind the signals already present in the MRC vectors. The light chain V region with signal fragment was constructed from two fragments, each derived from a separate PCR reaction as follows: Reaction 1: The DNA template was the MRC light chain vector. The fragment amplified contained the signal peptide plus a portion of framework (FR) 1. The two oligonucleotides used were:

Hind III

**5'VLlyssig (sense): 5' CCGCAAGCTTCATGGATGGAG 3'**

TthIII

**3'VLlyssig (antisense):**

**5'GCTGCTTGGGGACTGGGTCAGCTGGAT 3'**

The antisense primer contained the FR 1 sequence of LO-Cd2a, not that found in the MRC vector for anti-lysozyme. The PCR reaction produced a 0.15Kb Hind III -Tth III fragment.

Reaction 2: The DNA template was the LO-CD2a $V_L$ clone in Bluescript. The fragment amplified included LO-CD2a FR 1 (from the Tth III site) to the end of FR 4. The 3' untranslated region found in the MRC light chain vector was added to the 3' end of LO-CD2a using the antisense oligonucleotide. The 2 oligonucleotides used were: 5' $V_L$ LO-CD2a (sense):

Tth III

**5'ATTCAGCTGACCCAGTCTCCA 3'**

**3'VL LO-CD2a (antisense):**

BamHI

**5'GATCGGATCCACCTGAGGAAGCAAAGTTTAAATTCTACTCAC GTTTCAGTTCCAGCTT 3'**

This reaction yielded a 0.35Kb TthIII-Bam HI fragment. Both PCR products were gel purified using Qiaex and restricted with the appropriate enzymes. The Hind III - Tth III fragment plus the Tth III - Bam HI fragment then were ligated between the Hind III and Bam HI sites of Bluescript in a 3-way ligation. This construct, containing the entire $V_L$ region of LO-CD2a plus the MRC signal peptide was then sequenced.

The heavy chain LO-CD2a V region construct contains the MRC signal sequence at its 5' end and the long 3' untranslated region, also derived from the MRC H chain vector, at its 3' end. The final construct was made from 3 separate PCR reactions as follows:

Reaction 1: The DNA template was the MRC H chain vector. Since $V_L$ and $V_H$ genes of anti-lysozyme use the same signal, the sense primer was the same as that used for the LO-CD2a $V_L$ construct, i.e., 5' $V_1$ lyssig. The antisense primer was 3'$V_H$lyssig:

Pst 1

**5'TCTCGTGCAGTGGGACCTCGGAGTGGACACC3'**

This reaction produced a 0.16Kb Hind III - Pst I fragment containing the MRC signal plus a portion of FR 1 of LO-CD2a. The fragment was gel purified, restricted, and ligated into Hind III - Pst I cut Bluescript for sequencing.
Reaction 2: The DNA template was the LO-CD2a $V_H$ region in Bluescript. This reaction yielded a 0.3Kb Pst I - Sty I fragment containing most of the $V_H$ region. Because there was an internal Pst 1 site in FR 3 of LO-CD2a, the Pst I - Sty I fragment had to be constructed from 2 PCR reactions as follows:

```
                        2   4
                                <-------                  <-------
Pst 1                               Pst 1                     Sty 1
_________________0.2 Kb______________________________0.1 Kb__________

FR 1                                 FR3                      FR4
------->                          --------->
 1                                   3
```

The template DNA shown above is clone 82-8, LO-CD2a $V_H$ in Bluescript.
Reaction A: Yields a 0.2 Kb fragment, using olignucleotides, also refered to as oligos 1 and 2, as primers:

**Oligo 1 is: 5'Pst I 82-8 (sense):** **Pst I** **5'GAGGTCCAGCTGCAGCAGTCT3'**

**Oligo 2 is: 3'int. Pst I (antisense): 5'CGATGTATCAGCTGTCAGTGTGGC3'**

Reaction B: Yields a 0.1 Kb fragment, using oligos 3 and 4 as primers. Oligo 3 is 5'int. Pst I (sense):

**5'GCCACACTGACAGCTGATACATCG3'**

**Oligo 4 is 3'Sty I 82-8 (antisense):** **Sty I** **5'CAGAGTGCCTTGGCCCCAGTA3'**

Oligos 2 and 3 above contain changes in nucleotide sequence which remove the internal Pst I site without changing the amino acid sequence of LO-CD2a. Aliquots (2-5µl) of the overlapping products of reactions A & B above were combined and served as templates for a third PCR reaction. The oligonucleotide primers for this reaction were numbers 1 and 4 from the previous diagram. The 0.3 Kb product was gel purified by Qiaex and restricted with Pst I and Sty I. Since the fragment remained intact, the internal Pst 1 site had been successfully mutated.
Reaction 3: The final $V_H$ fragment was produced using the MRC heavy chain vector as template. This 0.23 Kb Sty I - Bam HI fragment contained a portion of FR4 of Lo-CD2a, and the entire 3' untranslated region from the MRC vector. The primers used were:

**5'$V_H$lys Sty I (sense):**
**Sty I**
**5'TACTGGGGCCAAGGCACCCTCGTCACA3'**

**3' $V_H$lys Bam HI (antisense):** **Bam HI** **5'GATCGGATCCCTTATAAATCTCTGGC3'**

The resulting fragment was gel purified and restricted with Sty I and Bam HI. The Pst I-Sty I and Sty I-Bam HI fragments were then ligated into Pst I-Bam HI cut Bluescript for sequencing.

All oligonucleotides were synthesized on an Applied Biosystems synthesizer. All sequencing reactions were carried out using The Sequenase ™ T7 Polymerase Kit (U.S. Biochemical, Cleveland, OH). All PCRs were carried out using the following protocol: 5 min. at 95°C, 35 cycles consisting of 1 min. at 94°C, 1 min. at 50°C, 2 min. at 72°C, a final extension of 5 min. at 72°C.

LO-CD2a $V_L$ and $V_H$ fragments containing the correct sequences were removed from Bluescript and cloned between the Hind III and Bam HI sites of the MRC light and heavy chain vectors, respectively. For the H chain, the 5' Hind III - Pst I fragment was first joined to the remainder of the construct (PstI-Bam HI) in Bluescript; the entire Hind III - Bam HI fragment was then cloned into the MRC vector.

### C. N-Terminal Amino Acid Sequencing of $V_H$ and $V_L$

N-terminal amino acid sequence analysis was performed by Harvard Microchemistry Laboratory in Cambridge, MA on samples of LO-CD2a heavy and light chains in order to confirm the sequences obtained using RNA-PCR. The samples were prepared as follows:
-200µg of LO-CD2a was applied across a 12% SDS polyacrylamide gel run in the presence of B-mercaptoethanol. Following electrophoresis, the protein was transferred to a PVDF membrane using a Western transfer apparatus. The membrane was stained briefly with Ponceau S, destained in 1% acetic acid, and the light and heavy chain bands were dried under vacuum and sent for amino acid analyses and N-terminal sequencing.

The amino acid sequence of the first 20 residues of LO-CD2a $V_H$ agreed completely with the cloned sequence; however, the sequence of $V_L$ indicated that residues 2, 3 and 7 in FR1 were different than those encoded by the cloned genes. These differences all reside in the PCR primer used for cloning purposes, based on a best guess sequence obtained from the previously cited literature.

### D. DNA Sequence Confirmation of N-Terminal Amino Acid Sequence and its Correction

In order to correct this sequence and simultaneously clone the native signal peptides of both $V_L$ and $V_H$ of LO-CD2a, RACE-PCR was employed was employed (Rapid Amplification of cDNA Ends): mRNA from LO-CD2a cells was reverse transcribed and the resulting cDNA was G-tailed at its 3' end using terminal transferase in the presence of dGTP. The cDNA was then amplified using a specific 3' oligonucleotide and a 5' oligonucleotide complementary to the G-tail. To simplify subcloning, a suitable restriction site was added to the 5' end of each oligonucleotide.

The oligonucleotides used for preparation of cDNA were as follows:

```
                                  Bam H1 Not I    Sal I
3' oligo Vk(VKA)                TTGGATCCGCGGCCGCGTCGACTACAGTTGGTGCAGCATCAGC

                                 Bam H1 Not 1    Sal 1
3' oligo Vh(CHA)                ATGGATCCGCGGCCGCGTCGACCCAGTGGATAGACCGATGG

        The oligonucleotides for RACE-PCR were as follows:

                               Xho I
5' Primer (TVl):  5' CCA TGG CCT CGA GGG CCC CCC CCC CCC CCC C 3'

                               Stu I
3' oligo Vh (BHA) 5' CCT GTT TAG GCC TCT GCT TCA CCC AGT AC 3'

                                          Sph I
3' oligo Vk (BKA) 5' GGA TAA TGG GTA AAT TGC ATG CAG TAA TA 3'
```

RACE-PCR reactions were carried out using the following protocol: 5 min. at 94°C, 40 cycles including 30 sec. at 94°C, 30 sec. at 50°C, and 50 sec. at 72°C, followed by a 5 min. extension at 72°C.

PCR products obtained for LO-CD2a $V_L$ and $V_H$ were gel extracted using Qiaex. The $V_H$ fragment was restricted with Xho I and Stu I and ligated into Xho I - Sma I cut Bluescript. The $V_L$ fragment was blunt-ended and ligated into Sma I cut Bluescript. A number of clones were sequenced for both light and heavy chain V regions and the signal sequences were identified.

Since signal sequences found in immunoglobulin genes generally have introns, these may be important for expression. Genomic clones containing the $V_L$ and $V_H$ leader sequences were identified as well. Genomic DNA was prepared as follows: 4 x $10^7$ LO-CD2a cells were spun down, washed in cold PBS, spun down, and washed with PBS again. Cells were resuspended in 0.4ml digestion buffer (with freshly added proteinase K). This mixture was incubated with shaking at 50°C for 12-15 hours, extracted with an equal volume of phenol/chloroform/isoamyl alcohol, and spun at 1700xg. The aqueous phase was transferred to a clean tube and 1/2 volume of 7.5 M ammonium acetate and 2 volumes of 95% ethanol were added. The DNA was pelleted by spinning 2 minutes, 1700xg. The pellet was washed with 70% ethanol and air dried. The pellet was resuspended in 80 ml TE, pH 8.0.

Using genomic DNA obtained from the cell line LO-CD2a as a template, the following oligonucleotides were designated in order to amplify the genomic leader sequences of both $V_L$ and $V_H$ as well as portions of the framework regions ending at unique restriction sites (Sph I for $V_L$, Pst I for $V_H$).

LVH #430 TGCAAGCTTCATGATGAGTCCTGTCCAGTC

Leader $V_L$ sense/Hind III

LVL #429 AGTAAGCTTCATGAAATGCAGGTGGATC

Leader $V_H$ sense/Hind III

PVHA # 428 GGGAGATTGCTGCAGCTGGACTTC $V_H$ antisense/Pst I

PCR reactions were carried out as follows: 100 ng genomic DNA from LO-CD2a cells, 200 pmol each of oligos LVL and BKA (for $V_L$ fragment) or 200 pmol each of LVH and PVHA (for $V_H$ fragment), 100 µl 1mm dNTPs, 10 µl 10 x Pfu buffer. 1 ml (2.5 units) pfu DNA polymerase (Stratagene, La Jolla, CA) deionized water to 100 µl. Pfu was used because of its greater accuracy than Taq polymerase.

The reaction conditions were as follows: 5 min. 94°C, 5 min. 50°C, 35 cycles of 1 min. 94°C, 1 min. 50°C, 1 min. 72°C, followed by 5 min. at 72°C. The PCR products were gel purified, restricted and ligated into Bluescript for sequencing. Once clones containing the correct sequence were identified, Bluescript vectors containing these clones were cut with Hind III and Sph I ($V_L$) or Hind III and Pst I ($V_H$) and the fragments were gel isolated. The 0.75 Kb Hind III-Sph I fragment was then ligated into Bluescript containing the original LO-CD2a $V_L$ construct from which the Hind III - Sph I fragment had been removed. The new construct contained the native LO-CD2a signal plus intron and a corrected FR1 sequence (in agreement with the N-terminal sequence). The 0.16 Kb Hind III - Pst I fragment was ligated into Bluescript containing the original LO-CD2a $V_H$ construct from which the Hind III - Pst I fragment had been removed. The new construct contained the native signal + intron. The newly constructed $V_L$ and $V_H$ fragments were then removed from Bluescript by digestion with Hind III and Bam HI and cloned into the MRC light and heavy chain vectors, respectively, for expression in COS cells.

**E. Transient expression in COS cells**.

COS 7 cells were obtained rom the ATCC and were grown in Dulbecco's Minimal Essential Medium (DMEM) with 10% fetal bovine serum (FBS). Optimal transfection was achieved at approximately 50% confluency of adherent cells. In preparation for transfection, plasmid DNA was added to DMEM containing NuSerum and DEAE-Dextran/chloroquine diphosphate. COS cell medium was removed, the DNA mixture was added and the cells incubated for 3 hours at 37°C. This medium then was removed, and 10% DMSO in PBS was added to the cells for 2 minutes and then removed. DMEM with 10% FBS was added to the cells. After overnight incubation, the medium was replaced and the cells were incubated for 2 days at 37°C. Supernatants were collected for assay by ELISA for the secretion of chimeric antibody.

**F. Detection of secreted chimeric by ELISA.**

Secretion of chimeric antibody was confirmed by assay of supernatants from the transfected COS cells in an ELISA designed to detect the presence of human antibody (or a portion thereof). Goat anti-human IgG (H+L) was diluted in phosphate buffered saline (PBS) to a concentration of 5 µg/ml and bound to the wells of ELISA microtiter plates by overnight incubation at 4°C. Plates were washed 3 times using an ELISA plate washer.

Remaining free sites were blocked by the addition of 200 µl PBS containing 1% bovine serum albumin (PBS-BSA) for 1/2 hr. at room temperature. Two-fold dilutions were prepared in PBS-BSA of the supernatants and of a positive control reference standard (purified human IgGlk). Media alone and/or PBS-BSA alone constituted negative controls. Antibody dilutions and controls were added to the wells and incubated at room temperature for 1 1/2 hours. Plates were then washed 3 times with a plate washer in PBS containing 0.05% Tween20. The appropriate dilution of a goat anti-human IgG (gamma chain specific)-horseradish peroxidase (HRP) conjugated antibody or goat anti-human kappa light chain -HRP conjugated antibody was added to each well and incubated at room temperature for 1 hour. Plates were washed with PBS-Tween20 as described above, after which the developing substrate, (ABTS) containing hydrogen peroxide, was added. Bound antibody was detected by reading absorbance at a wavelength of 405 nm.

**G. Binding specificity of secreted chimeric antibody.**

Binding specificity of the chimeric was evaluated by flow cytometric analysis of antibody binding to the CD2-expressing mutant Jurkat cell line JRT3-T3-5. The binding profile of the chimeric antibody (human IgG1) was compared with those of the native rat antibody (IgG2b) and the isotype-matched control MABs (human IgG1 and rat IgG2b) which exhibit irrelevant (non-CD-2) binding specificities.

*Preparation of JRT3-T3-5 (Jurkat) cell line.* The Jurkat cell line was obtained from the ATCC ( ) and was propagated in D-MEM containing 10% fetal bovine serum (FBS), 10% amino acid supplement (NCTC), and 6 mM L-glutamine (complete medium). The cells were maintained at 37°C with 10% $CO_2$ and were passaged three times per week at a ratio of 1:4 (the cell concentration at passage being approximately 3 x $10^6$/ml). Jurkat cells were harvested, centrifuged to remove spent medium, and washed in DMEM. The cells were then resuspended in phosphate buffered saline (PBS) with 0.1% sodium azide (NaAz), and an aliquot was removed for cell quantification. The number of viable cells was determined by trypan blue exclusion.

*Indirect staining of Jurkat cells.* Cell surface staining was carried out in a 96 well U-bottom microtiter plate. Approximately 6 x $10^5$ cells in a volume of 90 µl were distributed into each well of the microtiter plate. Dilutions of the antibodies to be tested were prepared in PBS with 0.1% NaAz and distributed into the appropriate wells in a volume of 10 µl. Cells were incubated with antibody for 15 minutes at room temperature, after which the cells were washed 3 times by adding PBS with 0.1% NaAz to each well and by centrifuging for 2 minutes at 1900 rpm (Sorvall RT6000D). Resuspension of cells was accomplished by gently tapping the plates. Ten ul aliquots of the appropriate fluorescein-isothiocyanate (FITC)-conjugated secondary antibody (anti-human Ig or anti-rat Ig) was added to the appropriate wells and incubated at room temperature for 15 minutes in the dark. Plates were washed 3 times in PBS with 0.1% NaAz as described above. Stained cells were fixed by the addition of 200 µl of 0.5% paraformaldehyde in PBS and were stored at 4°C (up to 1 week).

*Flow cytometric analysis of stained Jurkat cells.* Stained cells were transferred to 12 x 17 mm polystyrene tubes for acquisition of data using a Becton-Dickinson FACScan. Data acquisition and analysis were carried out using LYSIS-II software. CD2-expressing Jurkat cells were incubated with the LO CD2a (rat IgG2b)MAB, the chimeric version of LO-CD2a (human IgG1), and the corresponding isotype matched controls. Bound antibody was detected using the appropriate FITC-conjugated secondary antibody according to the protocol described above. Analysis shows similar binding patterns of the native rat LO CD2a and the chimeric human-rat LO-CD2a.

## Claims

1. An antibody which specifically binds to the same epitope on human lymphocytes as the monoclonal antibody produced by the cell line deposited as ATCC HB 11423.

2. The antibody of claim 1 wherein said antibody is a monoclonal antibody or fragment thereof.

3. The antibody of claim 1 wherein said antibody is a monoclonal antibody identical to the monoclonal antibody produced by said deposited cell line.

4. The antibody according to claim 1 wherein said antibody is an antibody having the same CDRs as the antibody produced by said deposited cell line.

5. The antibody according to claim 1 or 4 wherein said antibody includes a $V_L$ chain having an amino acid sequence as shown in figure 29 A or figure 29 B.

6. The antibody according to claim 1 or 4 wherein said antibody includes a $V_H$ chain having an amino acid sequence as shown in figure 30 A or figure 30 B.

7. The antibody according to one of the preceding claims wherein said antibody binds to a conformational epitope.

8. The antibody according to one of the preceding claims wherein said antibody binds to the CD2 epitope of CD2 positive human T-cells.

9. The antibody according to one of the preceding claims wherein said antibody binds to at least a portion of human $CD2^+$NK cells.

10. The antibody of any one of claims 1 to 4 wherein said antibody is a humanized form of the antibody.

11. The antibody of any one of claims 1 to 4 wherein said antibody is a chimeric form of the antibody.

12. The antibody of claim 10 or 11 wherein said antibody elicits alloantigen specific hyporesponsiveness.

**13.** An antibody according to claim 12 which elicits antigen specific hyporesponsiveness.

**14.** The antibody of claim 13 which elicits alloantigen specific hyporesponsiveness.

**15.** The antibody of claim 13 or 14 which competes for binding to an antigen with the monoclonal antibody produced by the cell line deposited as ATCC HB 11423.

**16.** Use of the antibody according to one of the preceding claims for the preparation of a medicine or a pharmaceutically active composition for inhibiting, treating or preventing an immune response.

**17.** Use of the antibody according to one of the claims 1 to 15 for the preparation of a medicine or a pharmaceutically active composition for treating an autoimmune disease.

**18.** Use according to claim 16 or 17 wherein said medicine or composition also comprises a pharmaceutically acceptable excipient.

**19.** Use according to claim 18, wherein said immune response is graft rejection or graft versus host disease.

**20.** Use according to claim 18 or 19 wherein said immune response is elicited upon allograft or xenograft transplantation.

**21.** Use according to one of the claims 18 to 20 wherein said medicine or said composition is administered prior to transplantation.

**22.** A composition comprising an antibody according to any one of claims 1 through 15 and a pharmaceutically acceptable carrier.

**Patentansprüche**

**1.** Antikörper, der spezifisch an dasselbe Epitop auf menschlichen Lymphozyten bindet wie der von der als ATCC HB 11423 hinterlegten Zellinie erzeugte monoklonale Antikörper.

**2.** Antikörper gemäß Anspruch 1, wobei der Antikörper ein monoklonaler Antikörper oder ein Fragment desselben ist.

**3.** Antikörper gemäß Anspruch 1, wobei der Antikörper ein monoklonaler Antikörper ist, der identisch zu dem von der hinterlegten Zellinie erzeugten Antikörper ist.

**4.** Antikörper gemäß Anspruch 1, wobei der Antikörper ein Antikörper mit denselben CDRs wie der von der hinterlegten Zellinie erzeugte Antikörper ist.

**5.** Antikörper gemäß Anspruch 1 oder 4, wobei der Antikörper eine $V_L$-Kette mit einer Aminosäuresequenz wie in Figur 29A oder Figur 29B dargestellt einschließt.

**6.** Antikörper gemäß Anspruch 1 oder 4, wobei der Antikörper eine $V_H$-Kette mit einer Aminosäuresequenz wie in Figur 30A oder Figur 30B dargestellt einschließt.

**7.** Antikörper gemäß einem der vorhergehenden Ansprüche, wobei der Antikörper an ein konformationelles Epitop bindet.

**8.** Antikörper gemäß einem der vorhergehenden Ansprüche, wobei der Antikörper an das CD2-Epitop von CD2-positiven menschlichen T-Zellen bindet.

**9.** Antikörper gemäß einem der vorhergehenden Ansprüche, wobei der Antikörper an wenigstens einen Bereich von menschlichen $CD2^+$NK-Zellen bindet.

**10.** Antikörper gemäß einem der Ansprüche 1 bis 4, wobei der Antikörper eine humanisierte Form des Antikörpers ist.

11. Antikörper gemäß einem der Ansprüche 1 bis 4, wobei der Antikörper eine Chimäre Form des Antikörpers ist.

12. Antikörper gemäß Anspruch 10 oder 11, wobei der Antikörper eine alloantigenspezifische Unterempfindlichkeit auslöst.

13. Antikörper gemäß Anspruch 12, der eine antigenspezifische Unterempfindlichkeit auslöst.

14. Antikörper gemäß Anspruch 13, der eine Alloantigen spezifische Unterempfindlichkeit auslöst.

15. Antikörper gemäß Anspruch 13 oder 14, der mit dem von der als ATCC HB 11423 hinterlegten Zellinie produzierten monoklonalen Antikörper um die Bindung an ein Antigen konkurriert.

16. Verwendung des Antikörpers gemäß einem der vorhergehenden Ansprüche zur Herstellung eines Medikaments oder einer pharmazeutisch aktiven Zusammensetzung zum Inhibieren, Behandeln oder Vorbeugen einer Immunantwort.

17. Verwendung des Antikörpers gemäß einem der Ansprüche 1 bis 15 zur Herstellung eines Medikaments oder einer pharmazeutisch aktiven Zusammensetzung zur Behandlung einer Autoimmunerkrankung.

18. Verwendung gemäß Anspruch 16 oder 17, wobei das Medikament oder die Zusammensetzung auch einen pharmazeutisch verträglichen Arzneimittelträger umfaßt.

19. Verwendung gemäß Anspruch 18, wobei die Immunantwort eine Transplantatabstoßung oder Transplantatwirtreaktion ist.

20. Verwendung gemäß Anspruch 18 oder 19, wobei die Immunantwort aufgrund einer Allotransplantation oder einer Xenotransplantation ausgelöst wird.

21. Verwendung gemäß einem der Ansprüche 18 bis 20, wobei das Medikament oder die Zusammensetzung vor einer Transplantation verabreicht wird.

22. Zusammensetzung, umfassend einen Antikörper gemäß einem der Ansprüche 1 bis 15 und einen pharmazeutisch verträglichen Träger.

## Revendications

1. Un anticorps qui se fixe spécifiquement au même épitope des lymphocytes humains que l'anticorps monoclonal produit par la lignée de cellules déposée en tant que ATCC HB 11423.

2. L'anticorps de la revendication 1, dans lequel ledit anticorps est un anticorps monoclonal ou un fragment de celui-ci.

3. L'anticorps de la revendication 1, dans lequel ledit anticorps est un anticorps monoclonal identique à l'anticorps monoclonal produit par ladite lignée de cellules déposée.

4. L'anticorps selon la revendication 1, dans lequel ledit anticorps est un anticorps ayant les mêmes CDRs que l'anticorps produit par ladite lignée de cellules déposée.

5. L'anticorps selon la revendication 1 ou 4, dans lequel ledit anticorps contient une chaîne $V_L$ ayant une séquence d'aminoacides telle que représentée à la figure 29A ou à la figure 29B.

6. L'anticorps selon la revendication 1 ou 4, dans lequel ledit anticorps contient une chaîne $V_H$ ayant une séquence d'aminoacides telle que représentée à la figure 30A ou à la figure 30B.

7. L'anticorps selon l'une quelconque des revendications précédentes, dans lequel ledit anticorps se fixe sur un épitope conformationnel.

8. L'anticorps selon l'une quelconque des revendications précédentes, dans lequel ledit anticorps se fixe sur l'épitope

CD2 des cellules T humaines CD2-positives.

9. L'anticorps selon l'une quelconque des revendications précédentes, dans lequel ledit anticorps se fixe sur au moins une portion des cellules humaines $CD2^+$NK.

10. L'anticorps selon l'une quelconque des revendications 1 à 4, dans lequel ledit anticorps est une forme humanisée de l'anticorps.

11. L'anticorps selon l'une quelconque des revendications 1 à 4, dans lequel ledit anticorps est une forme chimérique de l'anticorps.

12. L'anticorps selon la revendication 10 ou 11, dans lequel ledit anticorps provoque une hypo-réactivité spécifique vis-à-vis d'un allo-antigène.

13. Un anticorps selon la revendication 12, qui provoque une hypo-réactivité spécifique vis-à-vis d'un antigène.

14. L'anticorps de la revendication 13, qui provoque une hypo-réactivité spécifique vis-à-vis d'un allo-antigène.

15. L'anticorps de la revendication 14 ou 15, qui est compétitif, à l'égard de sa fixation sur un antigène, avec l'anticorps monoclonal produit par la lignée de cellules déposée sous la référence ATCC HB 11423.

16. Utilisation de l'anticorps selon l'une quelconque des revendications précédentes, pour la préparation d'un médicament ou d'une composition pharmaceutiquement active pour inhiber, traiter ou prévenir une réponse auto-immune.

17. Utilisation de l'anticorps selon l'une quelconque des revendications 1 à 15 pour la préparation d'un médicament ou d'une composition pharmaceutiquement active pour le traitement d'une affection auto-immune.

18. Utilisation selon la revendication 16 ou 17, dans laquelle ledit médicament ou ladite composition comporte également un excipient pharmaceutiquement acceptable.

19. Utilisation selon la revendication 18, dans laquelle ladite réponse immune est le rejet d'une greffe ou un conflit entre un hôte et une greffe.

20. Utilisation selon la revendication 18 ou 19, dans laquelle ladite réponse immune est provoquée lors d'une transplantation du type allogreffe ou xénogreffe.

21. Utilisation selon l'une quelconque des revendications 18 à 20, dans laquelle ledit médicament ou ladite composition est administré préalablement à la transplantation.

22. Une composition comprenant un anticorps selon l'une quelconque des revendications 1 à 15 et un véhicule pharmaceutiquement acceptable.

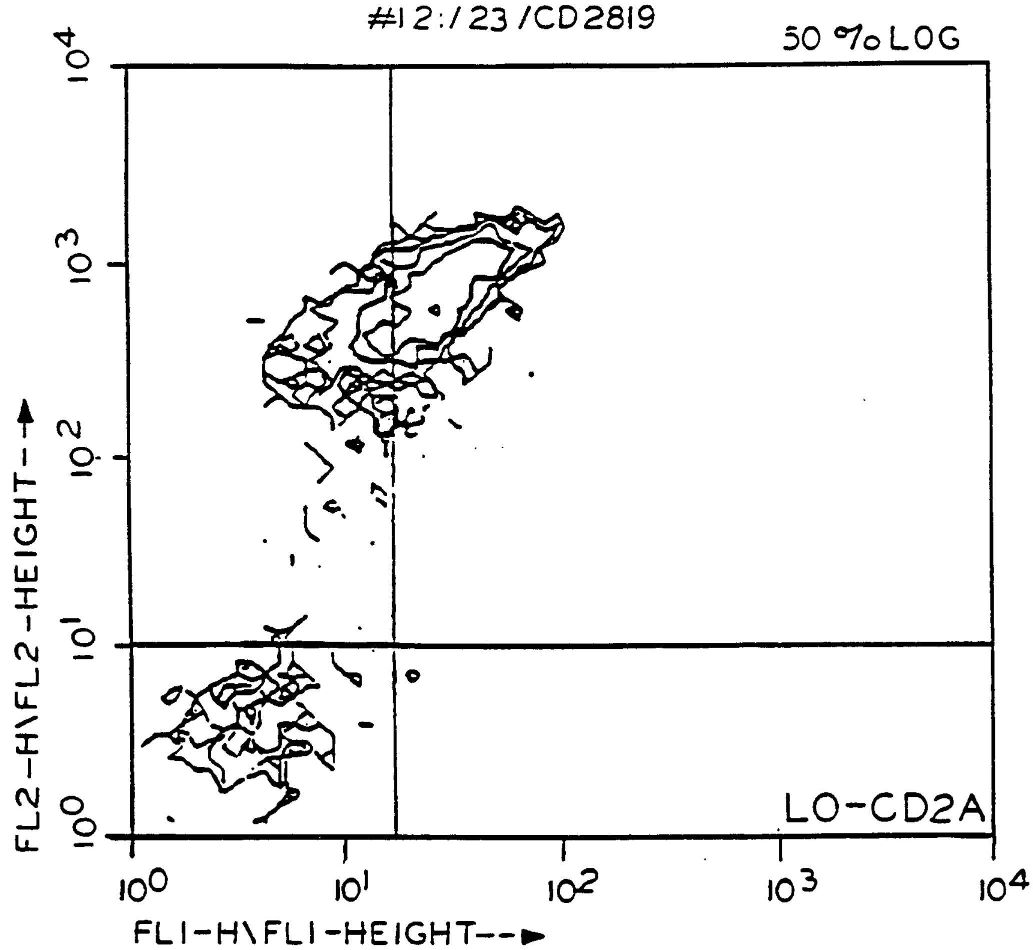


--- QUAD STATS ---

FILE: #12:/23/CD2019 SAMPLE: 059

DATE: 9/24/92 GATE G1 - R1

PARMETER : FL1 - H\(LOG) FL2-H(LOG) QUAD LOCATION:17.15 ,9

TOTAL= 5000 GATED = 1290

| QUAD | EVENTS | % GATED | %TOTAL | X MEAN | Y MEAN |
|---|---|---|---|---|---|
| 1UL | 299 | 23.18 | 3.98 | 11.41 | 284.69 |
| 2UR | 851 | 65.97 | 17.02 | 32.70 | 630.65 |
| 3LL | 135 | 10.47 | 2.70 | 4.08 | 3.31 |
| 4LR | 5 | 0.39 | 0.10 | 25.11 | 6.54 |

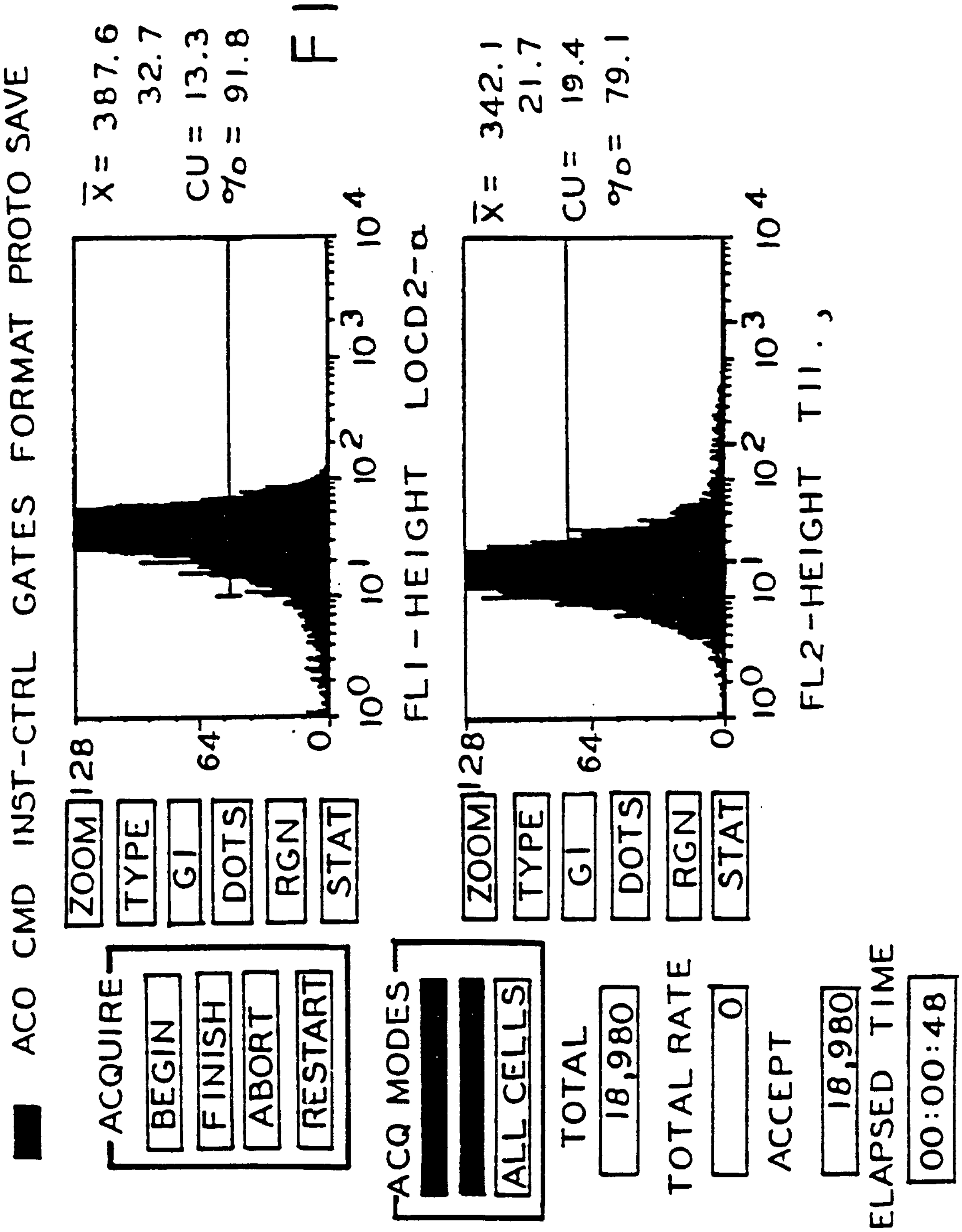
ACQ CMD INST-CTRL GATES FORMAT PROTO SAVE
ACQUIRE
BEGIN
FINISH
ABORT
RESTART
ACQ MODES
ALL CELLS
TOTAL
18,980
TOTAL RATE
0
ACCEPT
18,980
ELAPSED TIME
00:00:48
ZOOM
TYPE
GI
DOTS
RGN
STAT
128
64
0
$10^0$
$10^1$
$10^2$
$10^3$
$10^4$
FL1-HEIGHT LOCD2-a
X̄ = 387.6
32.7
CU = 13.3
% = 91.8
FL2-HEIGHT T11
X̄ = 342.1
21.7
CU = 19.4
% = 79.1

FIG. 2A

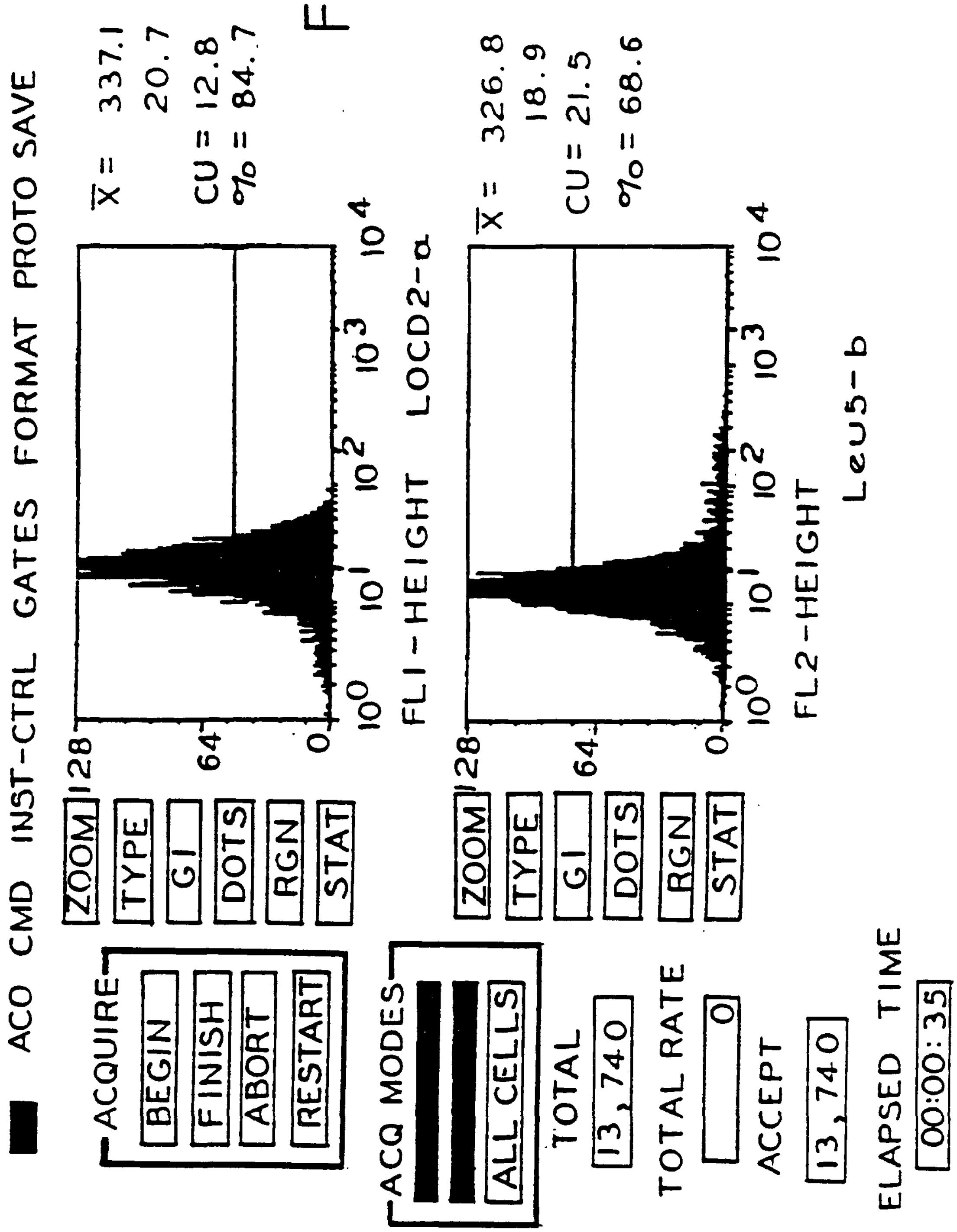
ACQ CMD INST-CTRL GATES FORMAT PROTO SAVE
ACQUIRE
BEGIN
FINISH
ABORT
RESTART
ACQ MODES
ALL CELLS
TOTAL
13, 740
TOTAL RATE
0
ACCEPT
13, 740
ELAPSED TIME
00:00:35
ZOOM
TYPE
GI
DOTS
RGN
STAT
128
64
0
10^0
10^1
10^2
10^3
10^4
FL1-HEIGHT LOCD2-a
X̄ = 337.1
20.7
CU = 12.8
% = 84.7
FL2-HEIGHT
Leu5-b
X̄ = 326.8
18.9
CU = 21.5
% = 68.6

FIG. 2B

FIG. 3A

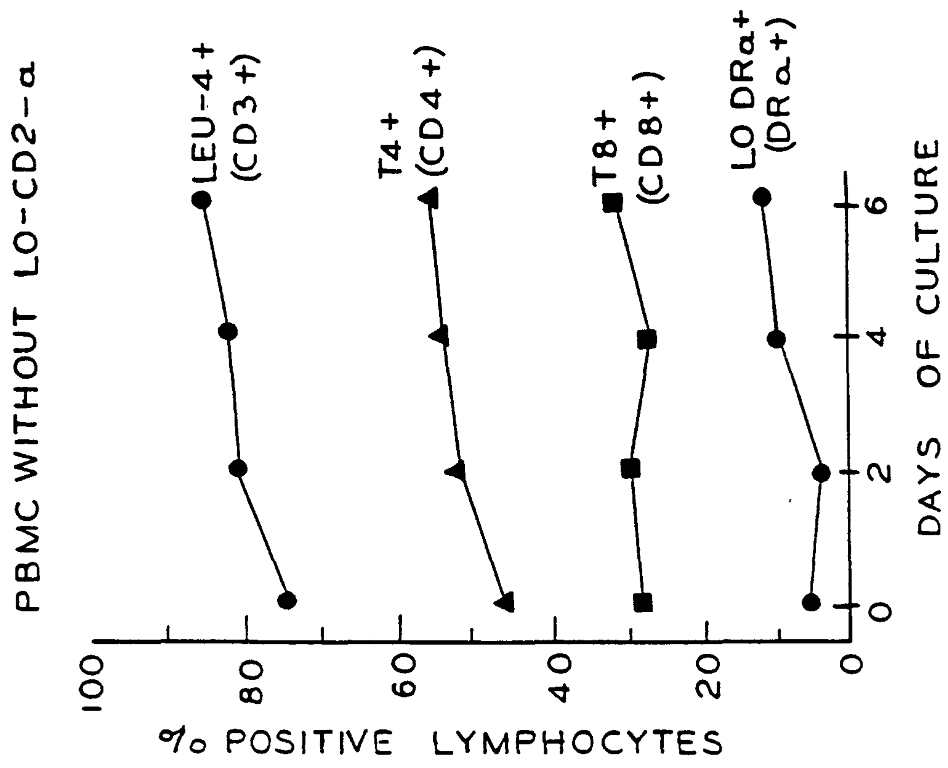
PBMC WITHOUT LO-CD2-α
LEU-4+ (CD3+)
T4+ (CD4+)
T8+ (CD8+)
LO DRα+ (DRα+)
% POSITIVE LYMPHOCYTES
100
80
60
40
20
0
0
2
4
6
DAYS OF CULTURE

FIG. 3B

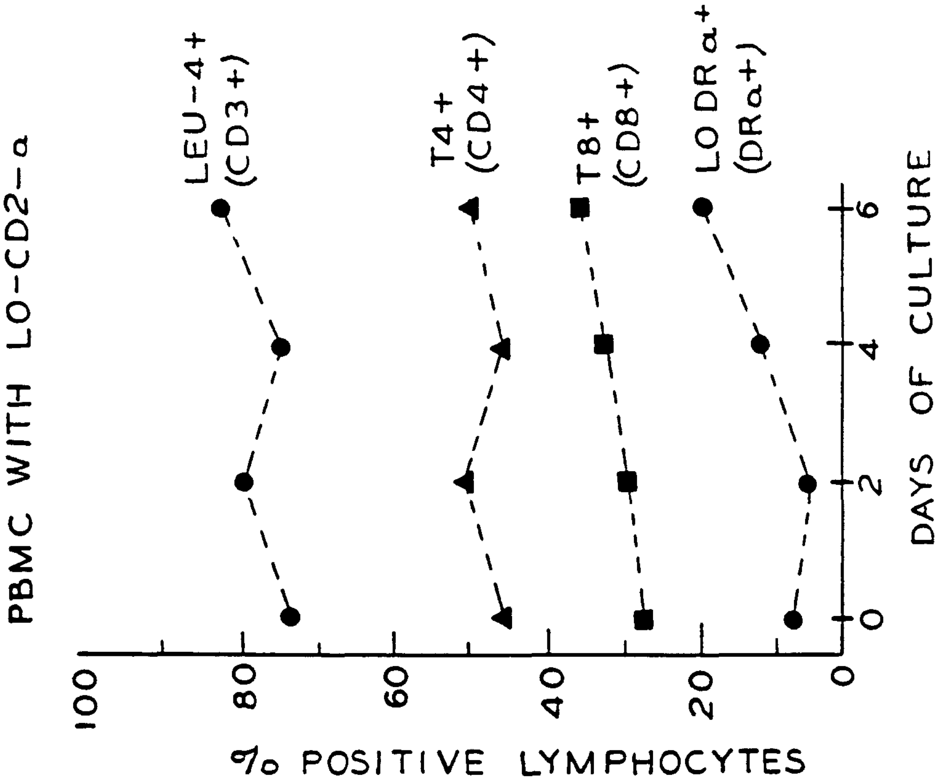
PBMC WITH LO-CD2-α
LEU-4+ (CD3+)
T4+ (CD4+)
T8+ (CD8+)
LO DRα+ (DRα+)
% POSITIVE LYMPHOCYTES
100
80
60
40
20
0
0
2
4
6
DAYS OF CULTURE

FIG. 4A

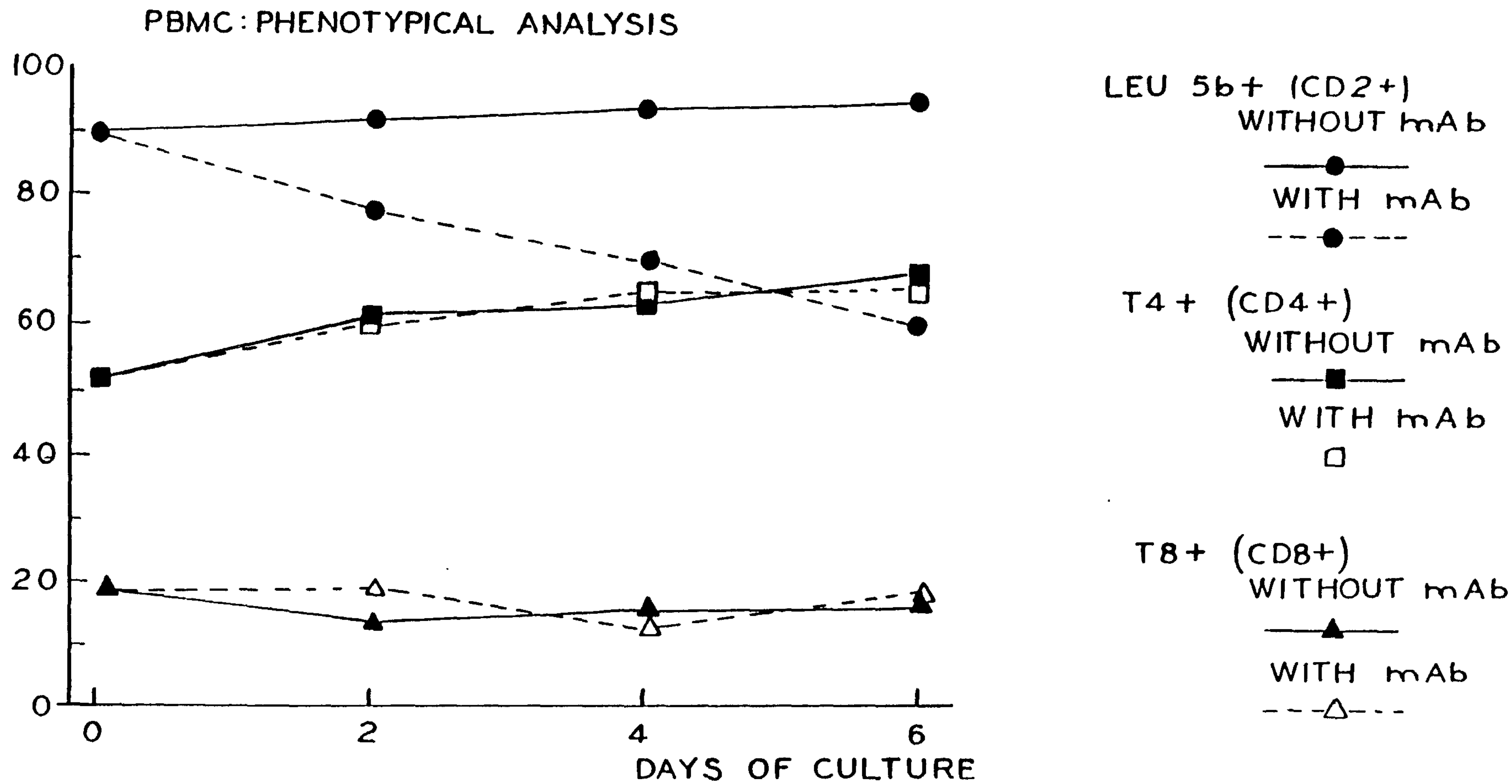
PBMC: PHENOTYPICAL ANALYSIS
100
80
60
40
20
0
0
2
4
6
DAYS OF CULTURE
LEU 5b+ (CD2+)
WITHOUT mAb
WITH mAb
T4+ (CD4+)
WITHOUT mAb
WITH mAb
T8+ (CD8+)
WITHOUT mAb
WITH mAb

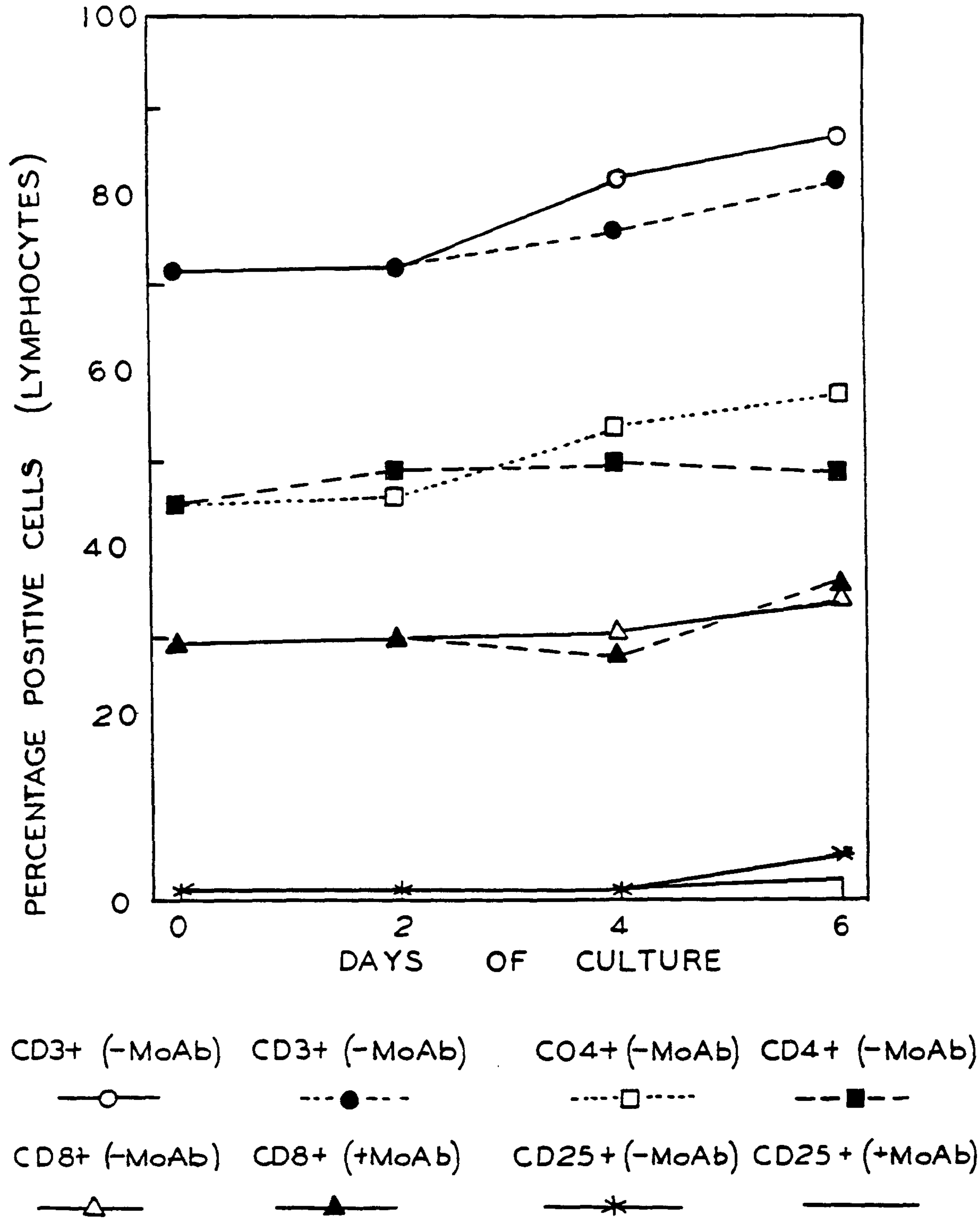
PERCENTAGE POSITIVE CELLS (LYMPHOCYTES)
100
80
60
40
20
0
0
2
4
6
DAYS OF CULTURE
CD3+ (-MoAb)
CD3+ (-MoAb)
CO4+ (-MoAb)
CD4+ (-MoAb)
CD8+ (-MoAb)
CD8+ (+MoAb)
CD25+ (-MoAb)
CD25+ (+MoAb)

FIG. 4B

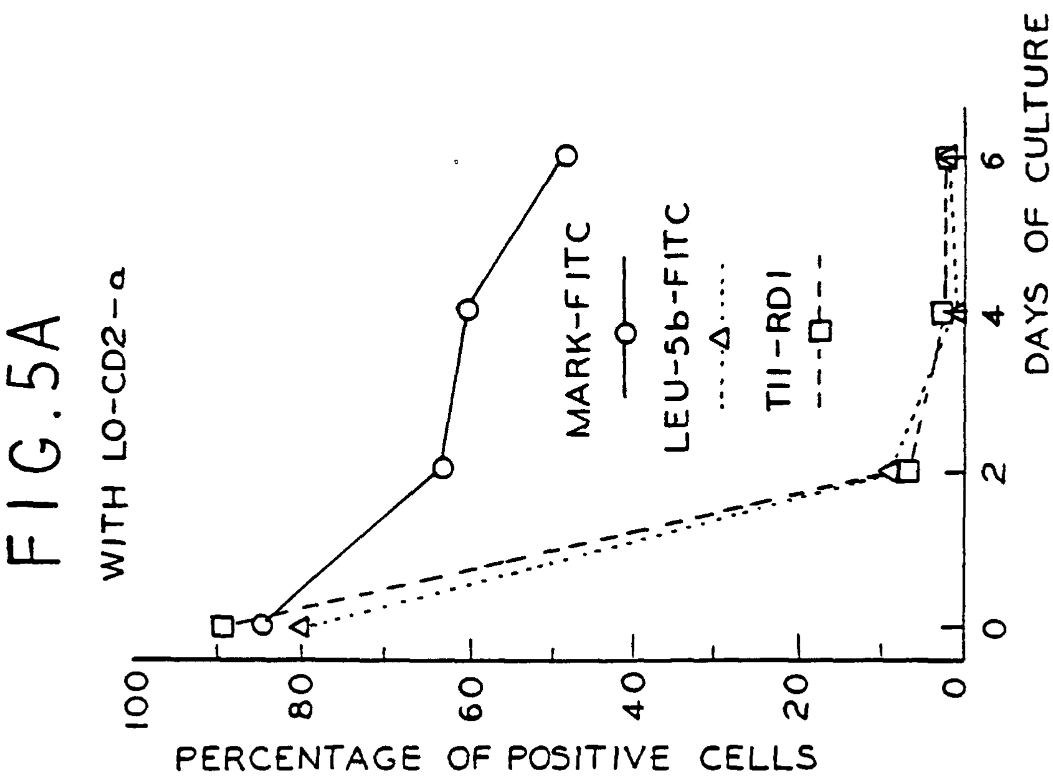
WITH LO-CD2-a
MARK-FITC
LEU-5b-FITC
TII-RDI
PERCENTAGE OF POSITIVE CELLS
DAYS OF CULTURE
100
80
60
40
20
0
0
2
4
6
FIG. 5A

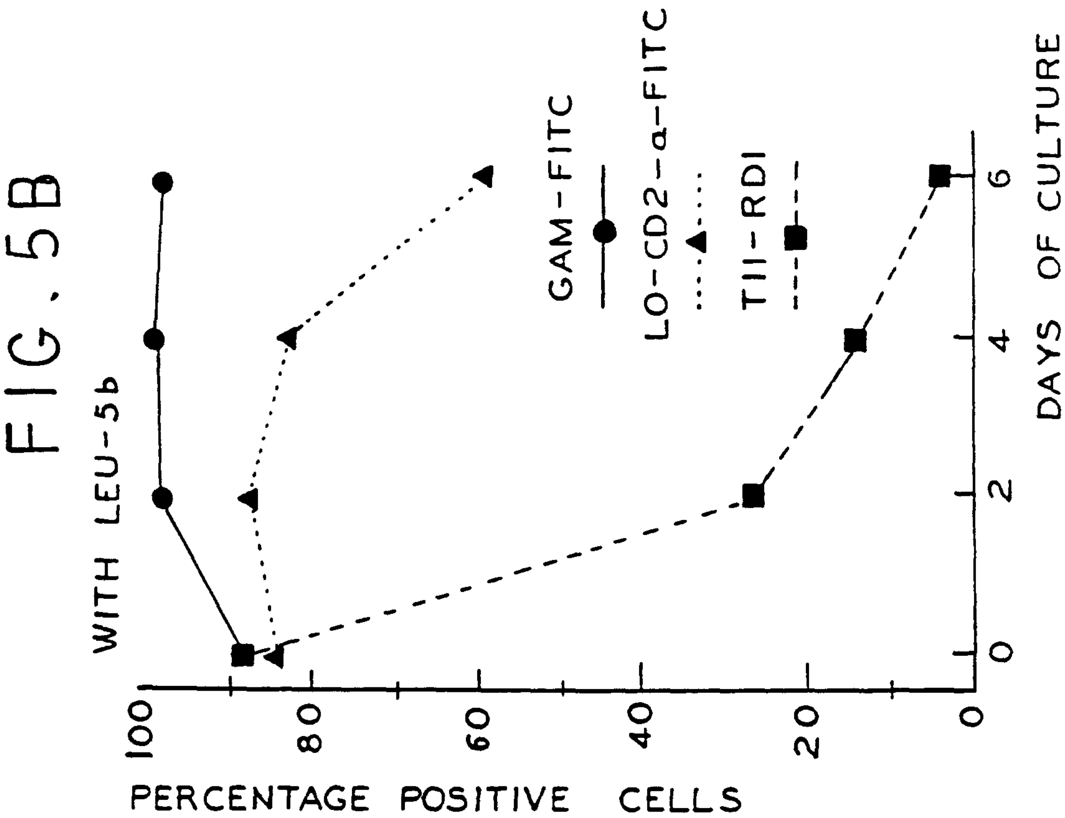
WITH LEU-5b
GAM-FITC
LO-CD2-a-FITC
TII-RDI
PERCENTAGE POSITIVE CELLS
DAYS OF CULTURE
100
80
60
40
20
0
0
2
4
6
FIG. 5B

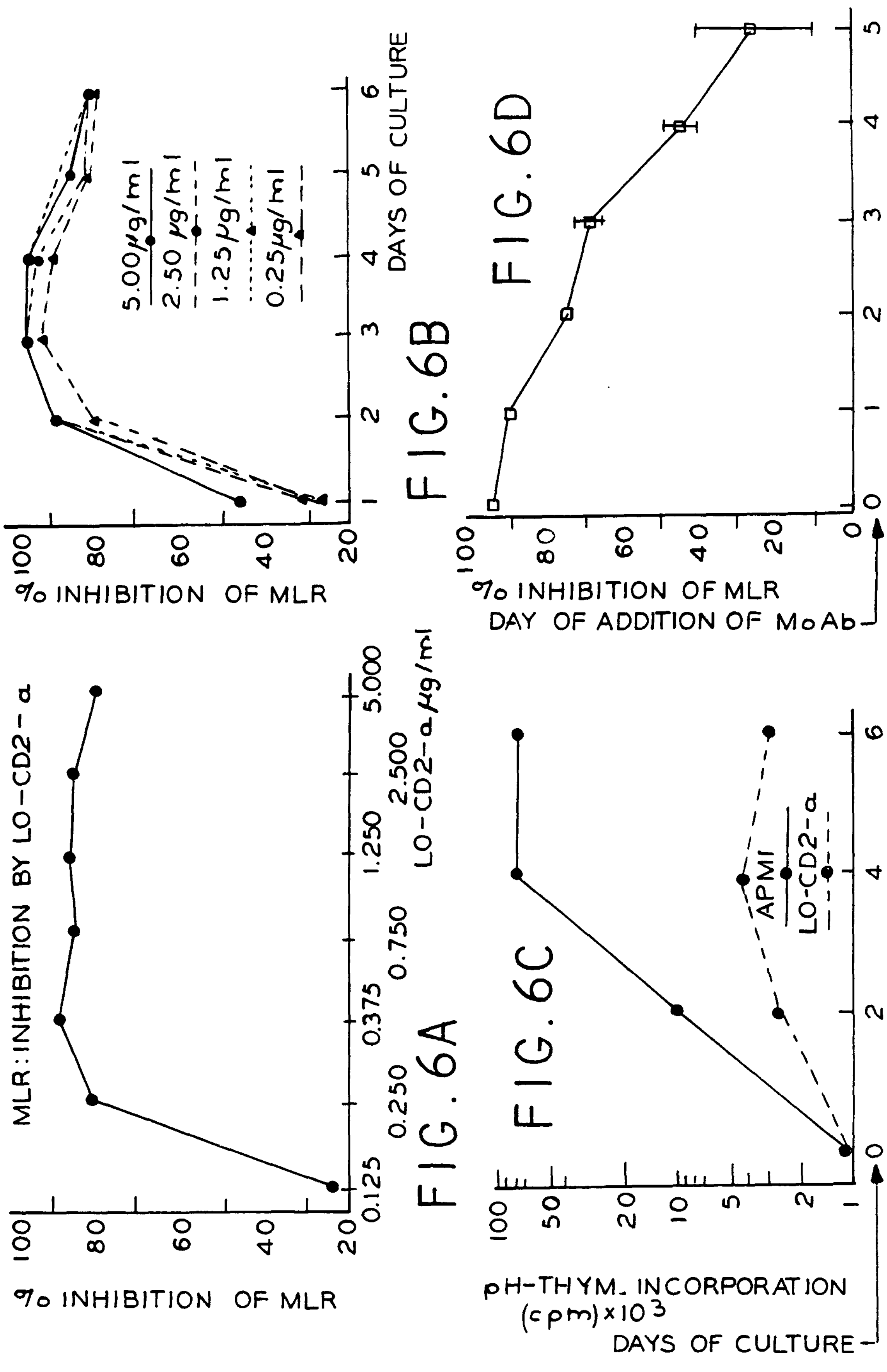


FIG. 6A

FIG. 6B

FIG. 6C

FIG. 6D

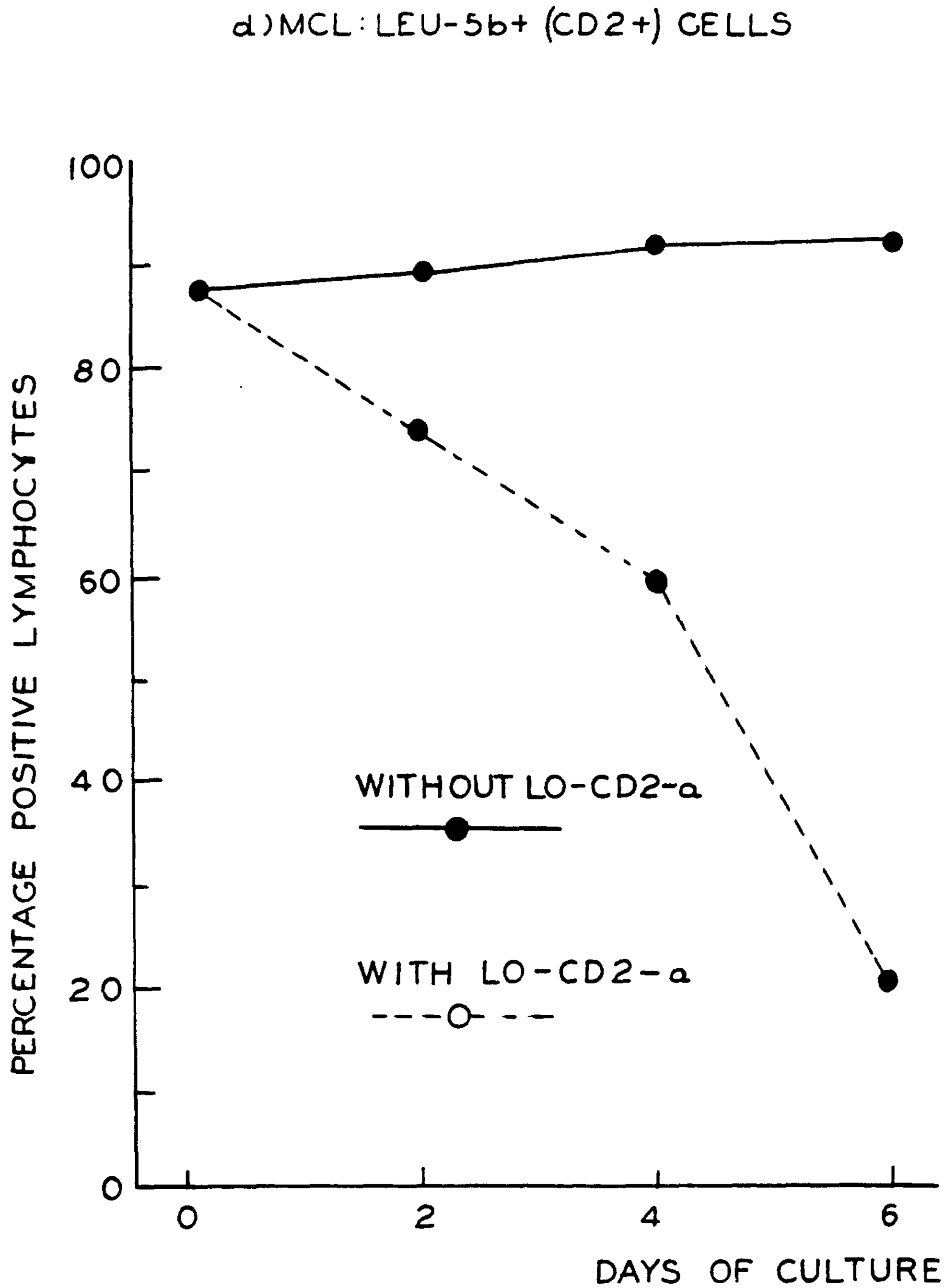
d) MCL: LEU-5b+ (CD2+) CELLS
PERCENTAGE POSITIVE LYMPHOCYTES
100
80
60
40
20
0
0
2
4
6
DAYS OF CULTURE
WITHOUT LO-CD2-a
WITH LO-CD2-a

FIG. 7

FIG. 8A

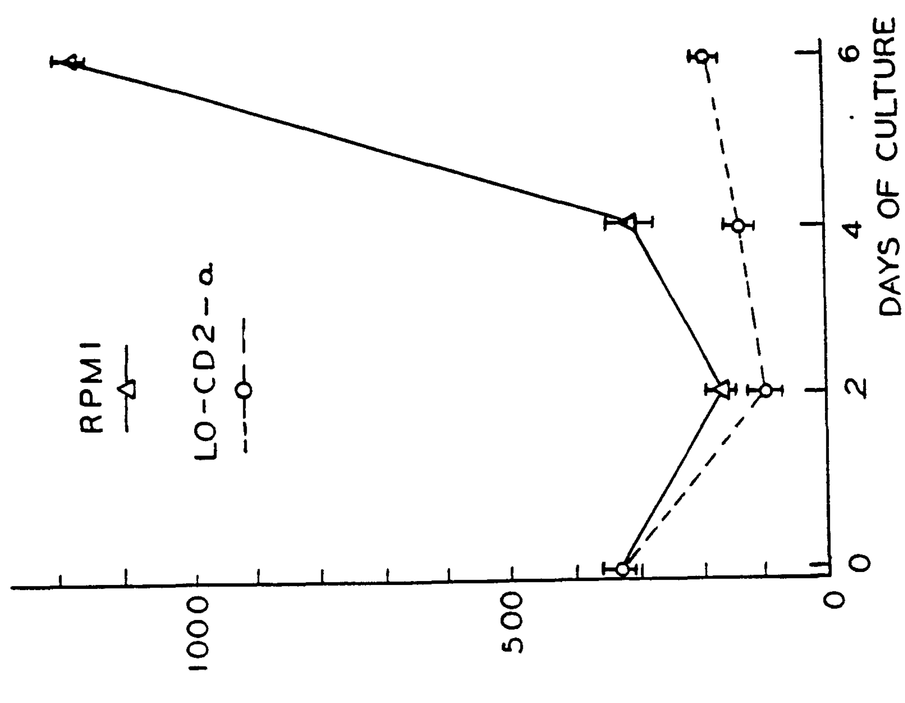
RPMI
LO-CD2-a
1000
500
0
0
2
4
6
DAYS OF CULTURE

FIG. 8B

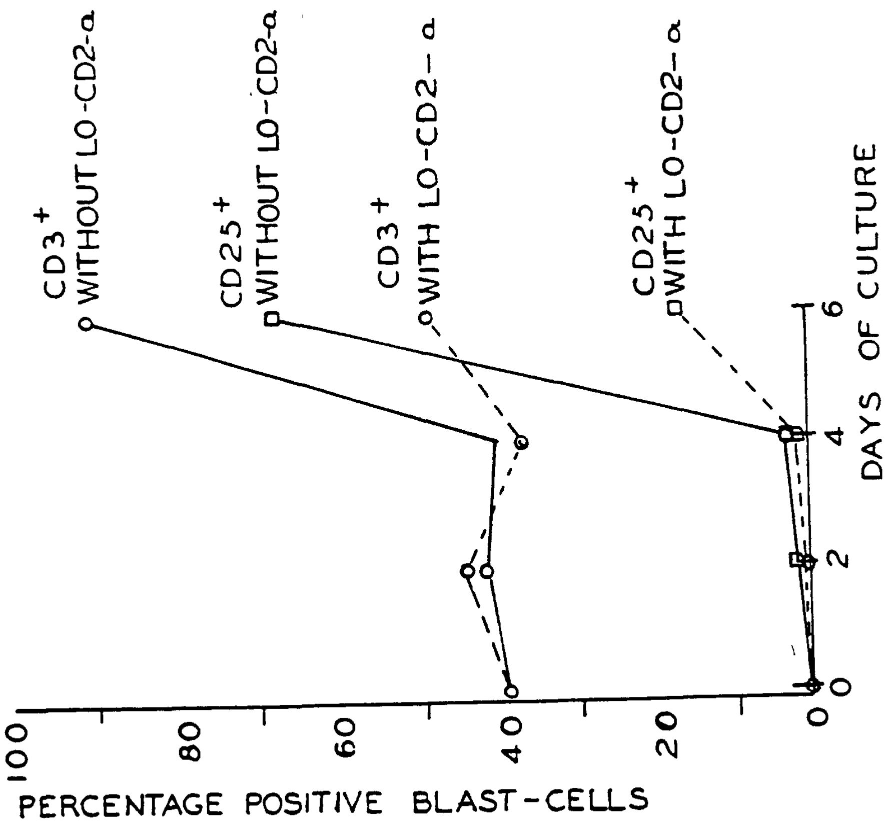
CD3+ WITHOUT LO-CD2-a
CD25+ WITHOUT LO-CD2-a
CD3+ WITH LO-CD2-a
CD25+ WITH LO-CD2-a
100
80
60
40
20
0
PERCENTAGE POSITIVE BLAST-CELLS
0
2
4
6
DAYS OF CULTURE

FIG. 9

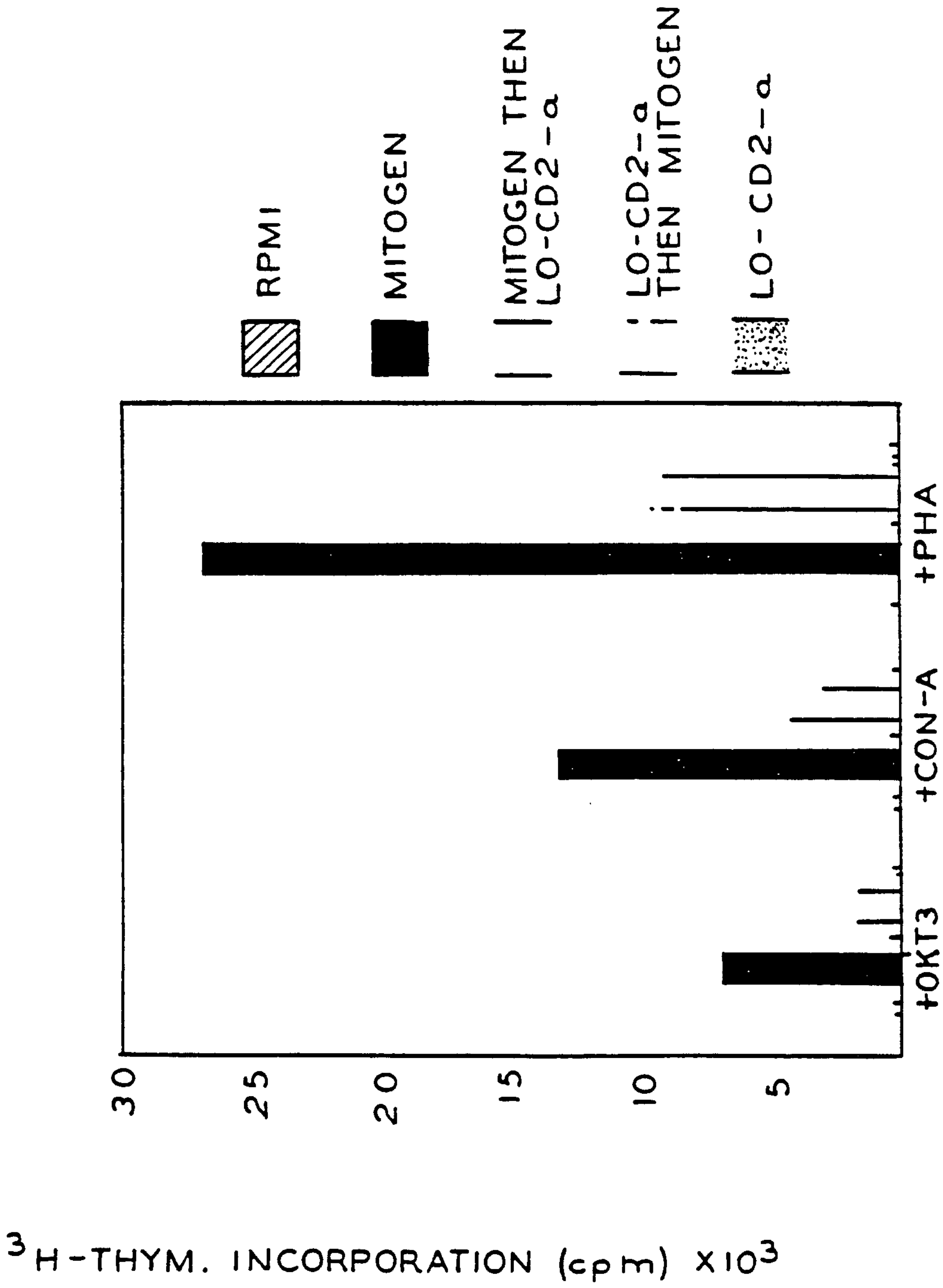
RPMI
MITOGEN
MITOGEN THEN LO-CD2-a
LO-CD2-a THEN MITOGEN
LO-CD2-a
30
25
20
15
10
5
+OKT3
+CON-A
+PHA
3H-THYM. INCORPORATION (cpm) X10^3

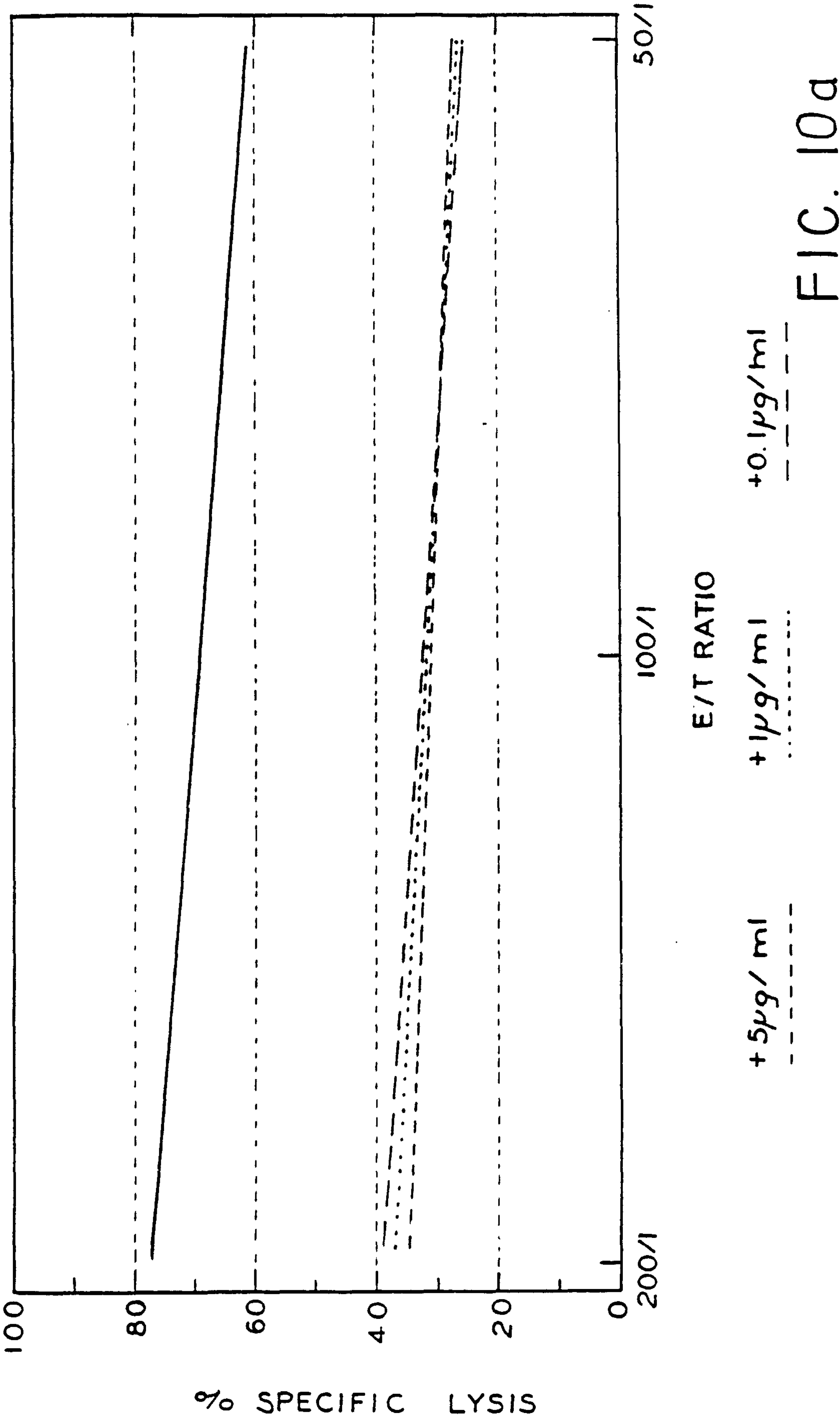
% SPECIFIC LYSIS
100
80
60
40
20
0
200/1
100/1
50/1
E/T RATIO
+5μg/ml
+1μg/ml
+0.1μg/ml

FIG. 10a

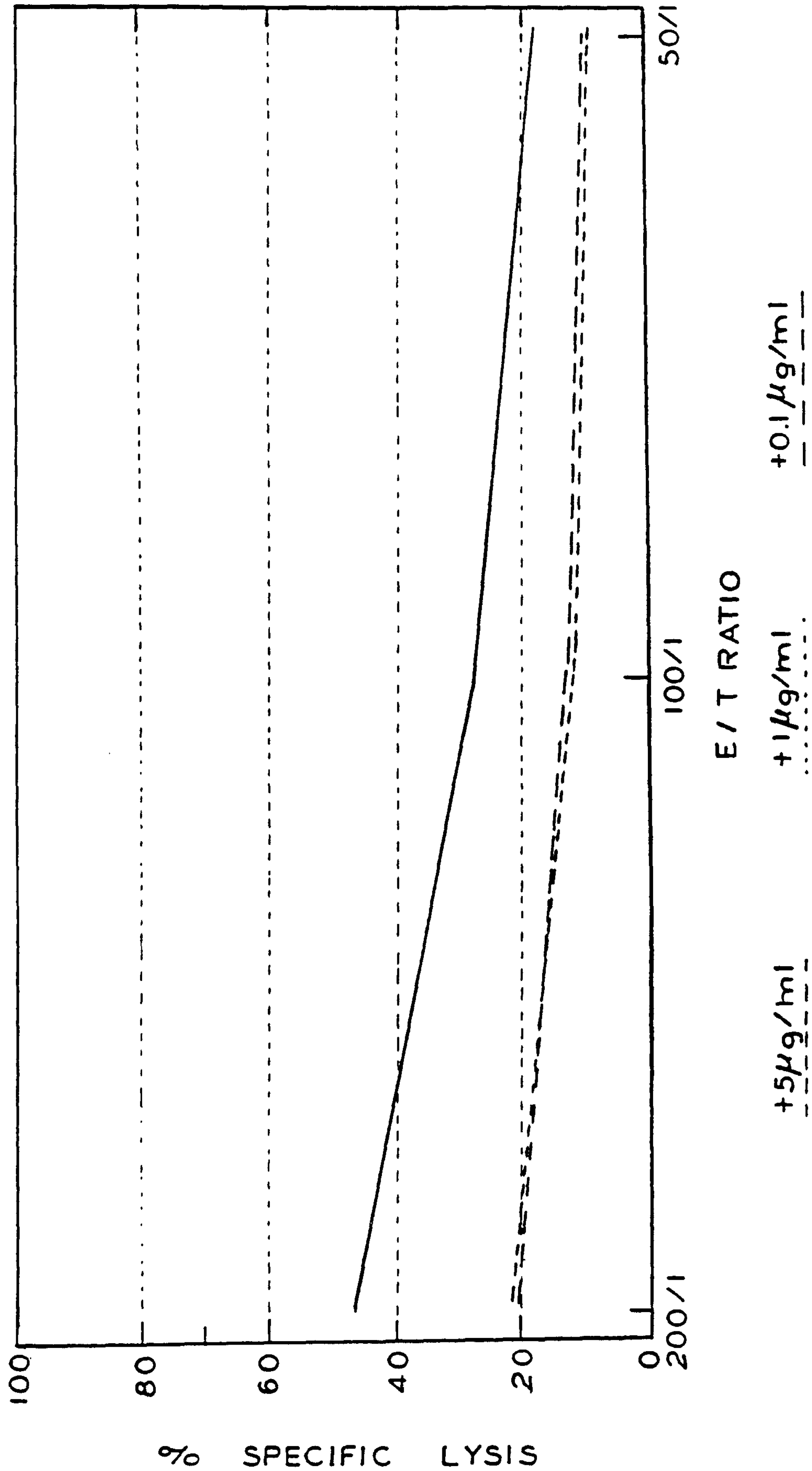
% SPECIFIC LYSIS
100
80
60
40
20
0
200/1
100/1
50/1
E/T RATIO
+5μg/ml
+1μg/ml
+0.1μg/ml

FIG. 10b

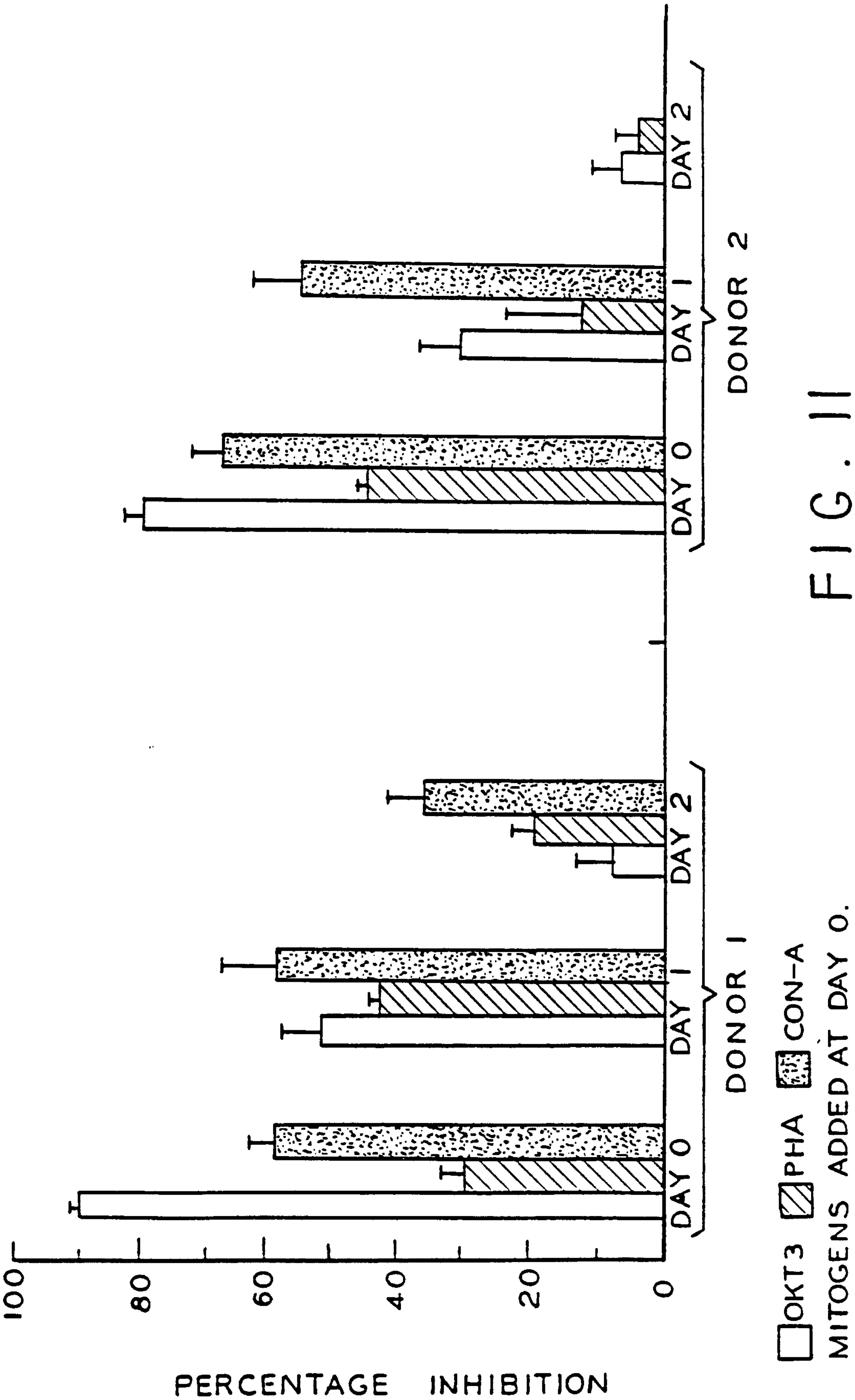
PERCENTAGE INHIBITION
0
20
40
60
80
100
DAY 0
DAY 1
DAY 2
DONOR 1
DONOR 2
OKT3
PHA
CON-A
MITOGENS ADDED AT DAY 0.

FIG. 11

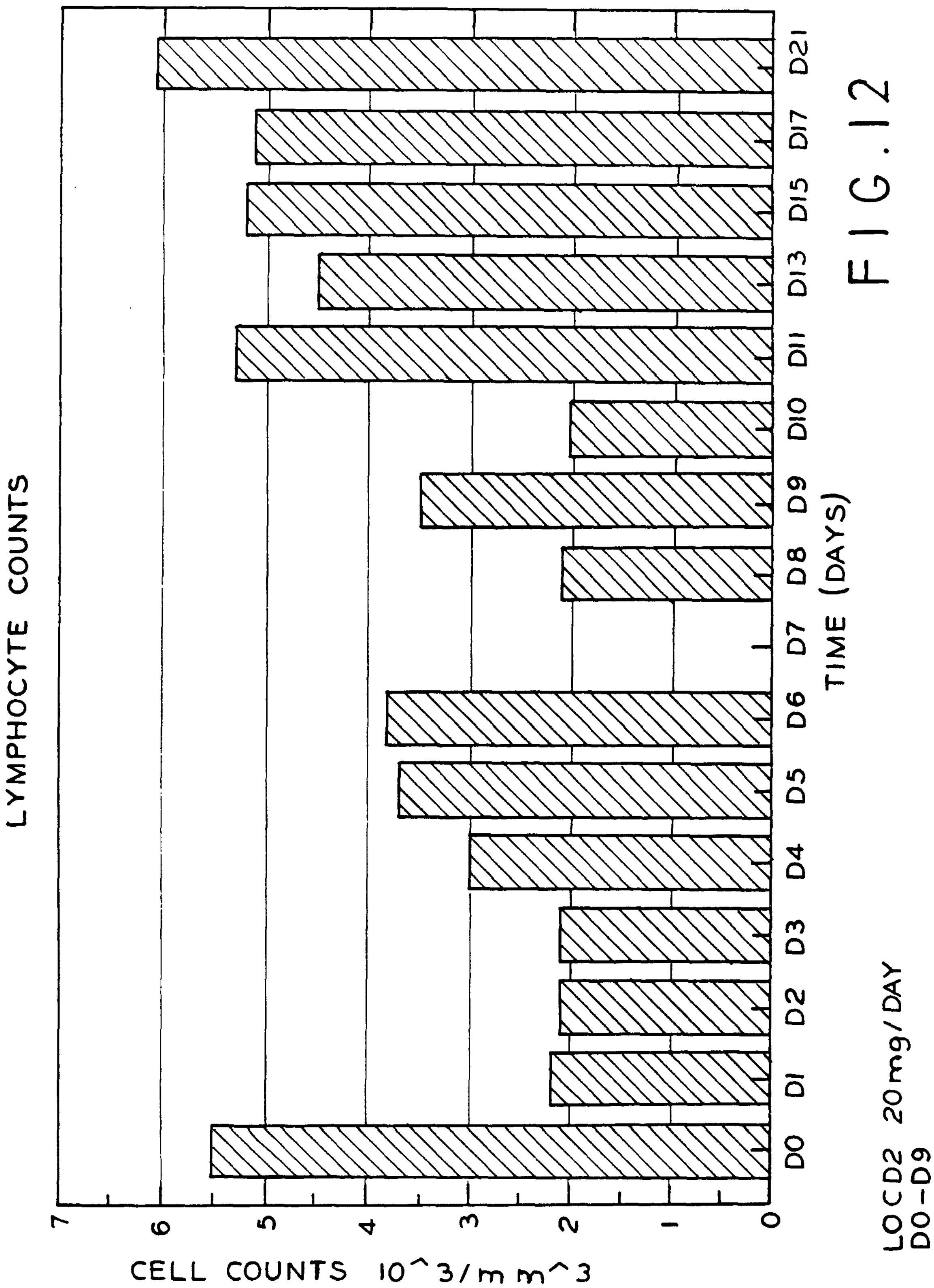


LOCD2 20mg/DAY
D0-D9

FIG. 12

FIG. 13

CELL POPULATIONS
LOCD2 : 20 mg/DAY
D0-D9

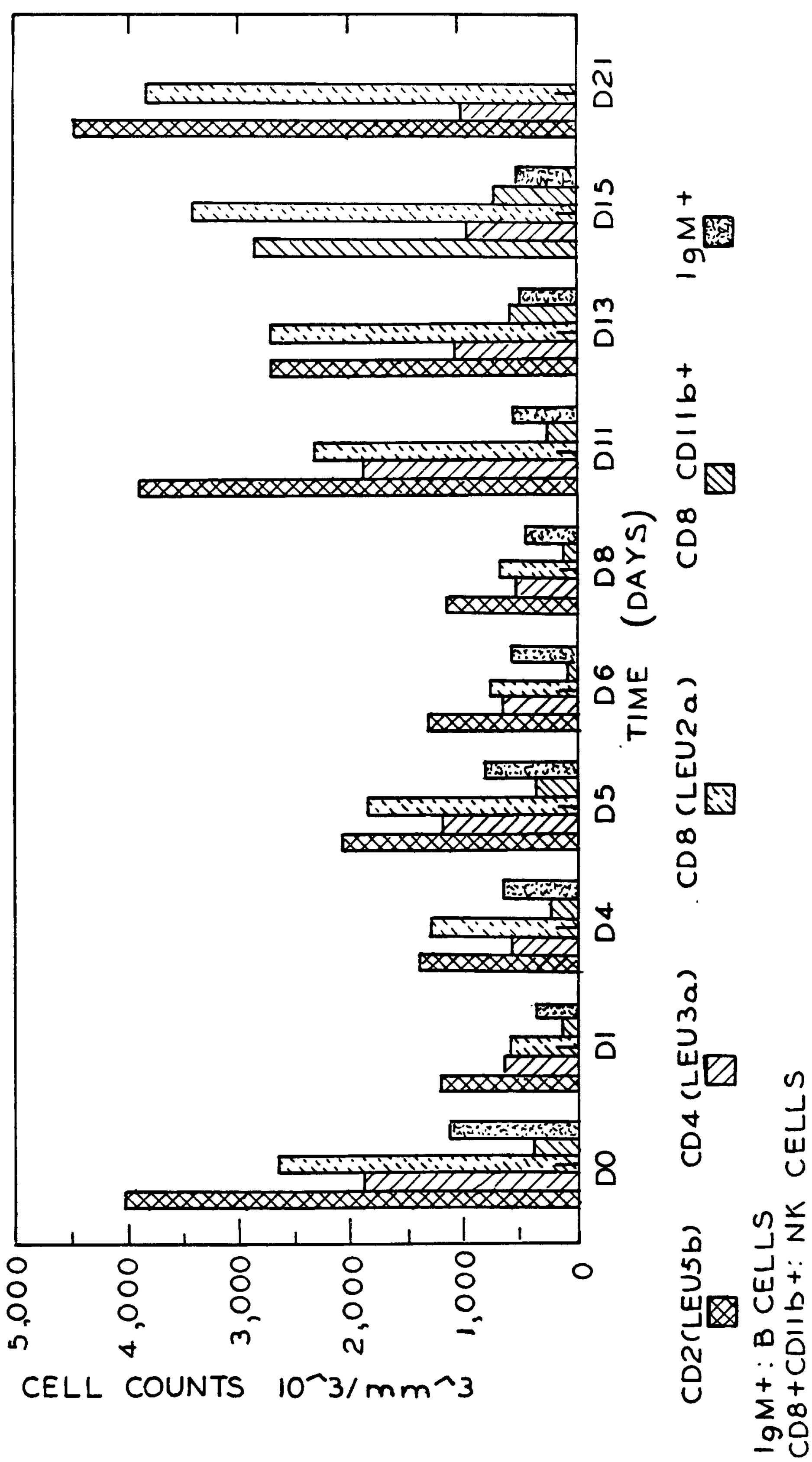

FIG. 14

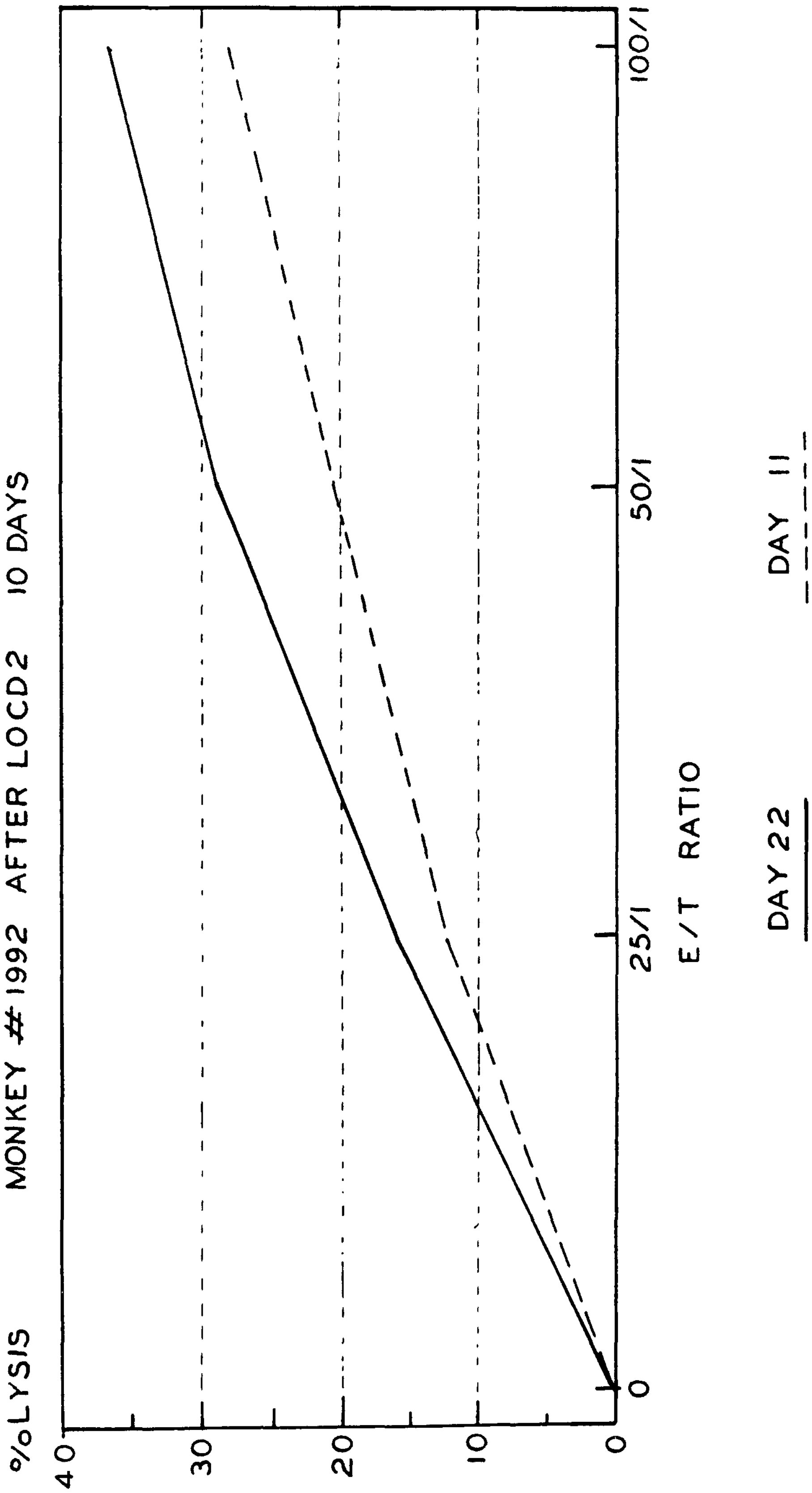
NK ACTIVITY
MONKEY #1992 AFTER LOCD2 10 DAYS
%LYSIS
40
30
20
10
0
0
25/1
50/1
100/1
E/T RATIO
DAY 22
DAY 11

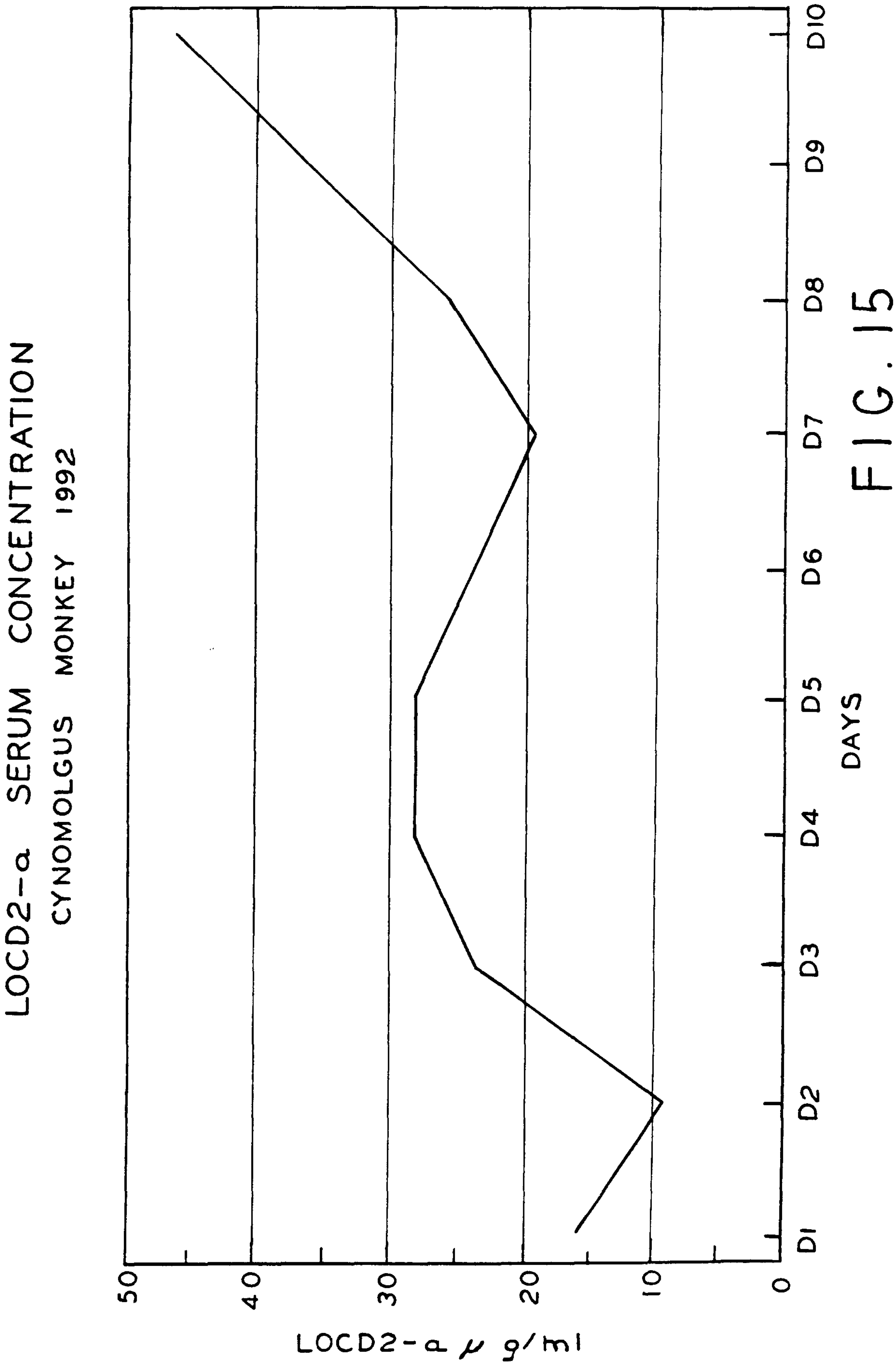
LOCD2-α SERUM CONCENTRATION
CYNOMOLGUS MONKEY 1992
LOCD2-α μg/ml
50
40
30
20
10
0
D1
D2
D3
D4
D5
D6
D7
D8
D9
D10
DAYS

FIG. 15

FIG. 16

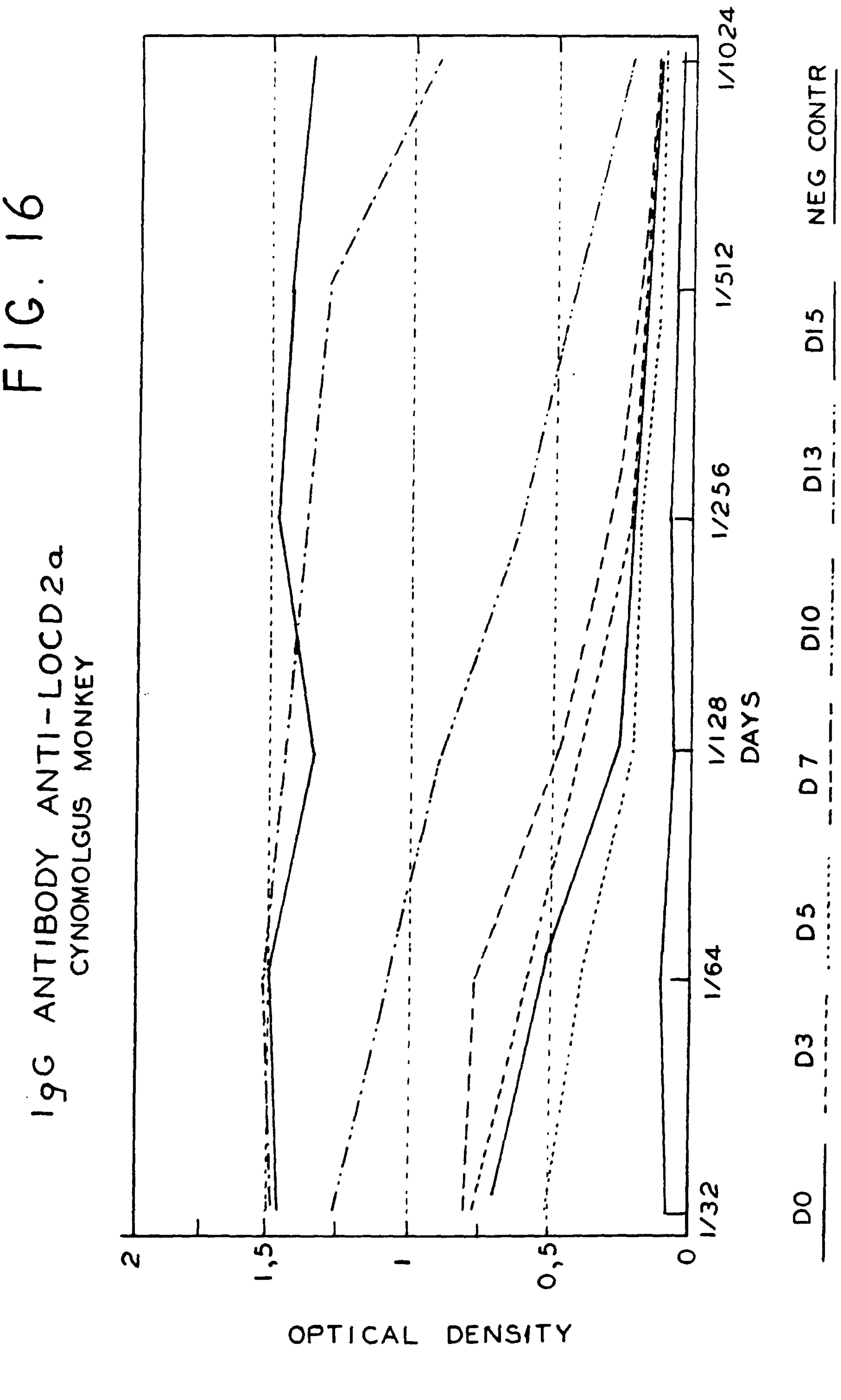
IgG ANTIBODY ANTI-LOCD2a
CYNOMOLGUS MONKEY
OPTICAL DENSITY
0
0,5
1
1,5
2
1/32
1/64
1/128
1/256
1/512
1/1024
DAYS
D0
D3
D5
D7
D10
D13
D15
NEG CONTR

FIG. 17B

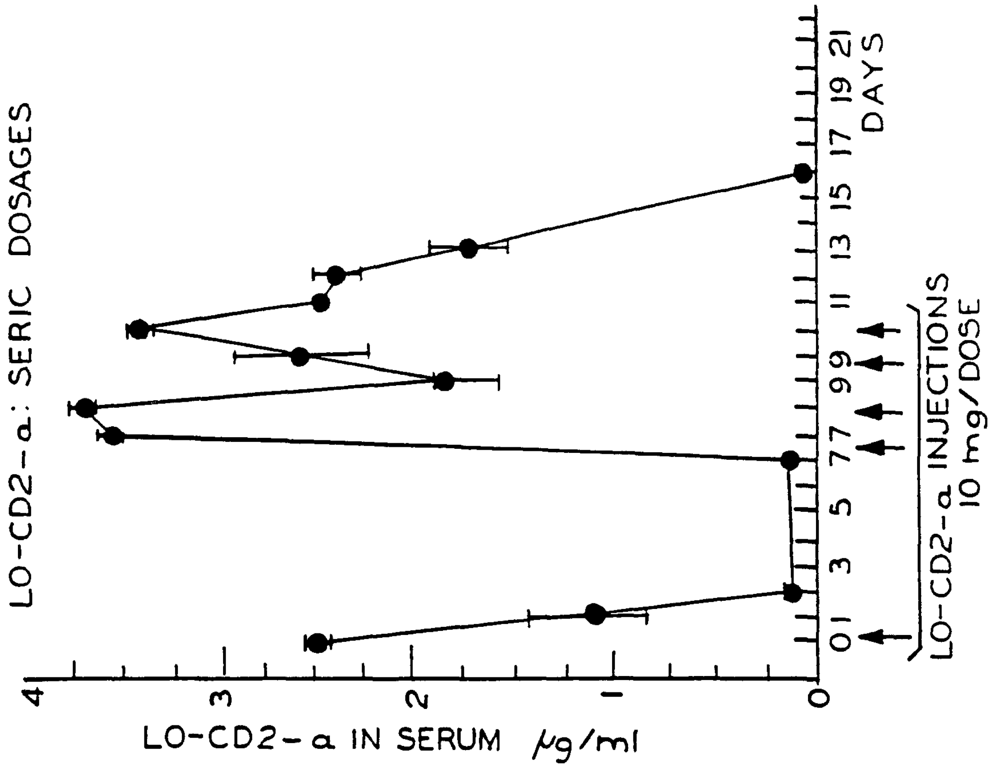


FIG. 17A

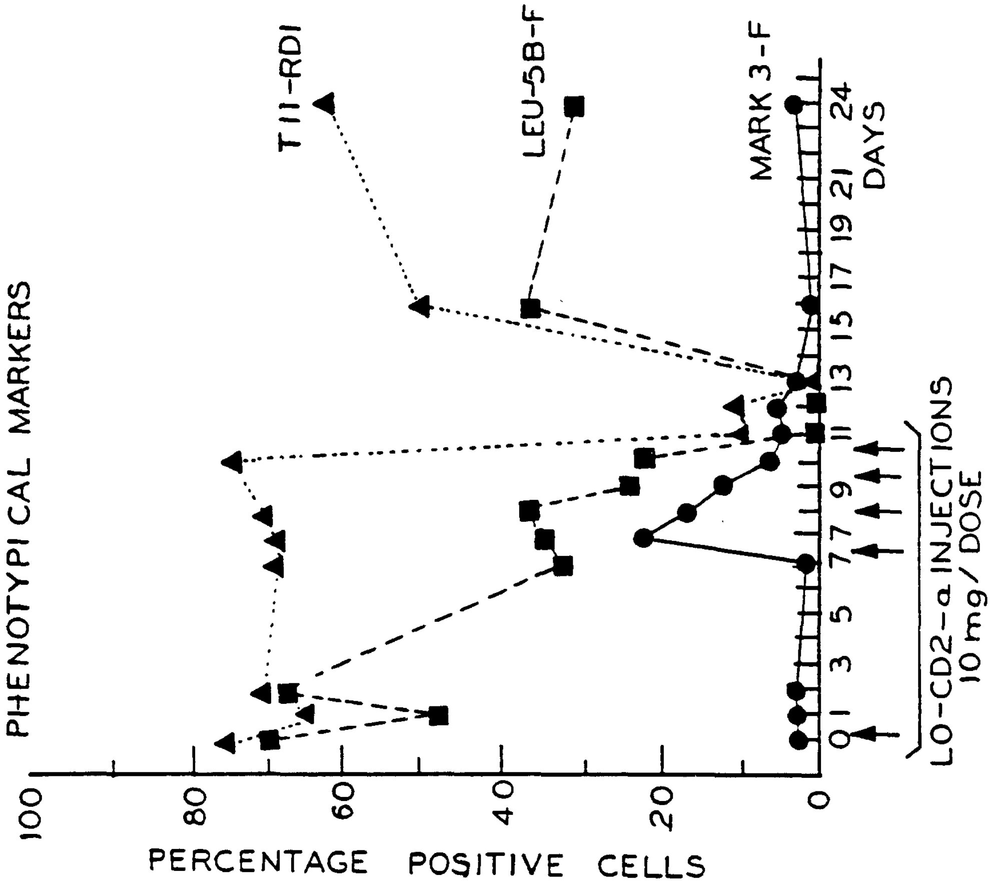

FIG. 18B

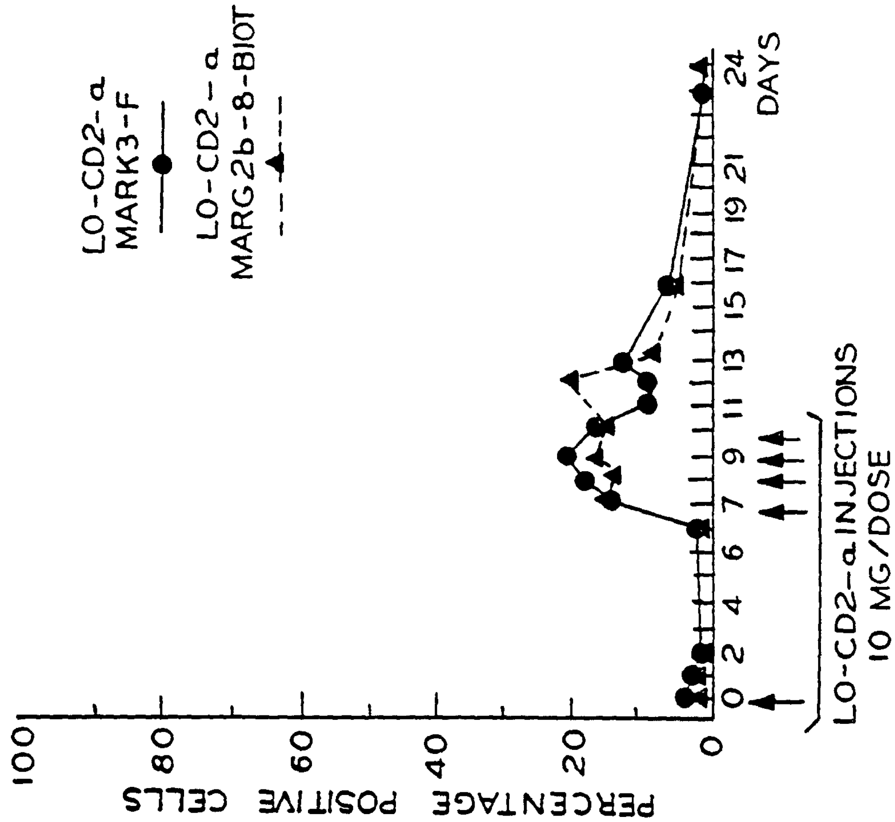


FIG. 18A

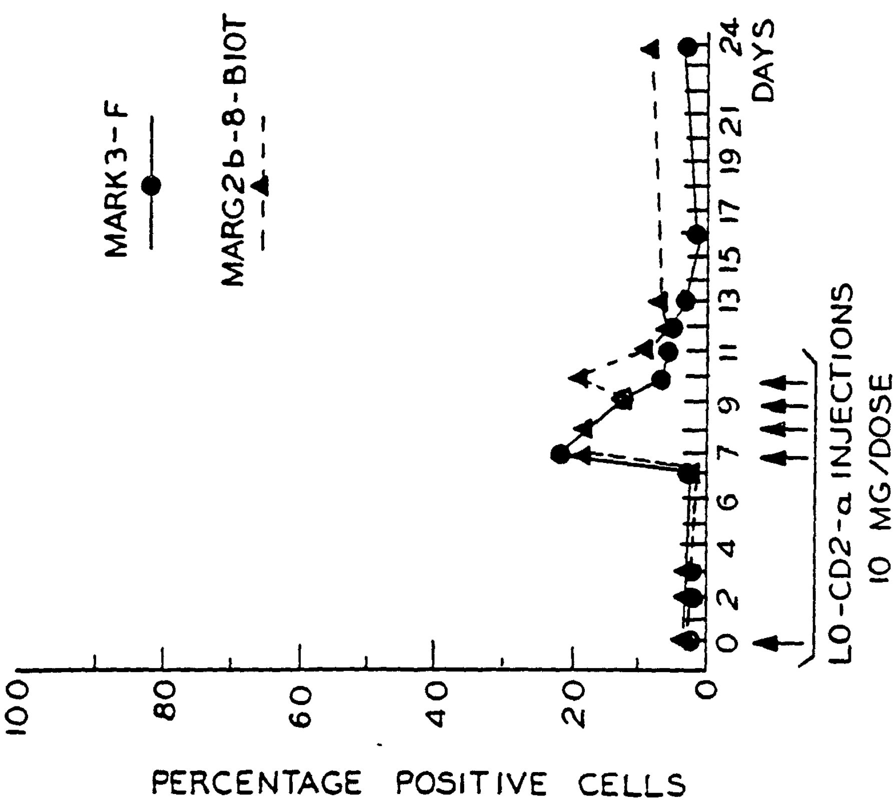

FIG. 19

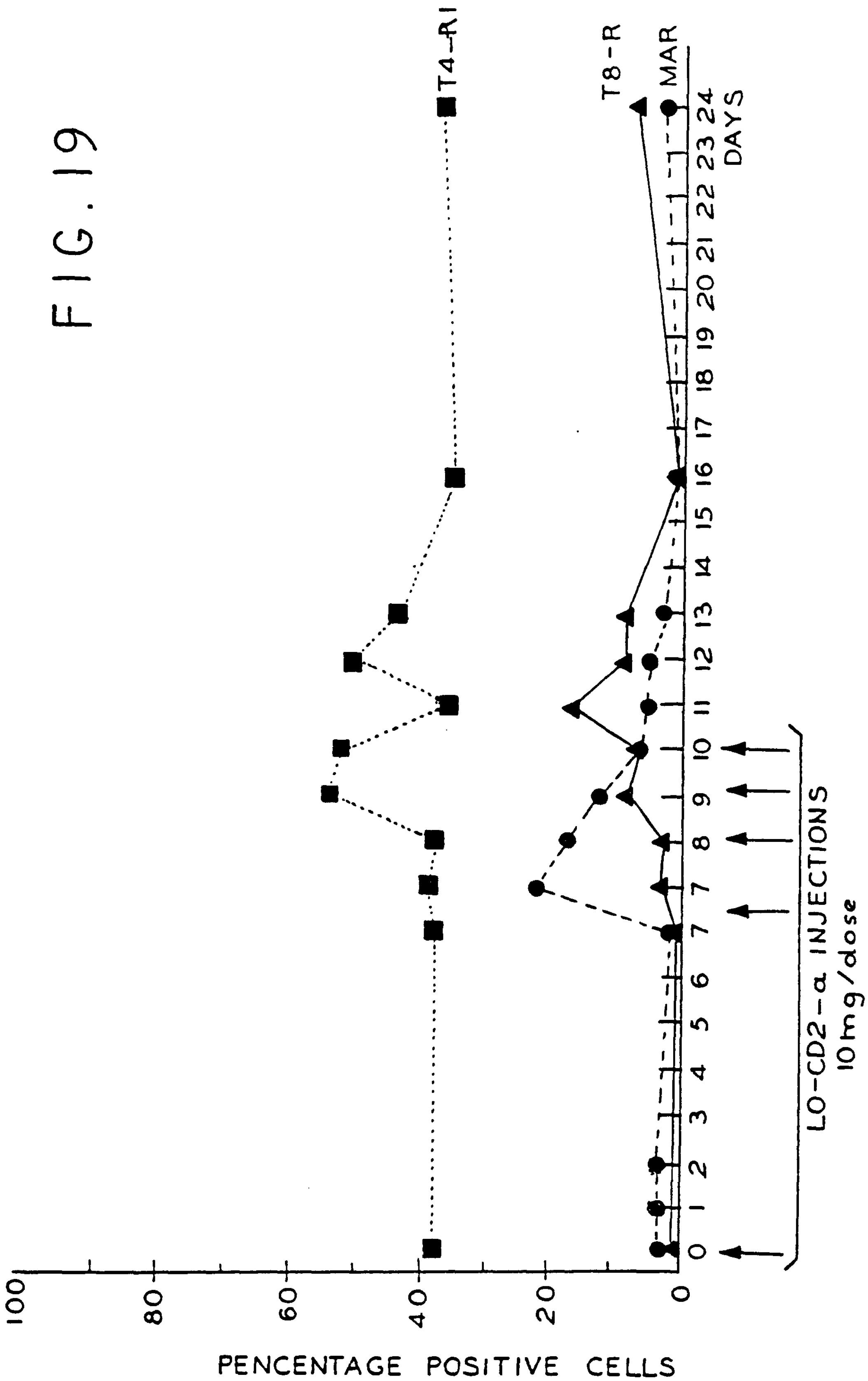
T4-R1
T8-R
MAR
DAYS
0 1 2 3 4 5 6 7 7 8 9 10 11 12 13 14 15 16 17 18 19 20 21 22 23 24
LO-CD2-α INJECTIONS
10mg/dose
100
80
60
40
20
0
PENCENTAGE POSITIVE CELLS

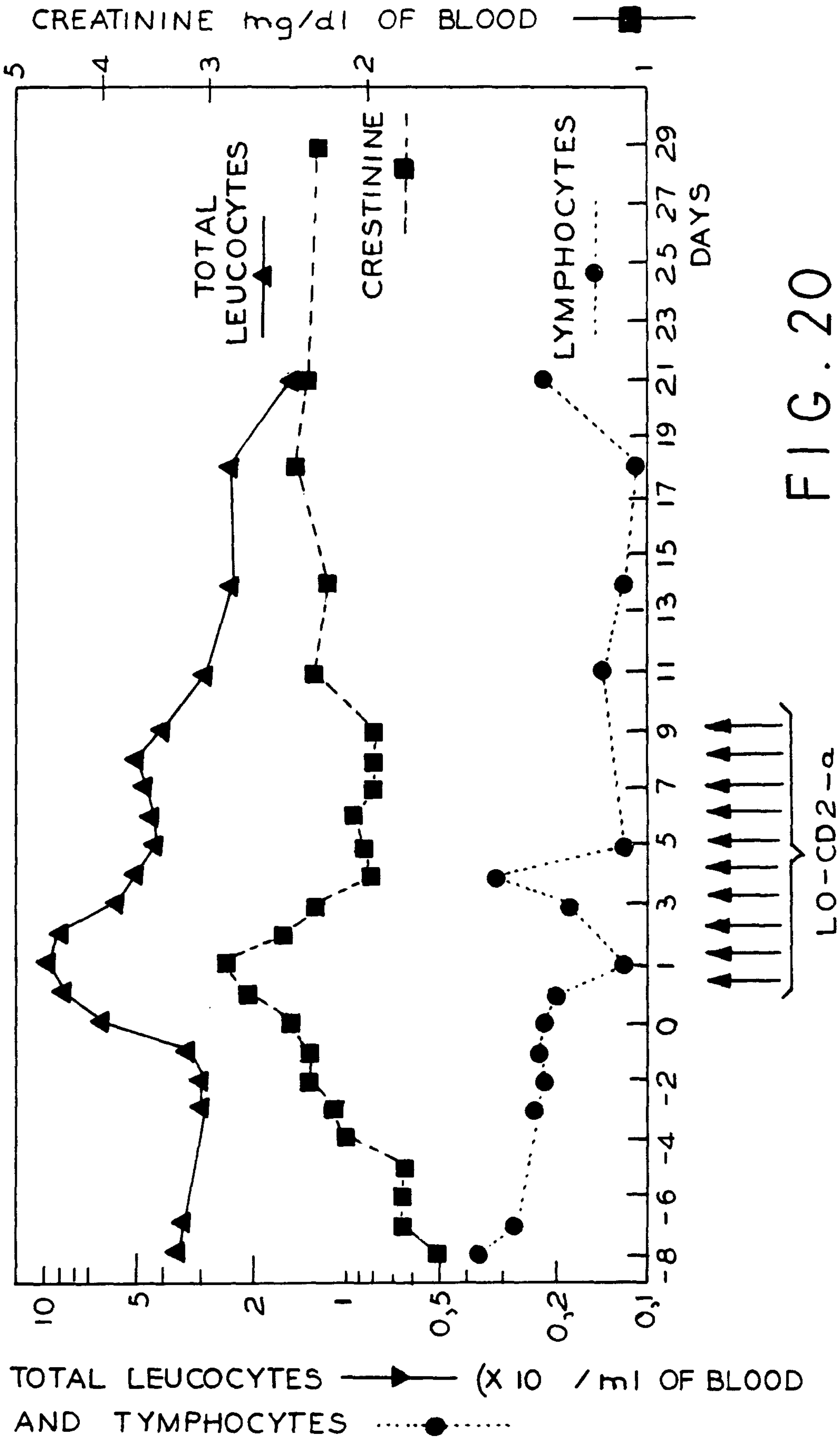
CREATININE mg/dl OF BLOOD
TOTAL LEUCOCYTES
CRESTININE
LYMPHOCYTES
DAYS
LO-CD2-a
TOTAL LEUCOCYTES (X 10 /ml OF BLOOD
AND TYMPHOCYTES

FIG. 20

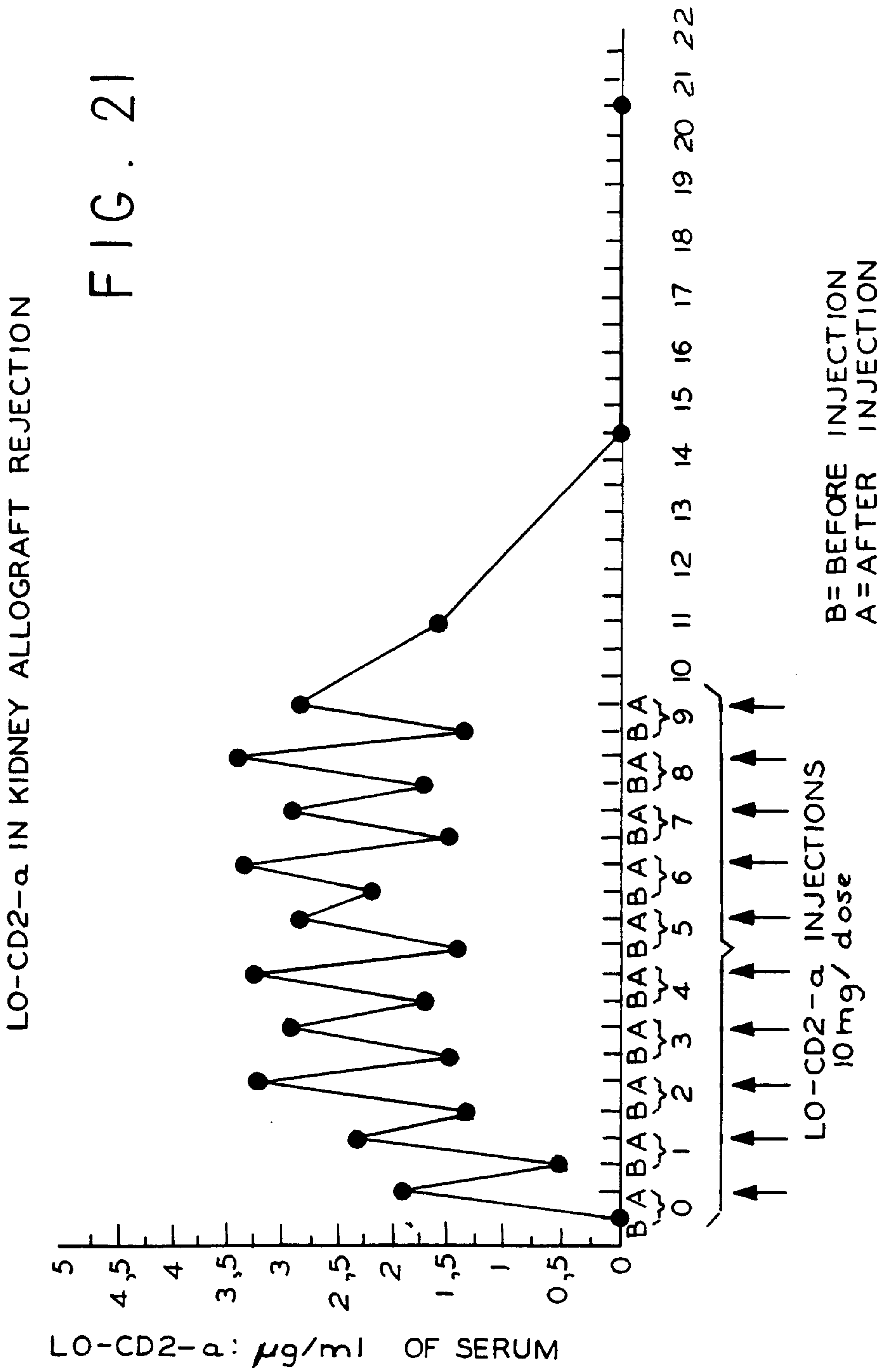
LO-CD2-a IN KIDNEY ALLOGRAFT REJECTION
LO-CD2-a: µg/ml OF SERUM
5
4,5
4
3,5
3
2,5
2
1,5
1
0,5
0
B A B A B A B A B A B A B A B A B A B A
0 1 2 3 4 5 6 7 8 9 10 11 12 13 14 15 16 17 18 19 20 21 22
LO-CD2-a INJECTIONS
10mg/ dose
B= BEFORE INJECTION
A=AFTER INJECTION

FIG. 21

FIG. 22

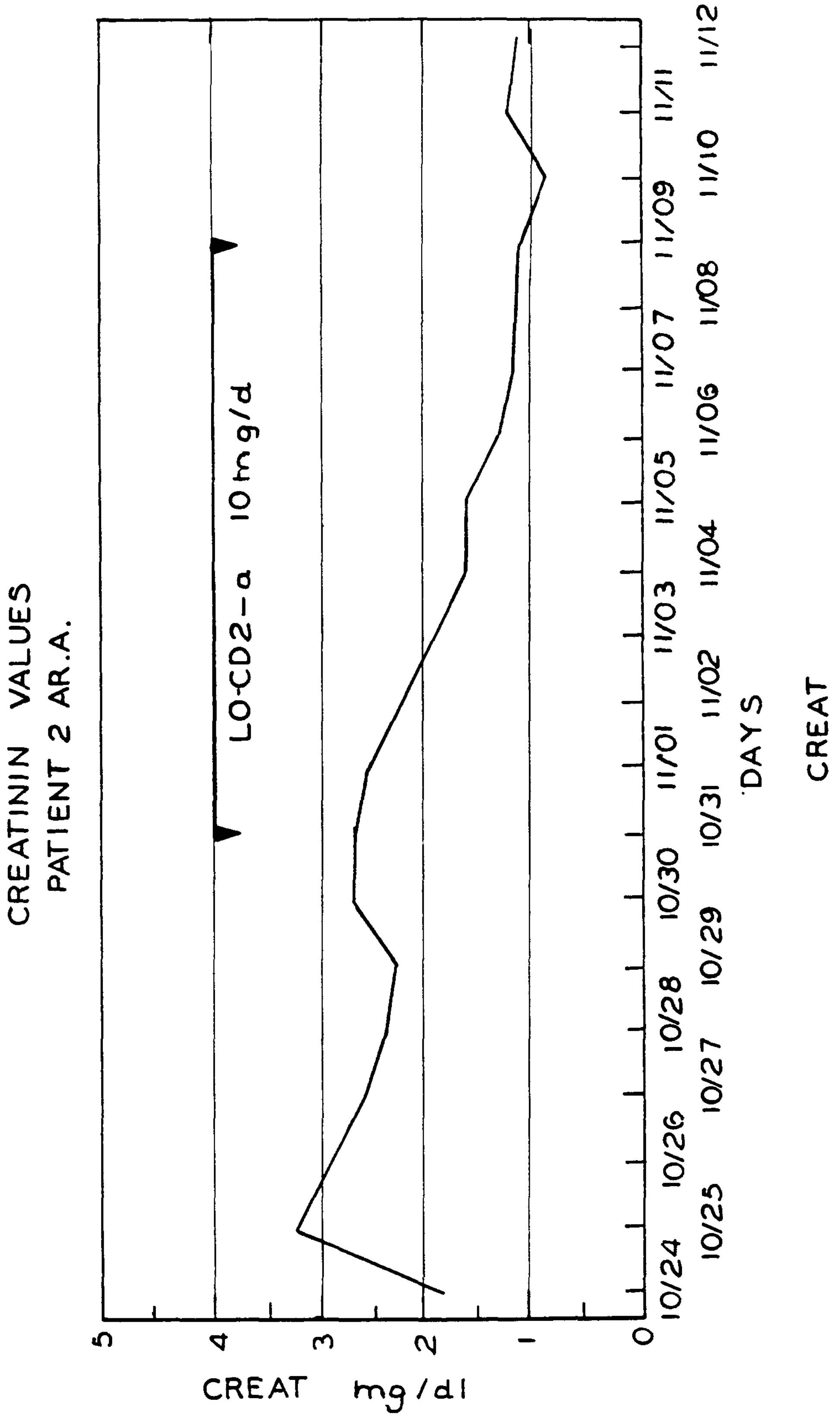
CREATININ VALUES
PATIENT 2 AR.A.
LO-CD2-a 10mg/d
CREAT mg/dl
5
4
3
2
1
0
10/24
10/25
10/26
10/27
10/28
10/29
10/30
10/31
11/01
11/02
11/03
11/04
11/05
11/06
11/07
11/08
11/09
11/10
11/11
11/12
DAYS
CREAT

FIG. 23

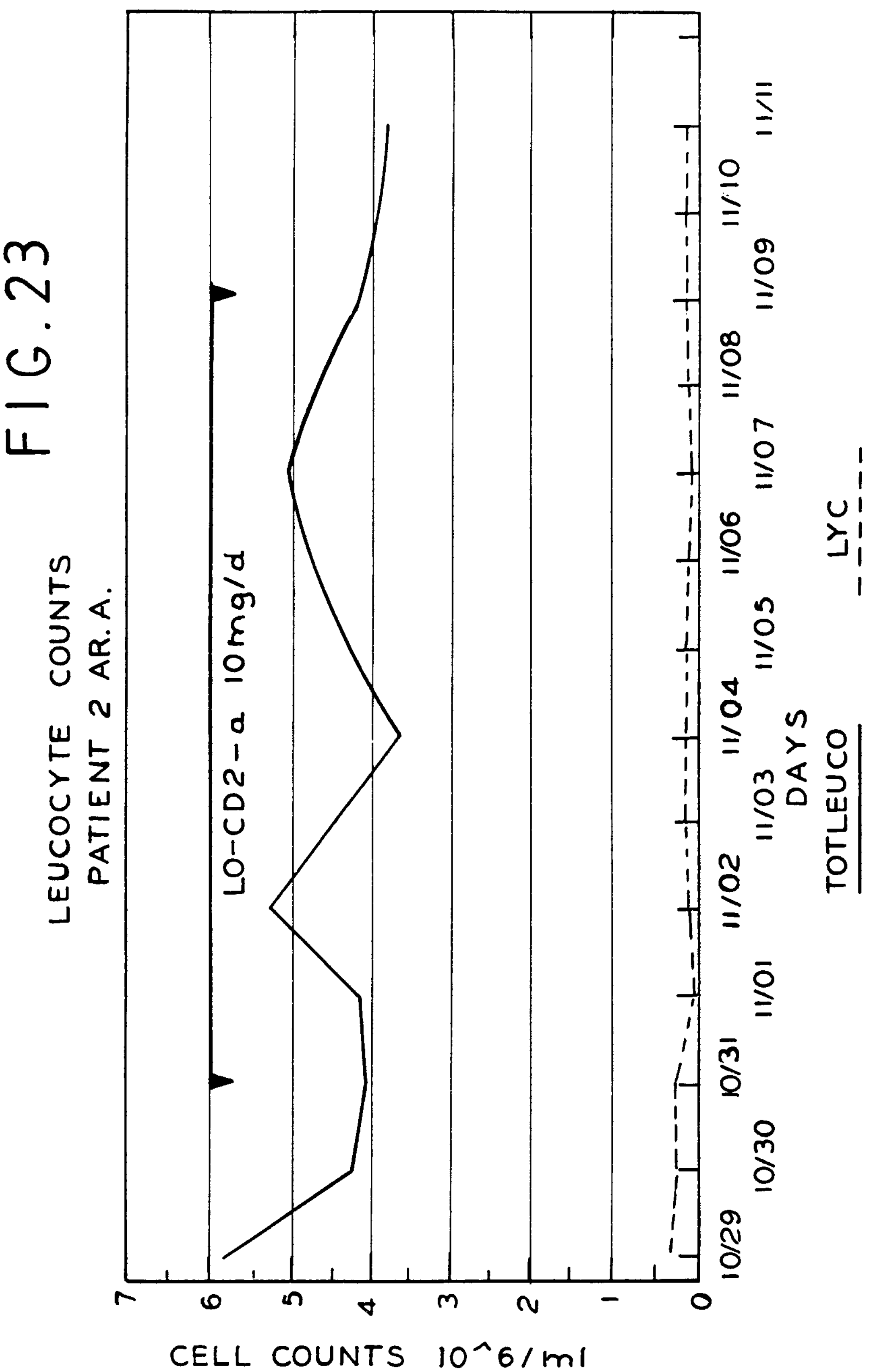
LEUCOCYTE COUNTS
PATIENT 2 AR. A.
LO-CD2-a 10mg/d
CELL COUNTS 10^6/ml
0
1
2
3
4
5
6
7
DAYS
10/29
10/30
10/31
11/01
11/02
11/03
11/04
11/05
11/06
11/07
11/08
11/09
11/10
11/11
TOTLEUCO
LYC

FIG. 24

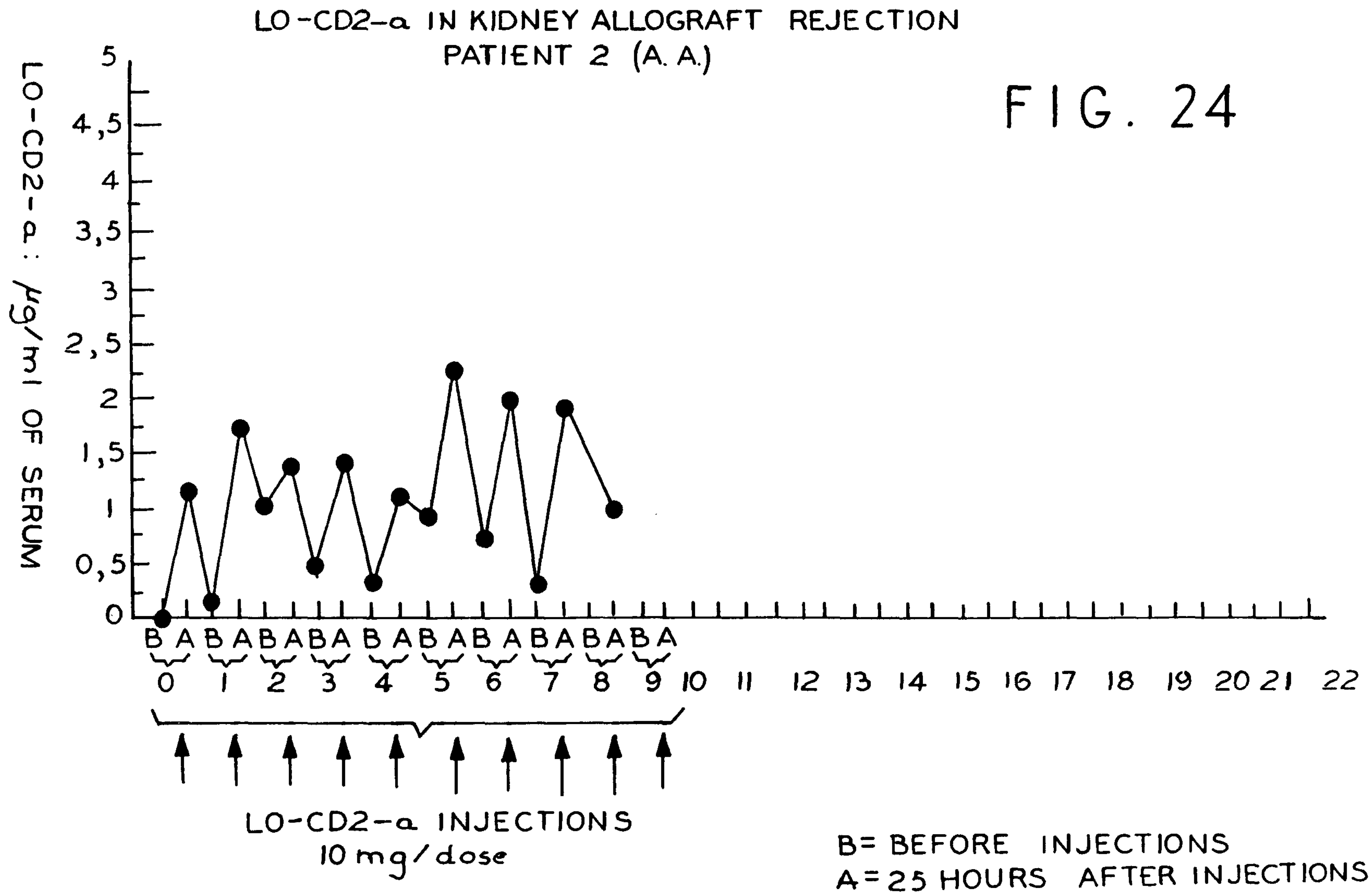
LO-CD2-a IN KIDNEY ALLOGRAFT REJECTION
PATIENT 2 (A.A.)
LO-CD2-a: μg/ml OF SERUM
0
0,5
1
1,5
2
2,5
3
3,5
4
4,5
5
B A B A B A B A B A B A B A B A B A B A
0 1 2 3 4 5 6 7 8 9 10 11 12 13 14 15 16 17 18 19 20 21 22
LO-CD2-a INJECTIONS
10 mg/dose
B= BEFORE INJECTIONS
A=25 HOURS AFTER INJECTIONS

FIG. 25

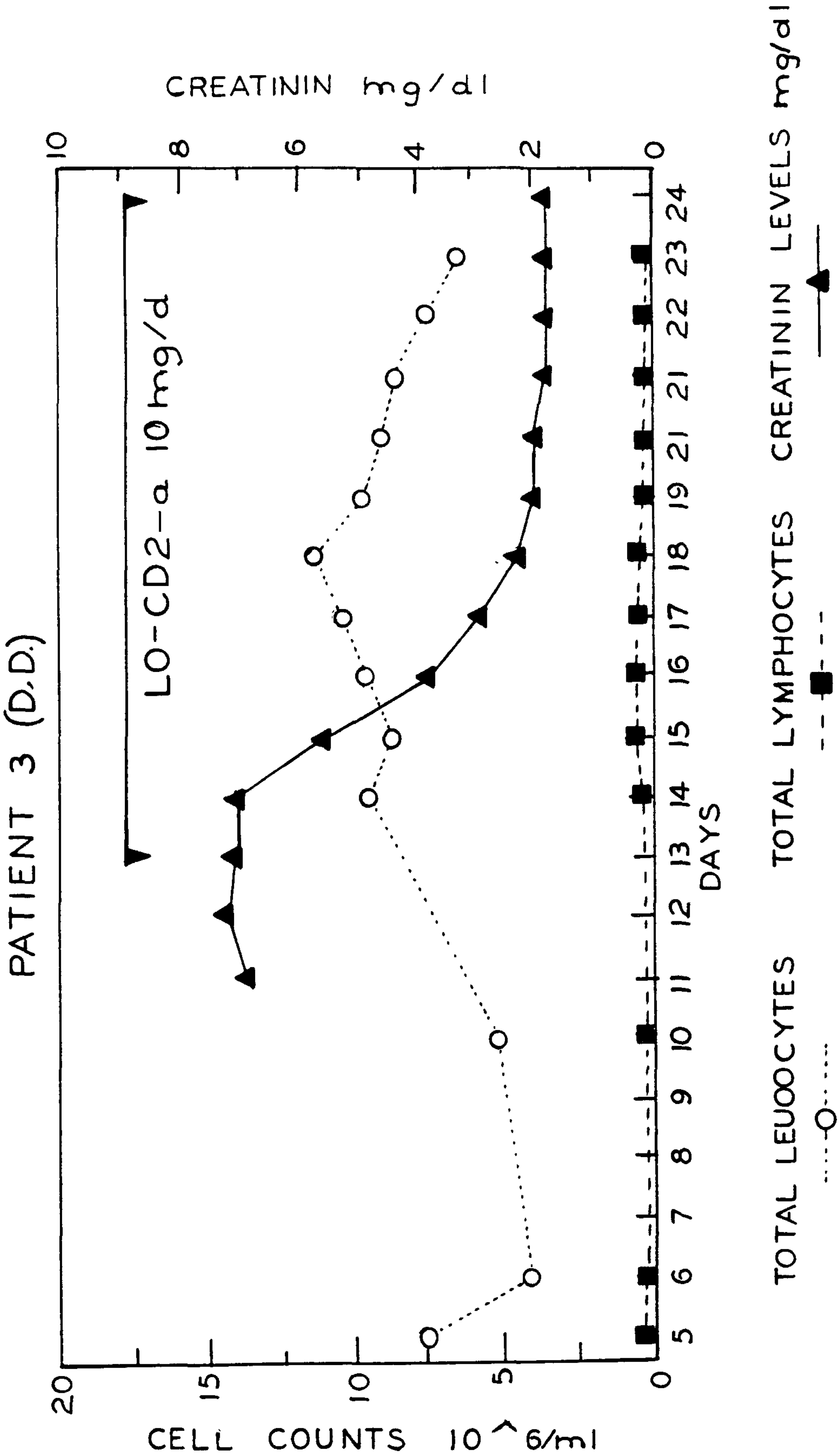
PATIENT 3 (D.D)
LO-CD2-a 10mg/d
CREATININ mg/dl
10
8
6
4
2
0
CELL COUNTS 10^6/ml
20
15
10
5
0
DAYS
5
6
7
8
9
10
11
12
13
14
15
16
17
18
19
21
21
22
23
24
TOTAL LEUOOCYTES
TOTAL LYMPHOCYTES
CREATININ LEVELS mg/dl

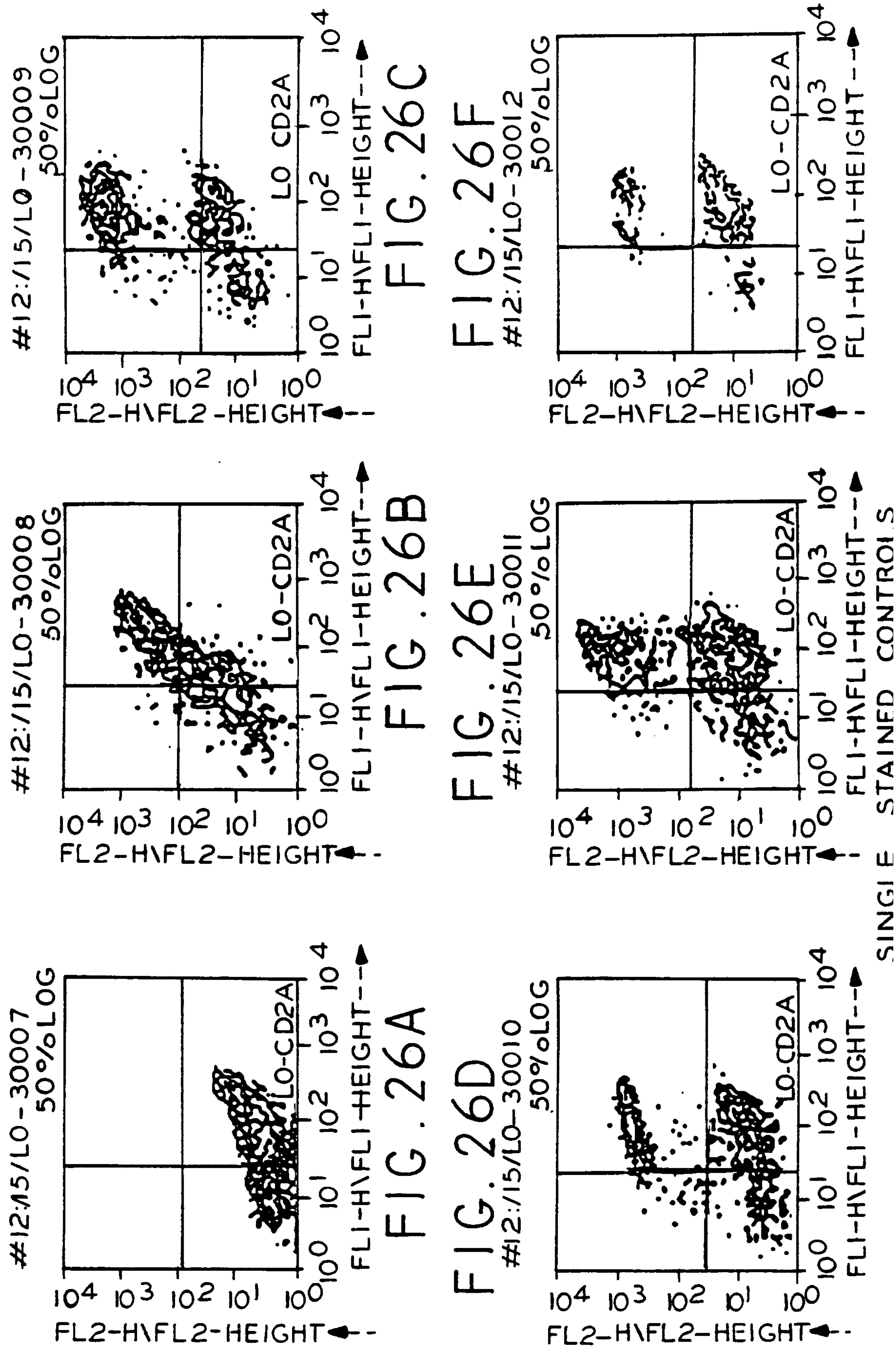


FIG. 26A

FIG. 26B

FIG. 26C

FIG. 26D

FIG. 26E

FIG. 26F

SINGLE STAINED CONTROLS

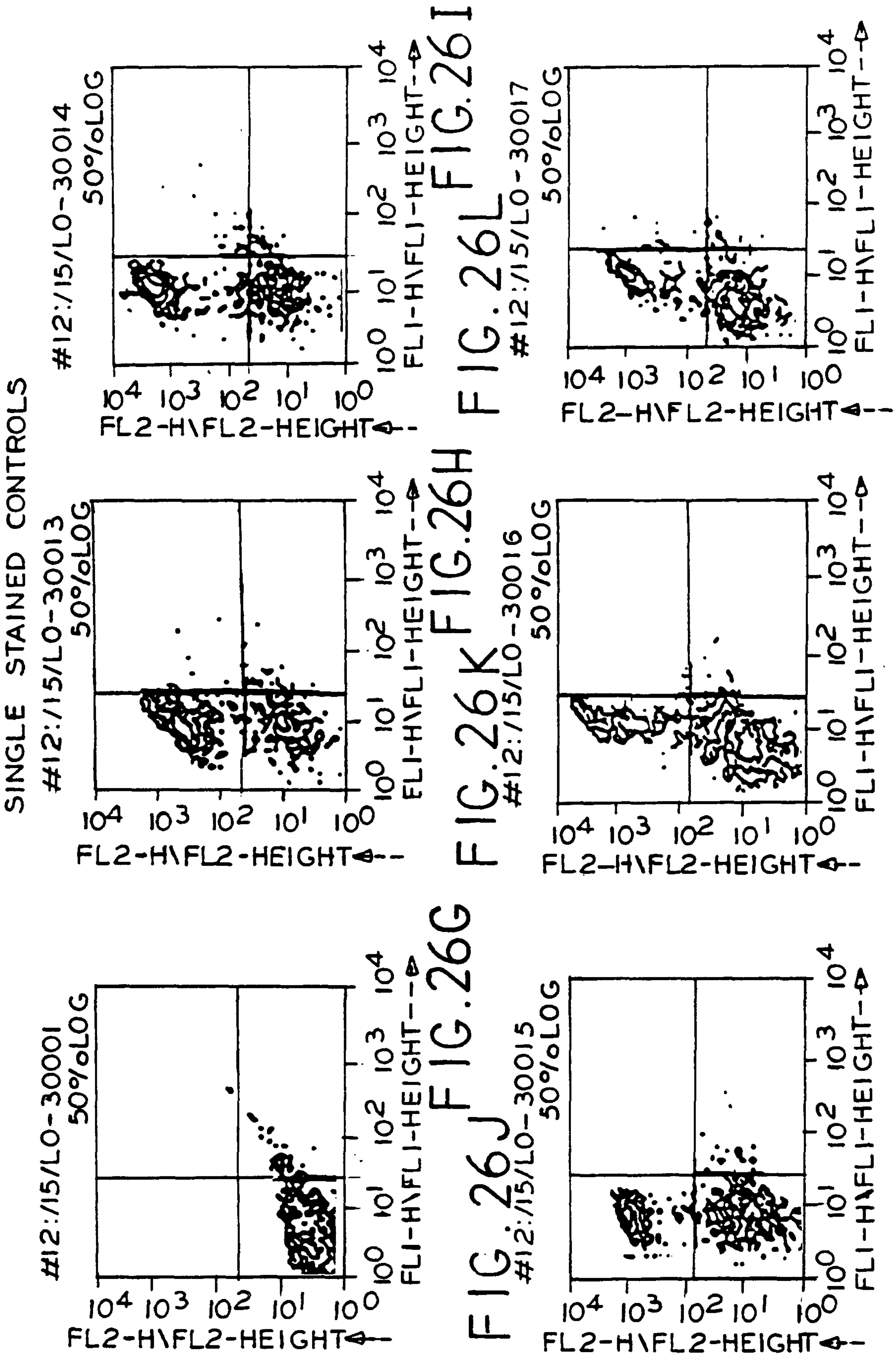


FIG. 26G

FIG. 26H

FIG. 26I

FIG. 26J

FIG. 26K

FIG. 26L

FIG. 27A

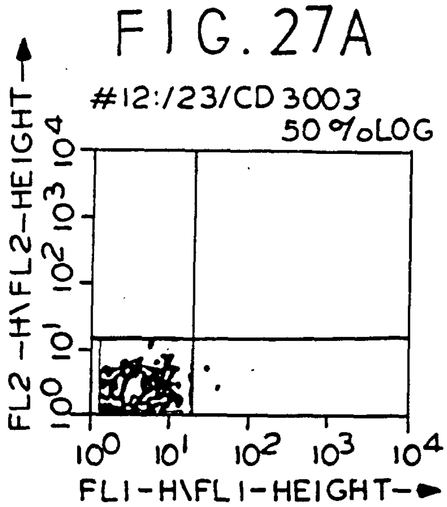


FIG. 27B

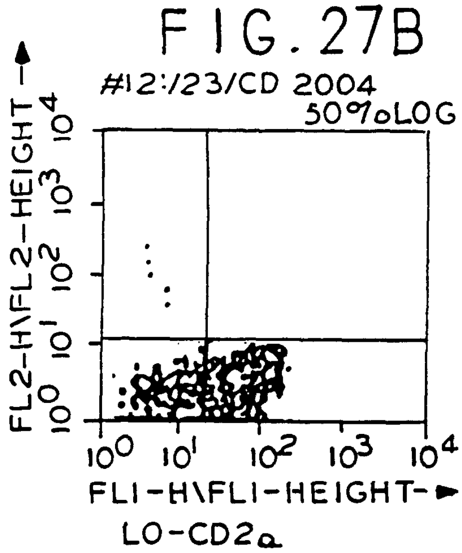


FIG. 27C

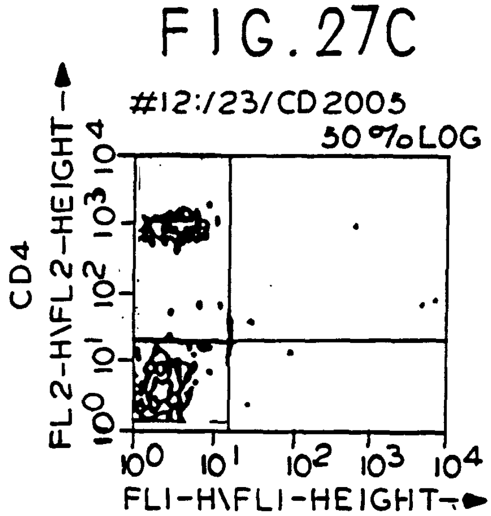


FIG. 27D

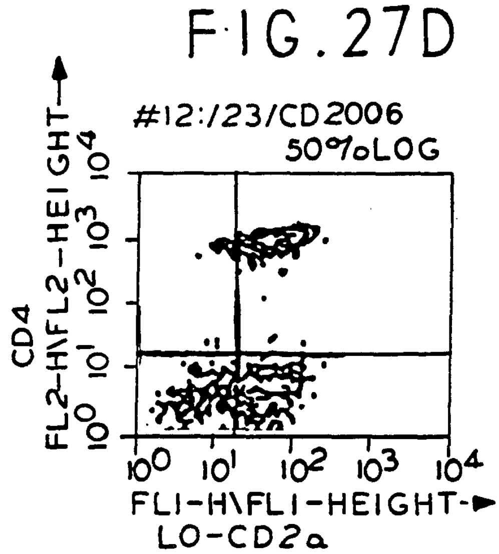


FIG. 27E

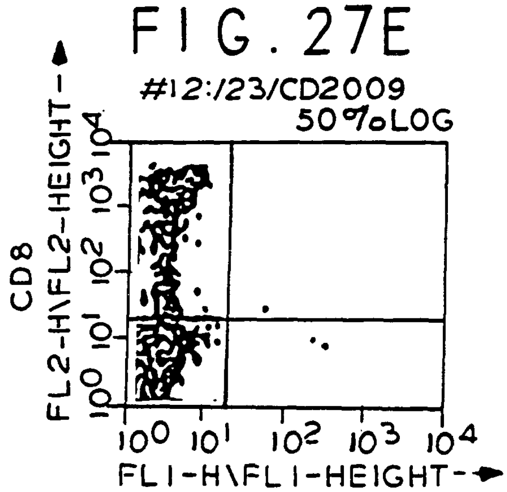


FIG. 27F

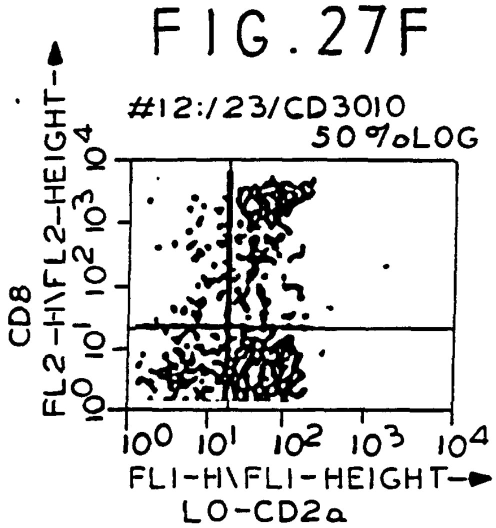

FIG. 27G

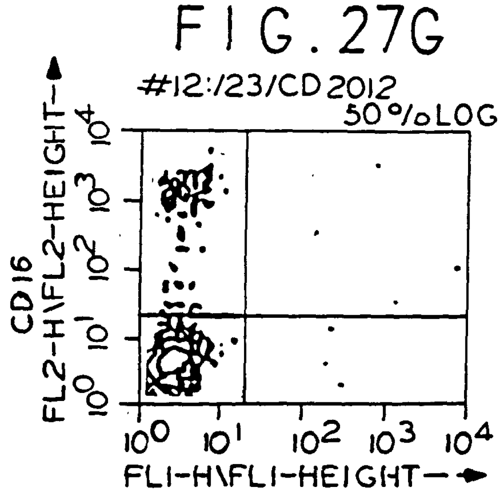


FIG. 27H

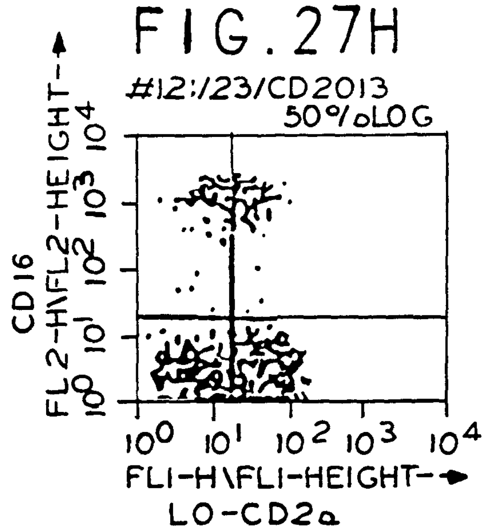


FIG. 27I

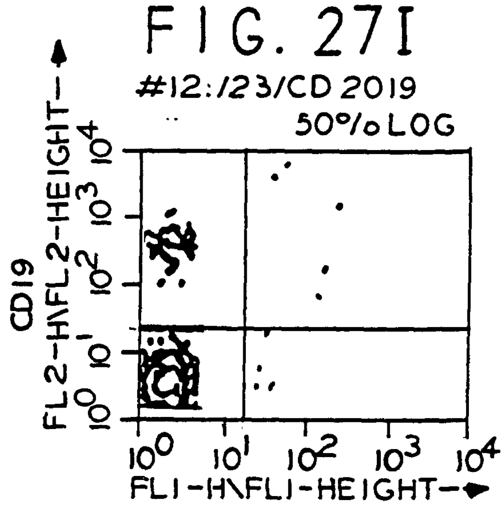


FIG. 27J

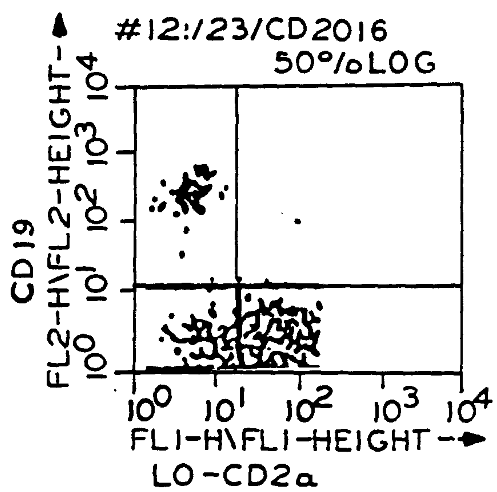


FIG. 27K

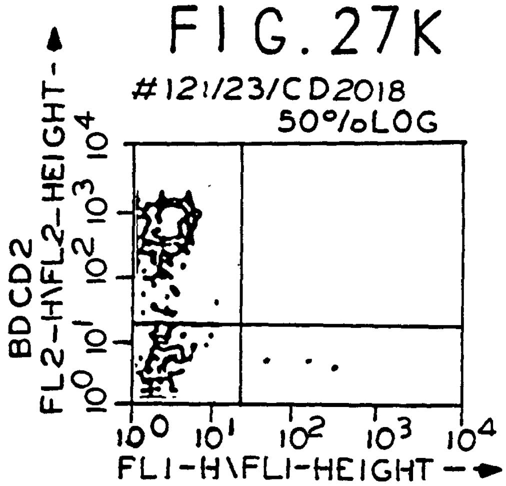


FIG. 27L

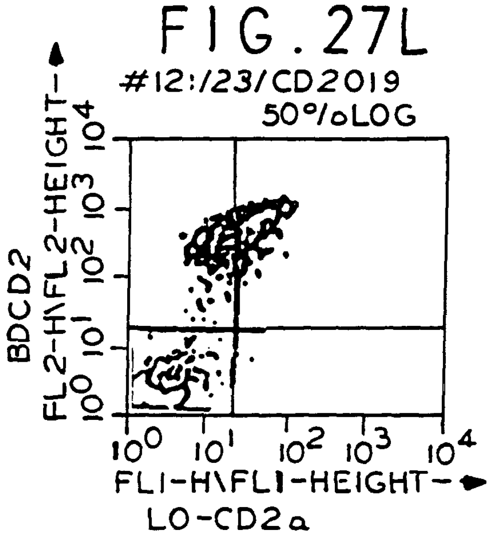

FIG. 28A

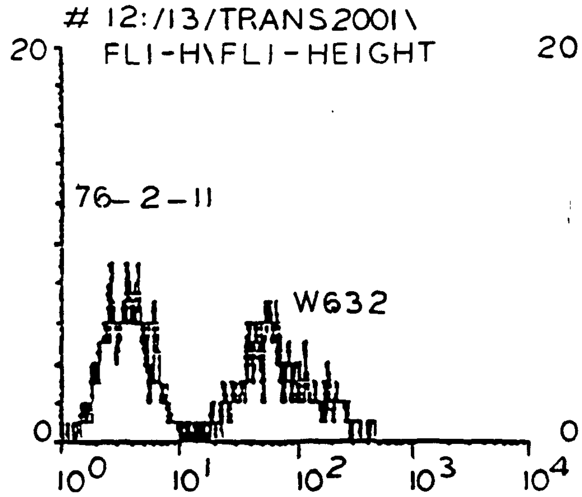


FIG. 28B

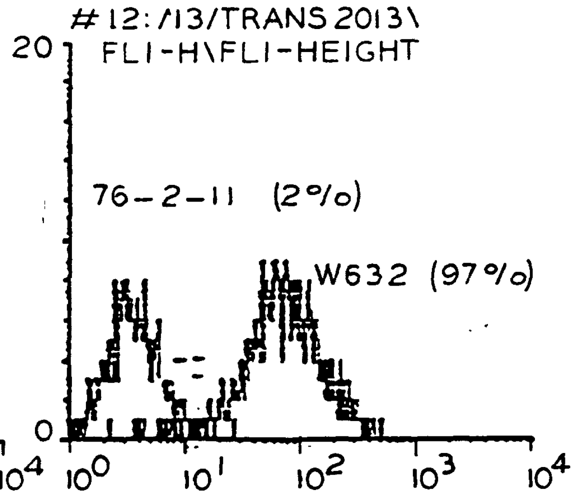


FIG. 28C

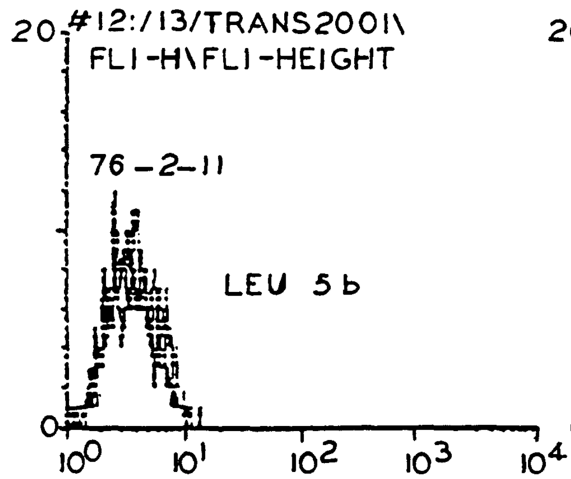


FIG. 28D

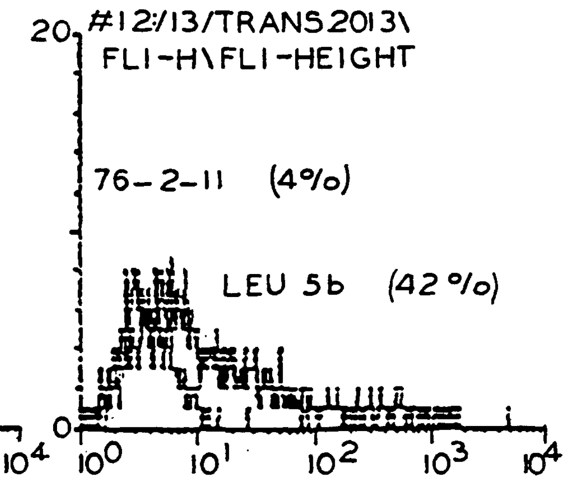


FIG. 28F

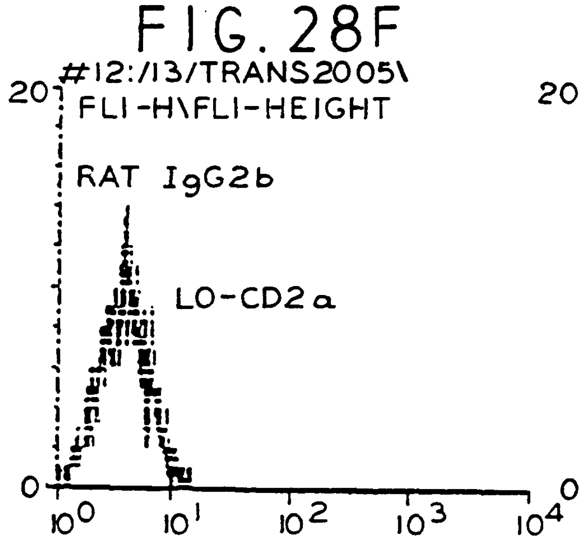


FIG. 28F

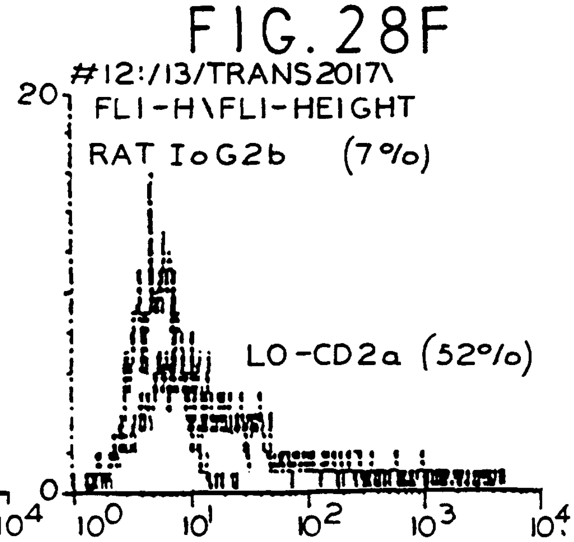

# FIG. 29Aa

Lo-CD2a $V_L$ WITH LEADER SEQUENCE FROM MRC VECTOR

```
         10        20        30        40        50        60
123456789012345678901234567890123456789012345678901234567890
ATGGATGGAGCTGTATCATCCTCTTCTTGGTAGCAACAGCTACAGGTAAGGGGCTCACA
M  G  W  S  C  I  I  L  F  L  V  A  T  A  T  C  K  G  L  T
 W  D  G  A  V  S  S  S  S  W  .  Q  Q  L  Q  V  R  G  S  Q
  G  H  E  L  Y  H  P  L  L  G  S  N  S  Y  R  .  G  A  H  S

CTCCACAGGTGTCCACTCCGACATCCAGCTGACCCAGTCTCCACCTACTTTATTGGCTAC
L  H  R  C  P  L     H  P  A  D  P  V  S  T  Y  F  I  G  Y
 S  T  G  V  H  S  D  I  Q  L  T  Q  S  P  P  T  L  L  A  T
  P  Q  V  S  T  P  T  S  S  .  P  S  L  H  L  L  Y  H  L  P

AAACACCTATTTAAATTGGTTGCTACAGAGGACAGGCCAATCTCCACAGCCGCTAATTTA
K  H  L  F  K  L  V  A  T  E  D     P  I  S  T  A  A  N  L
 N  T  Y  L  N  W  L  L  Q  R  T  G  Q  S  P  Q  P  L  I  Y
  T  P  I  .  I  G  C  Y  R  G  Q  A  M  L  H  S  R  .  F  I

AGATTTCACACTCAAAATCAGTGGACTGGAAGCTGAGGATTTGGGGCTTTATTACTGCAT
F  F  H  T  Q  N  Q  W  S  G  S  .  G  F  G  G  L  L  L  H
 D  F  T  L  K  I  B  G  V  E  A  E  D  L  G  V  Y  Y  C  M
  I  S  H     E  S  V  E  W  K  L  R  I  W  G  F  I  T  A  C
```

MATCH WITH FIG. 29Ab

# FIG. 29Ab

```
          70        80        90        100       110       120
123456789012345678901234567890123456789012345678901234567890
GTAGCAGGCTTGAGGTCTGGACATATATATGGGTGACAATGACATCCACCTTGCCTTTCT   120
V  A  G  L  R  S  G  H  I  Y  G  .  Q  .  H  P  L  C  L  S
 .  Q  A  .  G  L  D  I  Y  M  G  D  N  D  I  H  F  A  P  L
  S  R  L  E  V  W  T  Y  I  W  V  T  M  T  S  T  L  P  F  S

CATTGGACAATCAGTCTCCATCTCTTGCAGGTCAAGTCAGAGTCTCTTACATAGTAGTGG   240
H  W  T  I  S  L  H  L  L  Q  V  K  S  E  S  L  T  .  .  W
 I  G  Q  S  V  S  I  S  C  R  S  S  Q  S  L  L  H  S  S  G
  L  D  N  Q  S  P  S  L  A  G  Q  V  R  V  S  Y  I  V  V  E

TTTGGTATCCAAACTGGAATCTGGGGTCCCCAACAGGTTCAGTGGCAGTCGGTCAGGAAC   360
F  G  I  Q  T  G     W  G  P  Q  Q  V  Q  W  Q  W  V  R  N
 L  V  S  K  L  E  S  G  V  P  N  R  F  S  G  S  G  H  G  T
  W  Y  P  N  W  N  L  G  S  P  T  G  S  V  A  V  G  Q  E  Q

GCAATTTACCCATTATCCGTACACGTTTGGAGCTGGGACCAAGCTGGAACTGAAA        475
A  I  Y  P  L  S  V  H  V  W  S  W     Q  A  G  T  E
 Q  F  T  H  Y  P  Y  T  F  G  A  C  T  K  L  E  L  K
  N  L  P  I  I  R  T  R  L  E  L  G  P  S  W  N  .
```

MATCH WITH FIG. 29Aa

MATCH WITH FIG. 29Bb

```
Lo-CD2a VL + NATIVE LEADER SEQUENCE
          10        20        30        40        50        60
123456789012345678901234567890123456789012345678901234567890
ATGATGAGTCCTGTCCAGTCCCTGTTTCTGTTATTGCTTTGGATTCTGGGTAAGTAGAGA
M  M  S  P  V  Q  S  L  F  L  L  L  L  W     L  G  K     R
 .  .  V  L  S  S  P  C     C  Y  C  F  G  F  W  V  S  R  E
  D  E  S  C  P  V  P  V  S  V  I  A  L  D  S  G  .  V  E
CATGTGTGGTAAGGCAGGTCCTATTTTCTAAGATGGACACTTGAGATTCCATTACTTGAT
H  V  W  .  G  R  S  Y  F  L  R  W  T  L  E  I  P  L  L  D
 N  C  G  K  A  G  P     F  .  D  G  H  L  R     H  Y  L  I
  C  V  V  R  Q  V  L  F  S  K  M  D  T  .  D  S     T  .  .
GTGTATGCCATTTAGGATCTGCAACCGAATTGTLTTGTGAAAAAGCATT GGTATATTTT
V  Y  A  I  .  D  C  Q  P  N  C  F  V  X  K  H  L  V  Y  F
 C  M  P  F  R  I  C  N  R  I  V  L  .  K  S           I  F
  V  C  B  L     S  A  T  E  L  F  C  E  K  A  F  G  I  F  F
AAGATTGGTTGCAAATTTTGCACATAACTTT TTCTGATCTATTATAATTTCAGGAACCA
K  I  G  C  K  F  C  T  .  L  C  S  D  L  L  .  F  Q  E  P
 R  L  V  A  N  F  A  H  N  F  V  L  I  Y  Y  N  F  R     Q
  D  W  L  Q  I  L  H  I  T  L  F  .  S  I  I  I  S  G  T  N
TCTCCATCTCTTGCAGGTCAAGTCAGAGTCTCTTACATAGTAGTGGAAACACCTATTTAA
S  P  S  L  A  G  Q  V  R  V  S  Y  I  V  V  E  T  P  I  .
 L  H  L  L  Q  V  K  S  E  S  L  T  .  .  W  K  H  L  F  K
  S  I  S  C  R  S  S  Q  S  L  L  R  S  S  G  N  T  Y  L  N
TGGAATCTGGGGTCCCCAACAGGTTCAGTGGCAGTGGGTCAGGAACAGATTTCACACTCA
W  N  L  G  S  P  T  G  S  V  A  V  G  Q     Q  I  S  H  S
 G  I  W  G  P  Q  Q  V  Q  Q  Q  W  V  R  N  R  F  H  T  Q
  E  S  G  V  P  N  H  F  S  G  S  G  S  G  T  D  F  T  L  K
ATCCGTACACGTTTGGAGCTGGGACCAAGCTQGAACTGAAA
I  R  T  R  L  E  L  G  P  S  W  N  .
 S  V  H  V  W  S  W  D  Q  A  G  T  E
  P  Y  T  F  G  A  G  T  K  L  E  L  K
```

FIG. 29Ba

```
          70        80        90        100       110       120
123456789012345678901234567890123456789012345678901234567890
ATGAGTTACAGGACAA AATGGGGATGGAGGATGAGTTCTGACTGCCCATGTTGGCTCTC  120
M  S  Y  R  T  R  M  G  N  E  D  E  F  .  L  P  M  L  A  V
 .  V  T  G  Q  E  W  G  W  R  M  S  S  D  C  P  C  W  L  E
  E  L  Q  D  K  N  G  D  G  G  .  V  L  T  A  H  V  G  C  P
AATGAGAAATTACAGATGAGATAGGATTTGTGCTAAGAGGATTCTAATGTAGATGAGAAG  240
  E  K  L  Q  M  R  .  D  L  C  .  E  D  S  N  V  D  E  K
 H  R  N  Y  R  .  D  R  I  C  A  K  R  I  L  M  .  M  R  N
  .  E  I  T  D  E  I  G  F  V  L  R  G  F  .  C  R  .  E  G
TTAAAAATCACAAAACACACCGGGATCTCACAGGAAATGAGTAACAAAAAGTAATTCACA  360
L  K  I  T  K  H  T  G  I  S  Q  E     S     K  K  .  F  T
 .  K  S  Q  N  T  P  G  S  H  R  K  .  V  P  K  S  N  S  Q
  K  N  H  K  T  H  R  D  L  T  G     E  .  Q  K  V  I  H  K
ATGGTGATGTTGTGCTGACCCAGACTCCACCTACTTTATTGGCTACCATTGGACAA CAG  480
M  V  M  L  C  .  P  R  L     L  L  Y  W  L  P  L  D  N  Q
 W  .  C  C  A  D  P  D  S  T  Y  F  I  G  Y  H  W  T  I  S
  G  D  V  V  L  T  Q  T  P  P  T  L  L  A  T  I  G  Q  S  V
ATTGGTTGCTACAGAGGACAGGCCAATCTCCACACCCGCTAATTTATTTGGTATCCAAAC  600
I  G  C  Y  R  G  Q  A  N  L  N  S  R  .  F  I  W  Y  P  N
 L  V  A  T  E  D  R  P  I  S  T  A  A  N  L  F  G  I  Q  T
  W  L  L  Q  R  T  G  Q  S  P  Q  P  L     Y  L  V  S  K  L
AAATCAGTGGAGTGGAAGCTGAGGATTTGGGGGTTTATTACTGCATGCAATTTACCCATT  720
K  S  V  E  W  K  L  R  I  N  G  F  I  T  A  C  N  L  P  I
 N  Q  W  S  G  S  .  G  F  G  G  L  L  L  H  A  I  Y  P  L
  I  S  G  V  E  A  E  D  L  G  V  Y  Y  C  N  Q  F  T  H  Y
                                                              761
```

FIG.29Bb

MATCH WITH FIG. 29Ba

MATCH WITH FIG. 30Ab

LoCD2a $V_H$ WITH LEADER SEQUENCE FROM MRC VECTOR

```
         10        20        30        40        50        60
123456789012345678901234567890123456789012345678901234567890
ATGGGATGGAGCTGTATCATCCTCTTCTTGGTAGCAACAGCTACAGGTAAGGGGCTCACA
M  G  W  S  C  I  I  L  F  L  V  A  T  A  T  G  K  G  L  T
 W  D  G  A  V  S  S  S  S  W  .  Q  C  L  Q  V  R  G  S  Q
  G  N  E  L  Y  H  P  L  L  G  S  N  S  Y  R  .  G  A  H  S

CTCCACAGGTGTCCACTCCGAGGTCCAACTGCAGCAGTCTSGACCTGAGCTTCAGAGACC
L     R  C  P  L  R  G  P  T  A  A  V  W  T  .  A  S  E  T
 S  T  G  V  H  S  E  V  Q  L  Q  Q  S  G  P  E  L  Q  R  P
  P  Q  V  S  T  P  R  S  N  C  S  S  L  D  L  S  F  R  D  P

GTACTGGGTGAAGCAGAGGCCTAAPCAGGGCCTGGAATTAGTAGGAACGATCGATCCTGA
V  L  G  E  A  E  A  .  T  G  P  G  I  S  R  K  D  R  S  .
 Y  W  V  K  Q  R  P  K  Q  G  L  E  L  V  G  R  I  D  P  E
  T  G  .  S  R  G  L  N  R  A  W  N  .  .  E  G  S  I  L  K

ATCGTCCAACACAGPCTACATGCAACTCAGCAGCCTGACATCTGAGGACACAGCAACCTA
I  V  Q  N  S  L  H  A  T  Q  Q  P  D  I  .  G  H  S  N  L
 S  S  N  T  A  Y  M  Q  L  S  S  L  T  S  E  D  T  A  T  Y
  R  P  T  Q  P  T  C  M  S  A  A  .     L  R  T  Q  Q  P  I
CACAGTCTCCTCA
H  S  L  L
 T  V  S  S
  Q  S  P
```

FIG. 30Aa

```
         70        80        90        100       110       120
1234567890123456789012345678901234567890123456789012345678901234567890
GTAGCAGSCTTGAGGTCTGGACATATATATGGGTGACAATGACATCCACTTTGCCTTTCT    120
V  A  G  L  R  S  G  H  I  Y  G  .  Q  .  H  P  L  C  L  S
 .  Q  A  .  G  L  D  I  Y  M  G  D  M  D  I  H  F  A  F  L
  S  R  L  E  V  W  T  Y  I  W  V     N  T  S  T  L  P  F  S

CGGGGCCTCACTCAAGTTCTCGTGCAAGGCTTCTGGCTATATATTTACAGAATACTATAT    240
R  G  I  S  Q  V  V  V  Q  G  F  W  L  Y  I  Y  R  I  L  Y
 G  A  S  V  K  L  S  C  R  A  S  G  Y  I  F  T  E  Y  Y  M
  G  F  Q  S  S  C  R  A  R  L  L  A  I  Y  L  Q  N  I  I  C

AGACGGTAGTATIGATTATGTTGAGAAGTTCAAAAAGAAGGCCACACTGACAGCTGATAC    360
R  R  .  Y  .  L  C  .  E  V  Q  K  E  G  H  T  D  S  .  Y
 D  G  S  I  D  Y  V  E  K  F  K  K  K  A  T  L  T  A  D  T
  T  V  V  L  I  N  L  R     S  K  R  R  P  H  .  Q  L  I  H

TTTTTGTGCTACCCGAAAATTCAACTATCGATTTGCTTACTGGGGCCAAGGCACCCTCGT    480
F  L  C  .  G  K  I  Q  L  S  I  C  L  L  G  P  R  H  P  R
 F  C  A  R  G  K  F  N  Y  R  F  A  Y  W  G  Q  G  T  L  V
  F  V     G  E  N  S  T  I  D  L  L  T  G  A  K  A  P  S  S
                                                                493
```

FIG. 30Ab

MATCH WITH FIG. 30Aa

MATCH WITH FIG. 30Bb

LoCD2a VH + NATIVE LEADER SEQUENCE

FIG. 30Ba

```
         10        20        30        40        50        60
123456789012345678901234567890123456789012345678901234567890

ATGAAATGCAGGTGGATCATCCTCTTCTTGATGGCAGTAGCTACAGGTAAGGCACTCCCA
M  K  C  R  W  I  I  L  F  L  M  A  V  A  T  G  K  A  L  P
 .  N  A  G  G  S  S  S     .  W  Q  .  L  Q  V  R  H  S  Q
  E  M  Q  V  D  H  P  I  L  D  G  S  S  Y  R  .  G  T  P  K
CAACAGGGGTCAACTCAGAAGTCCAGCTGCAGCAGTCTGGACCTGAGCTTCAGAGACCCG
Q  Q  G  S  T  Q  K  S  S  C  S  S  L  D  L  S  F  R  D  P
 N  R  G  Q  L  R  S  P  A  A  A  V  W  T  .  A  S  E  T  R
  T  G  V  N  S  E  V  Q  L  Q  Q  S  G  P  E  L  Q  R  P  G
ACTGGGTGAAGCAGAGGCCTAAACAGGGCCTGGAATTAGTAGGAAGGATCGATCCTGAAG
T  G  .  S  R  G  L  N  R  A  W  N  .  .  E  G  S  I  L  K
 L  G  R  A  E  A  .  T  G  P  G  I  S  R  K  D  R  S  .  R
  W  V  K  Q  R  P  K  Q  G  L  E  L  V  G  R  I  D  P  E  D
CGTCCAACACAGCCTACATGCAACTCAGCAGCCTGACATCTGAGGACACAGCAACCTATT
R  P  T  Q  P  T  C  N  S  A  A  .     L     T  Q  Q  P  I
 V  Q  H  S  L  N  A  T  Q  Q  P  D  I  .     H  S  N  L  F
  S  N  T  A  Y  N  Q  L  S  S  L  T  S  E  D  T  A  T  Y  F
CAGTCTCCTCA
Q  S  P
 S  L  L
  V  S  S
```

# FIG. 30Bb

```
         70        80        90        100       110       120
123456789012345678901234567890123456789012345678901234567890

AGTCCTAAACTTGAGAGATCATACACTTGGGAGACAGTGACACTATCTTTGGATTTCTTT  120
S  F  K  L  E  R  S  Y  T  W  E  T  V  T  L  S  L  D  F  F
 V  L  H  L  R  D  H  T  L  G  R  Q  .  H  Y  L  W  I  S  F
  S  .  T  .  E  I  I  H  L  G  D  S  D  T  I  F  G  F  L  S

GGGCCTCAGTCAAGTTGTCGTGCAAGGCTTCTGGCTATATATTTACAGAATACTATATGT  240

G  P  Q  S  S  C  R  A  R  L  L  A  I  Y  L  Q  N  T  I  C
 G  L  S  Q  V  V  V  Q  G  F  W  L  Y  I  Y  R  I  L  Y  V
  A  S  V  K  L  S  C  K  A  S  G  Y  I  F  T  E  Y  Y  M  Y
ACGGTAGTATTGATTATGTTGAGAAGTTCAAAAAGAAGGCCACACTGACAGCTGATACAT  360
T  V  V  L  I  M  L  R  S  S  K  R  R  P  H  .  Q  L  I
 R  .  Y  .  I  C  .  E  V  Q  R  E  G  H  T  D  S  .  Y  I
  G  S  I  D  Y  V  E  K  F  K  K  K  A  T  L  T  A  D  T  S
TTTGTGCTAGGGGAAAATTCAACTATCGATTTGCTTACTGGGGCCAAGGCACCCTCGTCA  480
 V  L  G  E  N  S  T  I  D  L  L  T  G  A  K  A  P  S  S
L  C  .  G  K  I  Q  L  S  I  C  L  L  G  P  P     P  R  H
 C  A  R  G  K  F  N  Y  R  F  A  Y  W  G  Q  G  T  L  V  T
                                                              491
```

MATCH WITH FIG. 30Ba